(19) **Europäisches Patentamt / European Patent Office / Office européen des brevets**

(11) **EP 4 644 569 A2**

(12) **EUROPEAN PATENT APPLICATION**

(43) Date of publication:
**05.11.2025 Bulletin 2025/45**

(51) International Patent Classification (IPC):
**C12Q 1/6841** (2018.01)

(21) Application number: **25189204.8**

(22) Date of filing: **27.05.2022**

(52) Cooperative Patent Classification (CPC):
(C-Sets available)
**C12Q 1/6841** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.05.2021 US 202163194536 P**

(62) Document number(s) of the earlier application(s) in accordance with Art. 76 EPC:
**22735702.7 / 4 347 878**

(71) Applicants:
• **The Broad Institute Inc.**
  **Cambridge, MA 02142 (US)**
• **Massachusetts Institute of Technology**
  **Cambridge, MA 02139 (US)**

(72) Inventors:
• **WANG, Xiao**
  **Cambridge, MA 02142 (US)**
• **ZENG, Hu**
  **Cambridge, MA 02142 (US)**
• **REN, Jingyi**
  **Cambridge, MA 02139 (US)**

(74) Representative: **Pinsent Masons LLP**
**30 Crown Place**
**London EC2A 4ES (GB)**

Remarks:
•This application was filed on 12.07.2025 as a divisional application to the application mentioned under INID code 62.
•Claims filed after the date of filing of the application / after the date of receipt of the divisional application (Rule 68(4) EPC).

(54) **CO-MAPPING TRANSCRIPTIONAL STATES AND PROTEIN HISTOLOGY**

(57) The present disclosure provides methods and systems for mapping gene and protein expression in a cell (*i.e.,* mapping gene and protein expression within the same cell simultaneously). The present disclosure also provides methods for diagnosing a disease or disorder (*e.g.,* a neurological disorder such as Alzheimer's disease) in a subject. Methods of screening for a candidate agent capable of modulating gene and/or protein expression are also provided by the present disclosure. The present disclosure also provides methods for treating a disease or disorder, such as Alzheimer's disease, in a subject in need thereof. A plurality of oligonucleotide probes, which may be useful for performing the methods described herein, are also described by the present disclosure, as well as kits comprising any of the oligonucleotide probes described herein. Additionally, the present disclosure provides methods, apparatuses, and non-transitory computer-readable storage media for identifying spatial variations of cell types in at least one image.

**FIG. 1A**

EP 4 644 569 A2

(52) Cooperative Patent Classification (CPC): (Cont.)

C-Sets
**C12Q 1/6841, C12Q 2525/161, C12Q 2531/125,
C12Q 2537/162, C12Q 2563/179**

**Description**

**RELATED APPLICATIONS**

This application claims priority under 35 U.S.C. § 119(e) to U.S. Provisional Application, U.S.S.N. 63/194,536, filed May 28, 2021, which is incorporated herein by reference.

**BACKGROUND OF THE INVENTION**

Alzheimer's disease (AD) is a progressive neurodegenerative disorder and the most common form of dementia in the elderly (Masters *et al.,* 2015). Widespread deposition of amyloid-β (Aβ) plaques and neurofibrillary tangles (hyperphosphorylated tau deposits), especially in the neocortex and hippocampus, are the neuropathologic hallmarks of AD (Braak and Braak, 1991; Hardy and Selkoe, 2002; Masters *et al.,* 2015). In addition, AD pathology also features gliosis (reactive changes of microglia and astrocytes) and white matter abnormalities (Beach *et al.,* 1989; Henstrindge *et al.,* 2019; Butt *et al.,* 2019). A key question in AD research is how the morphological hallmarks correlate with cellular gene pathways that drive neurodegeneration. Genome-wide association studies (GWAS) have revealed genes associated with AD risk, contributing to unveiling the mechanism of AD pathology, and a majority of AD risk genes have been shown to be highly expressed in microglia (Pimenova *et al.,* 2018; Cauwenberghe *et al.,* 2016). Multiple bulk and scRNA-seq studies from AD mouse models and other neurodegeneration models found populations of microglia with distinctive transcriptional states, referred to as DAM (disease-associated microglia) (Bohlen *et al.,* 2019; Hansen *et al.,* 2018). In addition to DAM, astrocyte populations associated with AD pathology have also been characterized. Established analytic methods are disadvantageous to uncover the molecular and cellular complexity of AD: bulk-tissue analyses mask the heterogeneity of cell populations in the brain, and standard imaging methods can visualize few genes and proteins and identify only limited cell types. The recent application of single-cell RNA sequencing (scRNA-seq) to AD brain tissue has revealed substantial heterogeneous changes in gene expression in major brain cell types (Grubman *et al.,* 2019; Keren-Shaul *et al.,* 2017; Mathys *et al.,* 2019). However, although scRNA-seq studies gain single-cell resolution, they cannot preserve spatial patterns. It is also not easy to isolate single-cell preparations of all cell types from the brain in an unbiased manner. To truly understand the scope and heterogeneity of diverse cellular responses to amyloid plaque, tau aggregation, cell death, and synapse loss, and to investigate the spatial relationships between the above localized pathologies and cellular responses, a fundamentally different technology platform is needed. Therefore, integrated methods of spatially resolved single-cell transcriptomics and tissue histology are highly desired in AD research and would be useful for many other applications as well.

Many existing spatially resolved transcriptomic technologies (*e.g.,* Spatial Transcriptomics, STARmap, *etc.)* are incompatible with protein detection in the same tissue sections (Ståhl *et al.,* 2016; Stuart and Satija, 2019; Wang *et al.,* 2018). Plaque-induced genes (PIG) have been uncovered using Spatial Transcriptomics (Ståhl *et al.,* 2016; Stuart and Satija, 2019; Wang *et al.,* 2018) with fluorescent staining of adjacent brain sections. However, the resolution is limited, and only a small set of genes have been verified at cellular resolution. Furthermore, because of the relative thickness of each section, the adjacent-section strategy is less accurate and cannot be used to explore the influence of tau tangles on gene expression in the same cells. Accordingly, new methods for mapping gene expression and protein histology in the same tissue sample are needed, and such methods would be useful in the study and treatment of Alzheimer's disease.

**SUMMARY OF THE INVENTION**

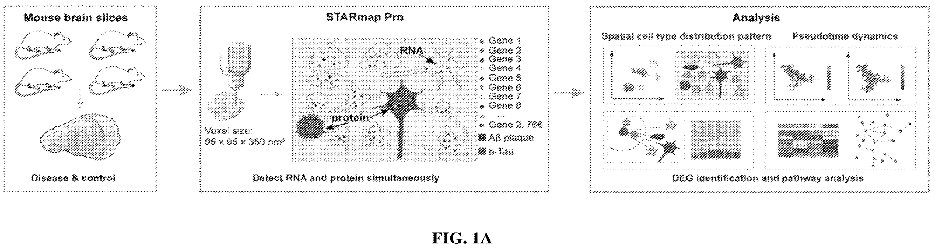

The present disclosure describes methods for profiling gene and protein expression within the same cell. In particular, the development of a method/system referred to herein as "STARmap Pro" is described in the present disclosure. STARmap Pro enables performing high-resolution spatial transcriptomics concomitantly with specific protein localization in the same tissue section. This method/system is useful, for example, for understanding AD pathophysiology with a comprehensive molecular atlas at subcellular resolution across multiple cell types (FIG. 1A). This method/system is also useful for studying, diagnosing, or treating any diseases which involve alterations in gene and/or protein expression, such as cancer, and for studying tissue development. STARmap Pro may also be employed to characterize gene and protein expression associated with any disease, to study development of a normal tissue, or to study the effect of an agent on a tissue (including screening for agents that have a particular effect on a tissue). For example, the present disclosure describes the use of an established mouse model of AD with both amyloid plaque and tau pathologies (TauPS2APP triple transgenic mice) that express mutant forms of hPresenilin 2 (PS2), hAPP, and hTAU and show age-related brain amyloid deposition, tauopathy, gliosis, and cognitive deficits (Grueninger *et al.,* 2010; Lee *et al.,* 2021). By mapping a targeted list of 2,766 genes extracted from previous bulk and single-cell RNA-seq references and diverse AD-related database, a spatial cell atlas of 8- and 13-month-old TauPS2APP mice was created in the context of extracellular Aβ plaques and intracellular phospho-Tau accumulation at subcellular resolution. Single-cell resolved transcriptomic analysis identified disease-associated gene pathways across diverse cell types in the cortical and hippocampal regions of the TauPS2APP model in comparison with control samples. Synthesizing the spatial maps of diverse cell types and states at different disease stages, a comprehensive spatiotemporal model of AD disease progression was established and is described herein.

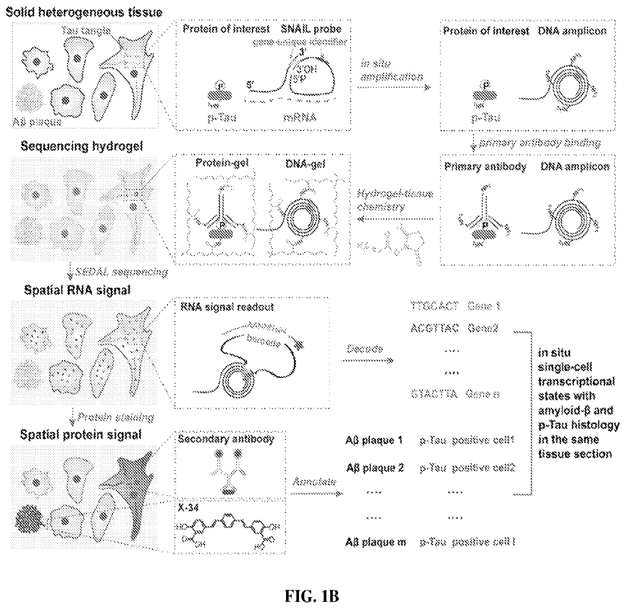

In one aspect, the present disclosure provides methods and systems for mapping gene and protein expression (of one or multiple genes and proteins) in the same cell (*i.e.,* at single-cell resolution; see, for example, FIGs. 1A and 1B). Gene and protein expression may also be mapped in multiple cells at once, for example, in multiple cells that are present in a tissue sample. In the methods disclosed herein, a cell may be contacted with one or more pairs of oligonucleotide probes to amplify nucleic acids of interest and produce one or more concatenated amplicons. The cell may then be contacted with one or more detecting agents (*e.g.,* an antibody, or an antibody fragment or variant), wherein each detecting agent binds to a protein of interest. The one or more concatenated amplicons and the one or more detecting agents may then be embedded in a polymeric matrix, and the one or more amplicons may be sequenced to determine the identity of the transcripts and their location within the polymeric matrix. The location of the detecting agents bound to one or more proteins of interest within the polymeric matrix may also be determined by imaging (*e.g.,* through confocal microscopy), allowing the location of the transcripts and the proteins of interest within the cell to be mapped simultaneously within the same sample (*e.g.,* the same cell). Using the locations of the transcripts and proteins of interest, individual cells, subcellular locations, and organelles can be identified based on the locations and expression patterns of specific genes and proteins. The method may be useful for comparing, for example, a cell (or multiple cells) from diseased and healthy tissue samples. The method may also be useful in drug discovery (*e.g.,* for screening for candidate agents that have specific effects), in studying drug side effects, and in diagnosing and treating diseases (*e.g.,* Alzheimer's disease and cancer).

In some embodiments, the present disclosure provides a method for mapping gene and protein expression in a cell comprising the steps of:

a) contacting the cell with one or more pairs of oligonucleotide probes, wherein each pair of oligonucleotide probes comprises a first oligonucleotide probe (also referred to herein as the "padlock" probe) and a second oligonucleotide probe (also referred to herein as the "primer" probe), wherein

i) the first oligonucleotide probe comprises a portion that is complementary to the second oligonucleotide probe, a portion that is complementary to a nucleic acid of interest, a first barcode sequence, and a second barcode sequence; and
ii) the second oligonucleotide probe comprises a portion that is complementary to the nucleic acid of interest, a portion that is complementary to the first oligonucleotide probe, and a barcode sequence, wherein the barcode sequence of the second oligonucleotide probe is complementary to the second barcode sequence of the first oligonucleotide probe;

b) ligating the 5' end and the 3' end of the first oligonucleotide probe together to produce a circular oligonucleotide;
c) performing rolling circle amplification to amplify the circular oligonucleotide using the second oligonucleotide probe as a primer to produce one or more concatenated amplicons;
d) contacting the cell with one or more detecting agents, wherein each detecting agent binds to a protein of interest;
e) embedding the one or more concatenated amplicons and the one or more detecting agents in a polymeric matrix;
f) contacting the one or more concatenated amplicons embedded in the polymeric matrix with a third oligonucleotide probe comprising a sequence that is complementary to the first barcode sequence of the first oligonucleotide probe; and
g) imaging the one or more concatenated amplicons embedded in the polymeric matrix and the one or more detecting agents embedded in the polymeric matrix to determine the location of the nucleic acids of interest and the proteins of interest within the cell and, optionally, map gene and protein expression.

The methods and systems described herein may be useful for studying gene and protein expression in tissue (*e.g.,* developing tissues), for diagnosing and treating various diseases, and for drug discovery. Thus, in another aspect, the present disclosure provides methods for diagnosing a disease or disorder (*e.g.,* Alzheimer's disease) in a subject. For example, the methods for profiling gene and protein expression described herein may be performed in a cell from a sample taken from a subject (*e.g.,* a subject who is thought to have or is at risk of having a disease or disorder, or a subject who is healthy or thought to be healthy). The expression of various nucleic acids and proteins of interest in the cell can then be compared to the expression of the same nucleic acids and proteins of interest in a non-diseased cell or a cell from a non-diseased tissue sample (*e.g.,* a cell from a healthy individual, or multiple cells from a population of healthy individuals). Any alteration in the expression of the nucleic acid of interest relative to expression in a non-diseased cell may indicate that the subject has the disease or disorder. Gene and protein expression in one or more non-diseased cells may be profiled alongside expression in a diseased cell as a control experiment. Gene and protein expression in one or more non-diseased cells may have also been profiled previously, and expression in a diseased cell may be compared to this reference data for a non-diseased cell.

In another aspect, the present disclosure provides methods for screening for an agent capable of modulating gene and/or protein expression of a nucleic acid or protein of interest, or of multiple nucleic acids and/or proteins of interest. For example, the methods for mapping gene and protein expression described herein may be performed in a cell in the presence of one or more candidate agents. The expression of various nucleic acids and/or proteins of interest in the cell (*e.g.,* a normal cell, or a diseased cell) can then be compared to the expression of the same nucleic acids and/or proteins of interest in a cell that was not exposed to the one or more candidate agents. Any alteration in the expression of the nucleic acid(s) and/or protein(s) of interest relative to expression in the cell that was not exposed to the candidate agent(s) may indicate that expression of the nucleic acid(s) and/or proteins of interest is modulated by the candidate agent(s).

In another aspect, the present disclosure provides methods for treating a disease or disorder (*e.g.,* Alzheimer's disease) in a subject. For example, the methods for profiling gene expression and protein expression described herein may be performed in a cell (or multiple cells, for example, that make up a tissue) from a sample taken from a subject (*e.g.,* a subject who is thought to have or is at risk of having a disease or disorder). The expression of various nucleic acids and/or proteins of interest in the cell can then be compared to the expression of the same nucleic acids and/or proteins of interest in a cell from a non-diseased tissue sample. A treatment for the disease or disorder may then be administered to the subject if any alteration in the expression of the nucleic acids and/or proteins of interest relative to expression in a non-diseased cell is observed. Gene and protein expression in one or more non-diseased cells may be profiled alongside expression in a diseased cell as a control experiment. Gene and protein expression in one or more non-diseased cells may have also been profiled previously, and expression in a diseased cell (or a test cell suspected of being a diseased cell) may be compared to this reference data for a non-diseased cell.

In another aspect, the present disclosure provides a plurality of oligonucleotide probes comprising a first oligonucleotide probe (also referred to herein as the "padlock" probe) and a second oligonucleotide probe (also referred to herein as the "primer" probe), wherein:

> i) the first oligonucleotide probe comprises a portion that is complementary to the second oligonucleotide probe, a portion that is complementary to a nucleic acid of interest, a first barcode sequence, and a second barcode sequence; and
> ii) the second oligonucleotide probe comprises a portion that is complementary to the nucleic acid of interest, a portion that is complementary to the first oligonucleotide probe, and a barcode sequence,

wherein the barcode sequence of the second oligonucleotide probe is complementary to the second barcode sequence of the first oligonucleotide probe.

In another aspect, the present disclosure provides kits (*e.g.,* a kit comprising any of the pluralities of oligonucleotide probes disclosed herein). In some embodiments, the kit comprises a library of pluralities of oligonucleotide probes as described herein, each of which can be used to identify a specific nucleic acid of interest. In some embodiments, the kit further comprises a detecting agent, or a library of detecting agents, for detecting various proteins of interest. The kits described herein may also include any other reagents or components useful in performing the methods described herein, including but not limited to cells, ligase, polymerase, amine-modified nucleotides, primary antibodies, secondary antibodies, buffers, and/or reagents for making a polymeric matrix (*e.g.,* a polyacrylamide matrix).

Another aspect of the present disclosure provides methods for identifying spatial variations of cell types in at least one image (*i.e.,* looking at variations in the spatial distribution of specific cell types relative to one another between multiple samples, for example, a healthy tissue compared to a diseased tissue). In some embodiments, such a method comprises steps of:

> receiving, for each of a plurality of cells in the at least one image, a spatial location of the cell in the at least one image;
> receiving, for each of a plurality of proteins in the at least one image, a spatial location of the protein in the image;
> for a first protein of the plurality of proteins, determining a number of cells of a first cell type having a distance to the first protein less than a threshold distance, wherein the distances are determined based on at least some of the spatial locations of the plurality of cells and at least some of the spatial locations of the plurality of proteins;
> based on the number of cells of the first cell type, identifying a spatial variation in cells of the first cell type in the at least one image; and
> outputting an indication of the spatial variation in cells of the first cell type in the at least one image. Such a method is useful, for example, for identifying cell types within a specific distance of a protein of interest which may be associated with a disease. For example, as described further herein, the presence of specific cell types with a certain distance from $A\beta$ and/or Tau inclusion bodies may be associated with Alzheimer's disease.

In another aspect, the present disclosure provides an apparatus comprising at least one computer processor;

and at least one non-transitory computer-readable storage medium encoded with a plurality of instructions that, when executed by at least one computer processor, perform a method of identifying spatial variations of cell types in at least one image, the method comprising:

receiving, for each of a plurality of cells in the at least one image, a spatial location of the cell in the at least one image;
receiving, for each of a plurality of proteins in the at least one image, a spatial location of the protein in the image;
for a first protein of the plurality of proteins, determining a number of cells of a first cell type having a distance to the first protein less than a threshold distance, wherein the distances are determined based on at least some of the spatial locations of the plurality of cells and at least some of the spatial locations of the plurality of proteins;
based on the number of cells of the first cell type, identifying a spatial variation in cells of the first cell type in the at least one image; and
outputting an indication of the spatial variation in cells of the first cell type in the at least one image.

In another aspect, the present disclosure provides at least one non-transitory computer-readable storage medium encoded with a plurality of instructions that, when executed by at least one computer processor, perform a method of identifying spatial variations of cell types in at least one image, the method comprising:

receiving, for each of a plurality of cells in the at least one image, a spatial location of the cell in the at least one image;
receiving, for each of a plurality of proteins in the at least one image, a spatial location of the protein in the image;
for a first protein of the plurality of proteins, determining a number of cells of a first cell type having a distance to the first protein less than a threshold distance, wherein the distances are determined based on at least some of the spatial locations of the plurality of cells and at least some of the spatial locations of the plurality of proteins;
based on the number of cells of the first cell type, identifying a spatial variation in cells of the first cell type in the at least one image; and
outputting an indication of the spatial variation in cells of the first cell type in the at least one image.

It should be appreciated that the foregoing concepts, and additional concepts discussed below, may be arranged in any suitable combination, as the present disclosure is not limited in this respect. Further, other advantages and novel features of the present disclosure will become apparent from the following detailed description of various nonlimiting embodiments when considered in conjunction with the accompanying figures.

## BRIEF DESCRIPTION OF THE DRAWINGS

The following drawings form part of the present specification and are included to further demonstrate certain aspects of the present disclosure, which can be better understood by reference to one or more of these drawings in combination with the detailed description of specific embodiments presented herein.

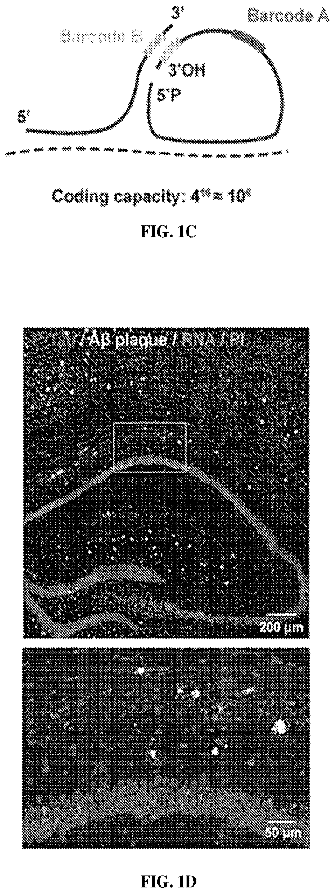

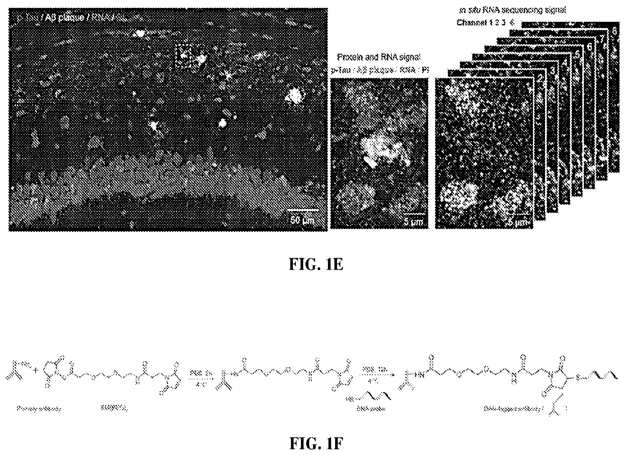

FIGs. 1A-1F show co-mapping of single-cell transcriptional states with amyloid-β and tau pathology in Alzheimer's disease using STARmap Pro. Simultaneous mapping of cell types, single-cell transcriptional states, and tissue histopathology at 200-nm resolution is shown. FIG. 1A provides an overview of the STARmap Pro method, which is an integrative *in situ* method capable of simultaneously mapping thousands of RNA species and protein disease markers in the same intact three-dimensional (3D) tissue at subcellular resolution (200 nm). STARmap Pro was applied to characterize TauPS2APP transgenic mice (a mouse disease model of Alzheimer's disease (AD) pathogenesis) with amyloid-β and tau pathology. Integrative analysis of single-cell transcriptional states with tissue histopathology (spatial cell typing, pseudotime trajectory, differential gene expression, and gene pathway analysis) was used to uncover key cell types, cell states, and gene programs along AD disease progression. FIG. 1B provides a schematic flowchart of the STARmap Pro method. Brain tissue was dissected and fixed, and intracellular mRNAs were recognized by a pair of SNAIL (specific amplification of nucleic acids via intramolecular ligation) probes and enzymatically ligated and rolling-circle amplified to synthesize amine-modified cDNA amplicons *in situ.* Protein targets were labeled with a primary antibody, and then tissues with amine-modified cDNA amplicons, proteins, and primary antibodies were functionalized with acrylic acid N-hydroxysuccinimide ester (AA-NHS) and copolymerized with acrylamide to generate a hydrogel-tissue hybrid that fixes the locations of biomolecules (*e.g.,* the amplicons, proteins, and antibodies) for *in situ* mapping. Each cDNA amplicon contains a gene-specific identifier sequence (as labeled at the top of FIG. 1B) that is read-out through *in situ* SEDAL (sequencing with error reduction by dynamic annealing and ligation), followed by fluorescent protein staining (with a secondary antibody and the small-molecule dye X-34) to visualize protein signals. FIG. 1C demonstrates the expanded coding capacity of STARmap Pro. SNAIL probes in STARmap Pro contain two 5-nt barcodes (labeled "Barcode A" and "Barcode B") with 1 million (4^10) coding capacity in theory. FIG. 1D provides imaging results of cell nuclei, cDNA amplicons, and protein signals in the TauPS2APP mouse brain at 13 months. The zoomed in image shows the p-Tau positive cells in the CA1 region of the hippocampus. Propidium iodide (PI) staining of cell nuclei, fluorescent DNA probe staining of all cDNA amplicons, X-34 staining of Amyloid β plaque, and immunofluorescent staining of p-Tau (AT8 primary antibody followed by fluorescent goat anti-mouse secondary antibody) are shown according to the legend provided. FIG. 1E provides representative imaging results showing the simultaneous mapping of cell nuclei, cDNA amplicons, and protein signals in a TauPS2APP mouse brain slice at 13 months old. A 3D projection of a raw confocal fluorescence image shows the p-Tau positive cells in the CA1 region of the hippocampus (left). A zoomed-in view of the dashed region in the left panel is also provided (middle), which shows the last cycle of tissue histopathology imaging that detects both protein and cDNA amplicon. Propidium iodide (PI) staining of cell nuclei, fluorescent DNA probe staining of all cDNA amplicons, X-34 staining of Amyloid β plaque, and immunofluorescent staining of p-Tau (AT8 primary antibody followed by fluorescent goat anti-mouse secondary antibody) are shown according to the legend provided. Eight cycles of *in situ* RNA sequencing of the view in the middle panel are also shown (right). Fluorescent channels that each represent one round of *in situ* sequencing are shown. FIG. 1F shows an exemplary synthetic scheme for the preparation of DNA-tagged antibodies.

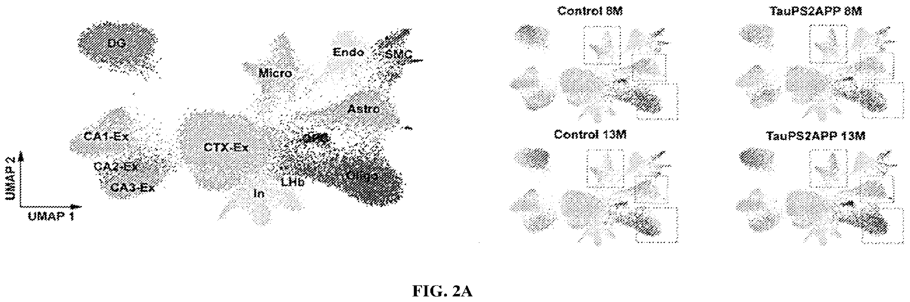

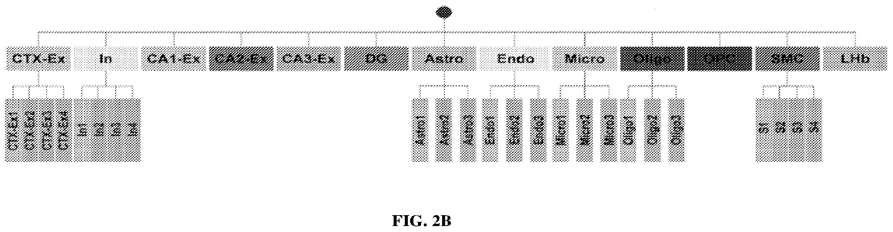

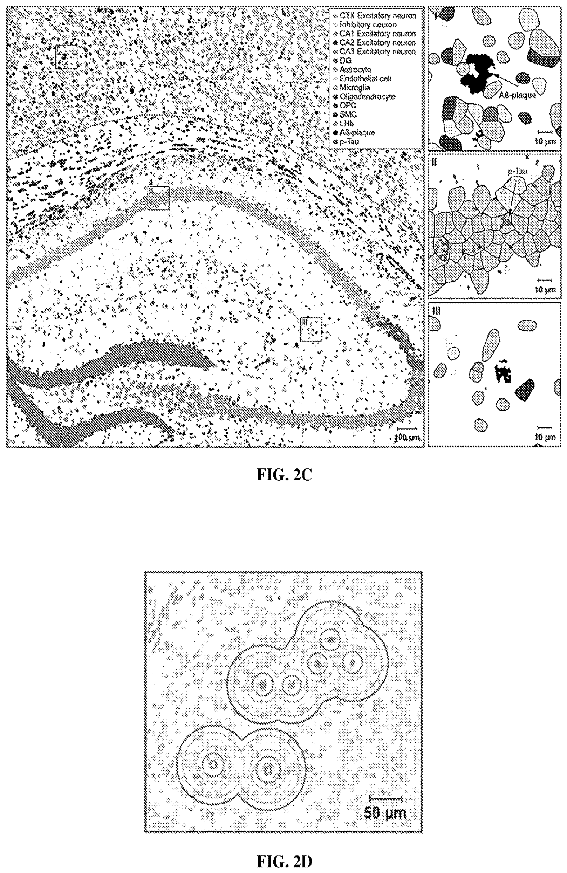

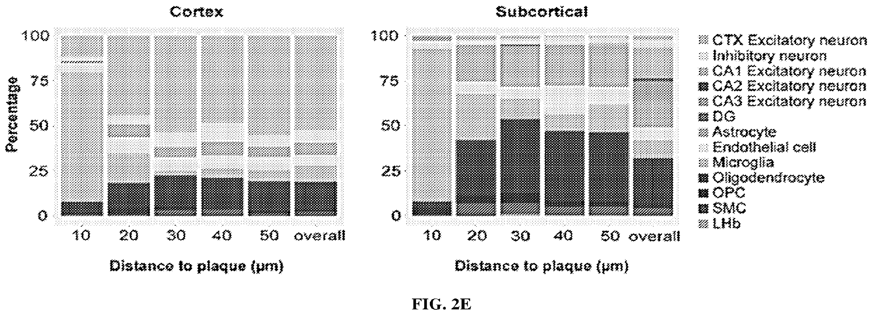

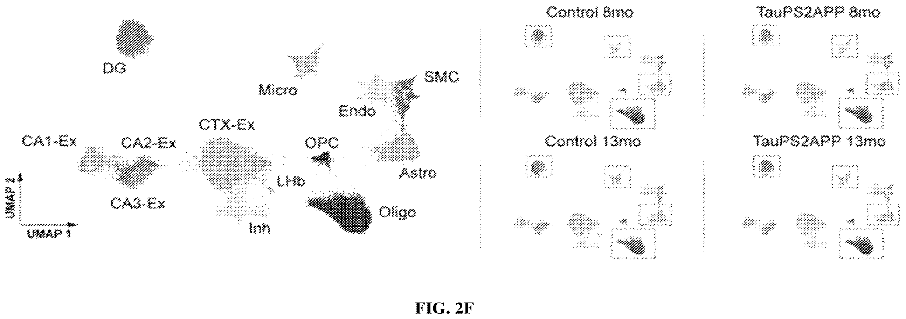

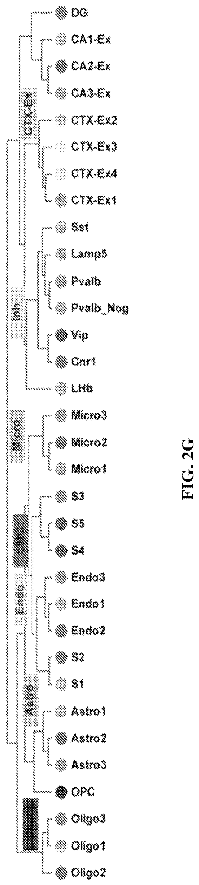

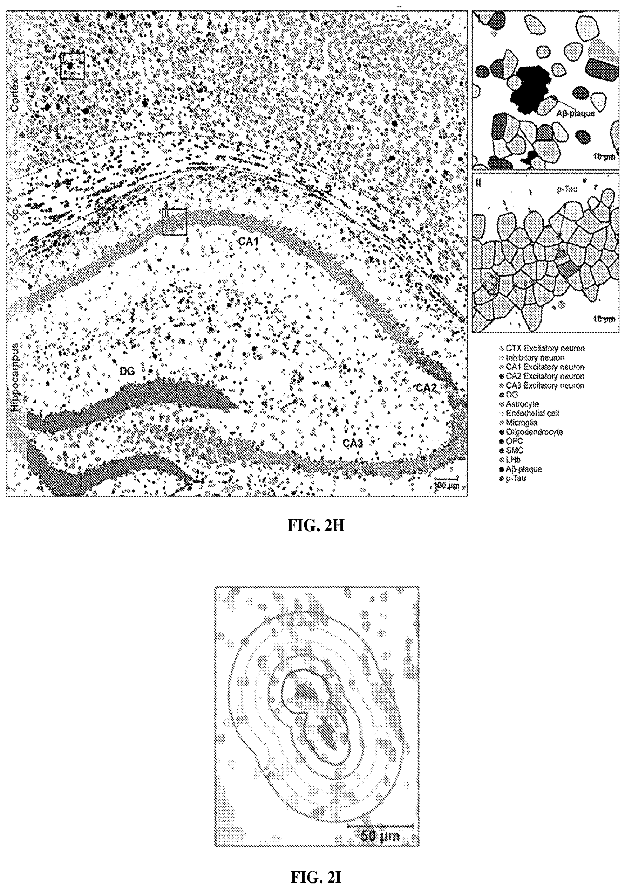

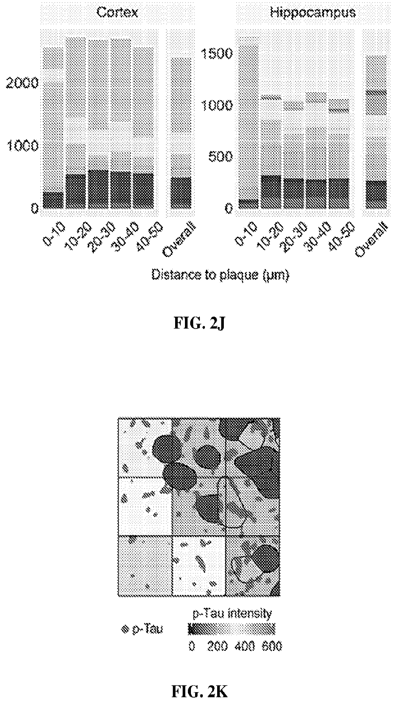

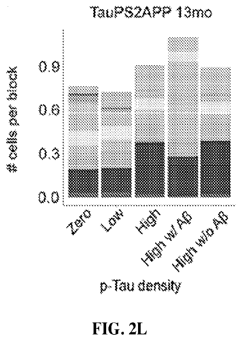

**FIGs. 2A-2L** show top-level cell type classification and spatial analysis in the brain slices of TauPS2AAPP and control mice. FIG. 2A provides a Uniform Manifold Approximation (UMAP) plot visualizing a non-linear dimensionality reduction for the transcriptomic profiles of 33,106 cells from four (4) samples. The Leiden algorithm was used to identify well-connected cells as clusters in low dimensional representation of the transcriptomics profile. Thirteen cell types were defined by gene markers enriched in each cluster. As visualized in the UMAPs of individual samples, clusters highlighted by dashed rectangles (Astrocyte, Microglia, Oligodendrocyte, and Dentate Gyrus) showed differential distribution of cell populations between TauPS2APP and control samples. FIG. 2B shows the hierarchical taxonomy of cell types. A plot is provided showing both the 13 top-level clusters (Cortex excitatory neuron (CTX-Ex, 8,687 cells), Inhibitory neuron (In, 2,005 cells), CA1 excitatory neuron (CA1-Ex, 2,754 cells), CA2 excitatory neuron (CA2-Ex, 436 cells), CA3 excitatory neuron (CA3-Ex, 1,878 cells), Dentate Gyrus (DG, 4,377 cells), Astrocyte (Astro, 2,884 cells), Endothelial cell (Endo, 1,849 cells), Microglia (Micro, 1,723 cells), Oligodendrocyte (Oligo, 4,966 cells), Oligodendrocyte precursor cell (OPC, 549 cells), Smooth muscle cell (SMC, 877 cells), Lateral Habenula neuron (LHb, 121 cells)) and 24 sub-level clusters identified during the cell type classification process. A gene expression profile of each interested top-level cluster was taken and used again for sub-level clustering with the same method. FIG. 2C shows a cell atlas of the cortex and hippocampus region of TauPS2APP 13-month sample with Aβ and tau pathologies. Top-level cell types and pathological signals are shaded as shown in the legend at the top right corner, while Aβ plaque and p-Tau protein are colored as black and gray, respectively, as shown in the legend provided. The imaging section was separated into cortical and subcortical manually, and the boundary is marked by a black dashed line. Scale bar, 100 μm. Zoomed-in sections: (I) is a zoomed-in section of the cortex region with an Aβ plaque shaded as black in the middle; (II-III) are zoomed-in sections of the subcortical region with p-Tau protein shaded as gray on top of the cells; Scale bar, 10 μm. FIG. 2D provides a schematic plot showing the strategy used for analyzing cell type composition around Aβ plaque. Taking the size of each plaque into consideration, five concentric circles with different radii (10, 20, 30, 40, and 50 μm) were generated to quantify the cell-type composition in different distance intervals away from the edge of each plaque. If a cell was present on the boundary between two of the concentric circles, they were merged to prevent repetitive counting. Scale bar, 50 μm. FIG. 2E shows cell type composition around an Aβ plaque in different distance intervals for TauPS2APP 13-month sample. A stacked bar plot is provided, showing the percentage of each top-level cell type in each distance interval (0-10, 10-20, 20-30, 30-40, and 40-50 μm) around the Aβ plaque. In each region, an overall (averaged) cell type composition is included as a reference. FIG. 2F provides a Uniform Manifold Approximation (UMAP) plot visualizing a non-linear dimensionality reduction for the transcriptomic profiles of 72,165 cells collected from coronal brain sections of TauPS2APP and control mice at 8 and 13 months. The Leiden algorithm was used to identify well-connected cells as clusters in low dimensional representation of the transcriptomics profile. Thirteen major cell types were identified by gene markers enriched in each cluster. As visualized in the UMAPs of individual samples, clusters highlighted by dashed rectangles (Astrocyte, Microglia, Oligodendrocyte, and Dentate Gyrus) showed differential distribution of cell type populations between TauPS2APP and control samples in the UMAPs. FIG. 2G shows the hierarchical taxonomy of cell types. A plot is provided showing both the 13 top-level clusters (Cortex excitatory neuron (CTX-Ex, 18,483 cells), Inhibitory neuron (Inh, 4,163 cells), CA1 excitatory neuron (CA1-Ex, 3,225 cells), CA2 excitatory neuron (CA2-Ex, 1,439 cells), CA3 excitatory neuron (CA3-Ex, 3,225 cells), Dentate Gyrus (DG, 9,562 cells), Astrocyte (Astro, 6,789 cells), Endothelial cell (Endo, 4,168 cells), Microglia (Micro, 3,732 cells), Oligodendrocyte (Oligo, 11,265 cells), Oligodendrocyte precursor cell (OPC, 1,269 cells), Smooth muscle cell (SMC, 2,397 cells), Lateral Habenula neuron (LHb, 204 cells)) and 27 sub-level clusters identified during the cell type classification process according to their representative gene markers. A gene expression profile of each interested top-level cluster was analyzed using Leiden clustering again to identify sub-level clusters with the same method. FIG. 2H shows a representative spatial cell type atlas of the cortex and hippocampus regions of TauPS2APP 13-month sample with Aβ and tau pathologies. Top-level cell types and pathological signals are shaded as shown in the legend at the top right corner, while Aβ plaque and p-Tau protein are colored as black and gray, respectively, as shown in the legend provided. The imaging section was separated into cortex, corpus callosum (CC), and hippocampus manually, and the boundary is marked by black dashed lines. Scale bar, 100 μm. Zoomed-in sections: (I) is a zoomed-in section of the cortex region with an Aβ plaque shaded as black in the middle, surrounded by different types of cells; (II) is a zoomed-in section of the hippocampal region with p-Tau protein shaded as gray on top of the cells; Scale bar, 10 μm. FIG. 2I provides schematic plots showing the strategy used for analyzing cell type composition around Aβ plaque. Taking the size of each plaque into consideration, five concentric boundaries (10, 20, 30, 40, and 50 μm away from each plaque) were generated to quantify the cell-type composition in different distance intervals away from the edge of each plaque. If a cell was present on the boundary between two of the stripes, they were merged to prevent repetitive counting. Scale bar, 50 μm. FIG. 2J shows a representative spatial distribution of cell type composition around an Aβ plaque in different distance intervals for TauPS2APP 13-month sample. A stacked bar plot is provided, showing the density (cell count per $mm^2$) of each top-level cell type in each distance interval (0-10, 10-20, 20-30, 30-40, and 40-50 μm) around the Aβ plaque. In each region, the cell density of each major cell type is included as a reference for comparison. FIG. 2K provides schematics illustrating the method used for p-Tau signal quantification. Tissue sections were divided into a grid of 20 μm x 20 μm. The shading represents integrated intensity of p-Tau in each square and was used as a metric to analyze the extent of co-localization of p-Tau with different cell types. FIG. 2L shows cell-type composition analysis based on the 20 μm x 20 μm grid in the TauPS2APP sample at 13 months ranked by p-Tau density. The blocks divided by the grid lines were ranked by the percentage of Tau positive pixels and grouped into 3 bins: 0% (zero p-Tau), 1-50% (low p-Tau), 51-100% (high p-Tau). The high p-Tau group was further divided by plaque positive versus negative groups to dissect the influence of plaque and tauopathy on cell-type distribution. Stacked bar plot showing the average number of cells per block for each major cell type.

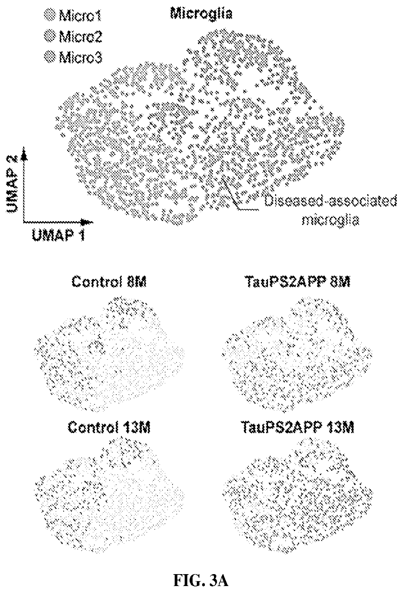

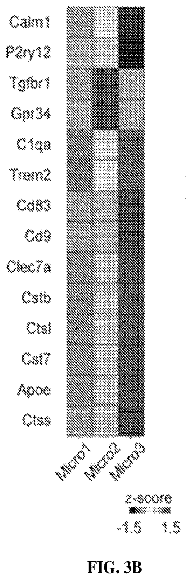

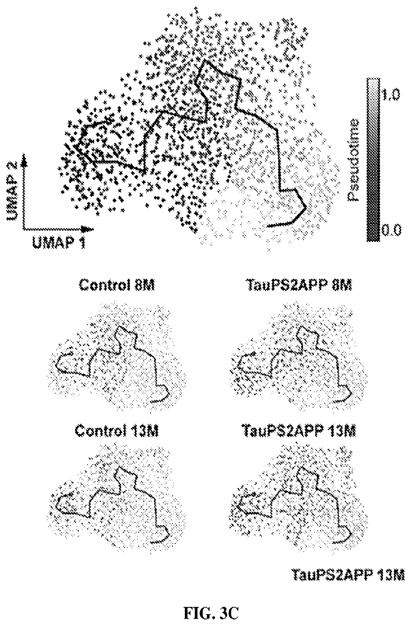

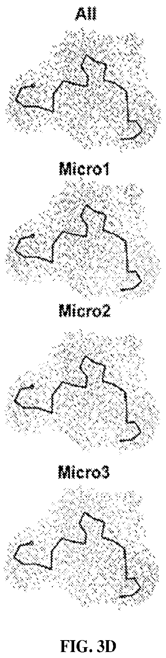

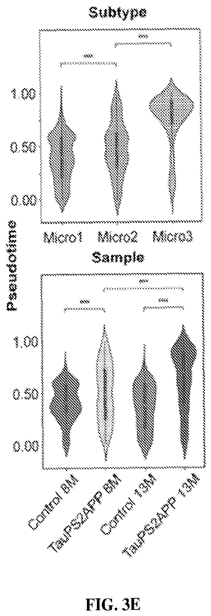

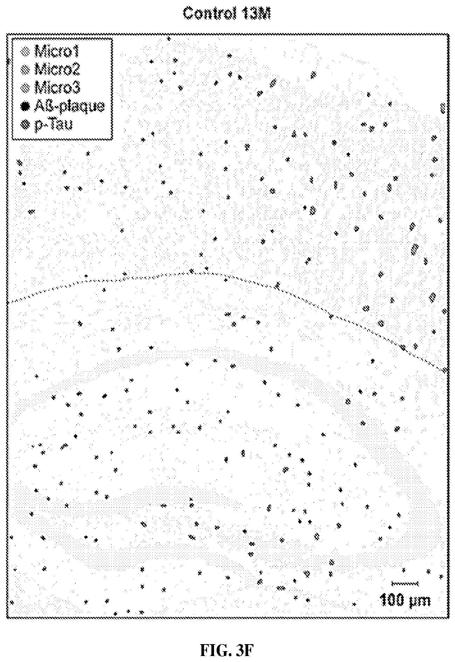

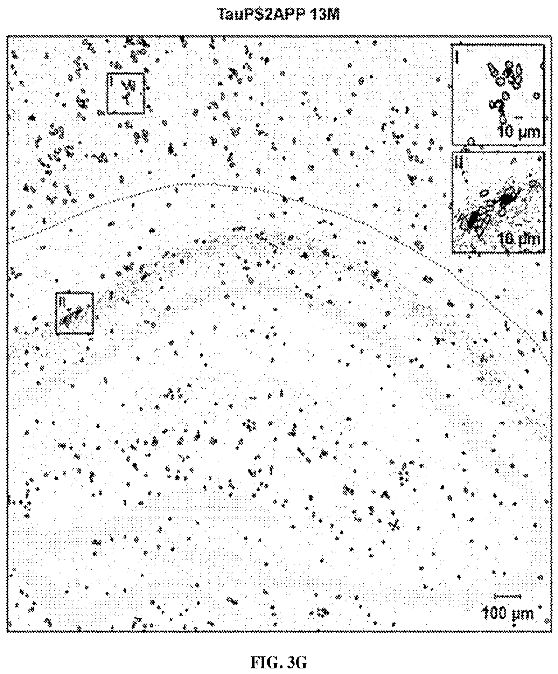

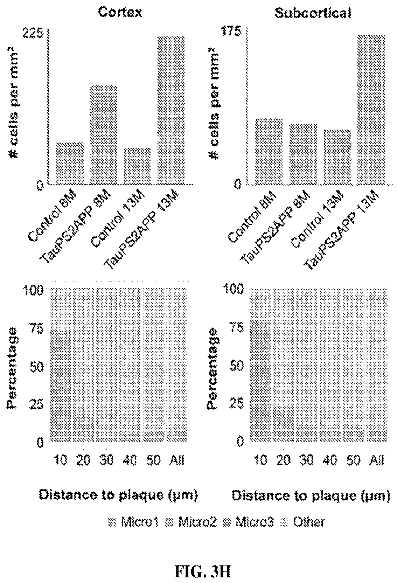

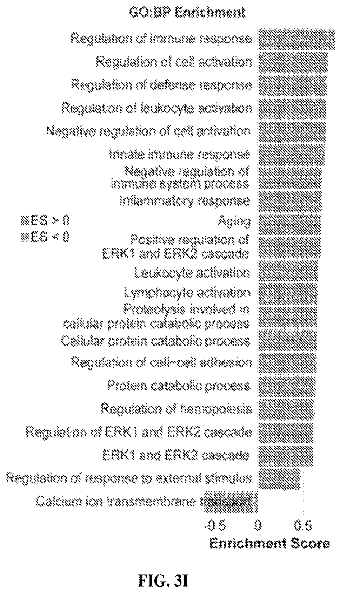

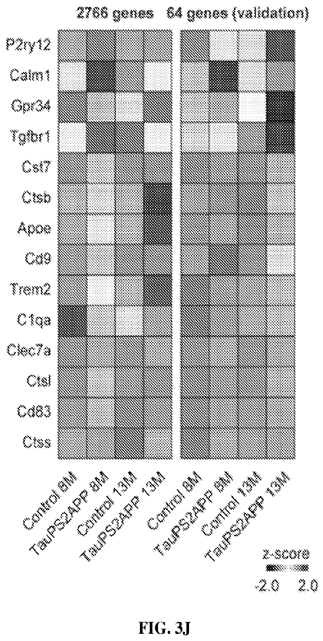

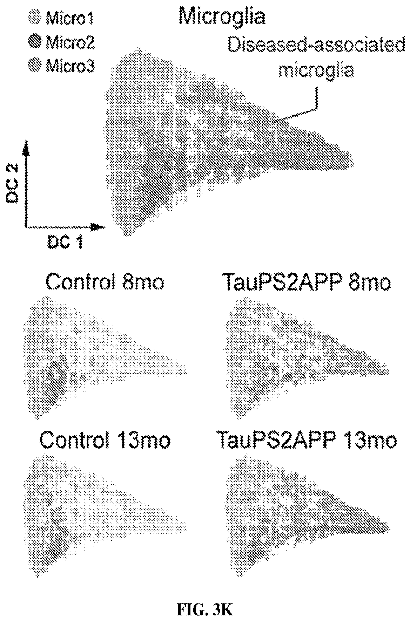

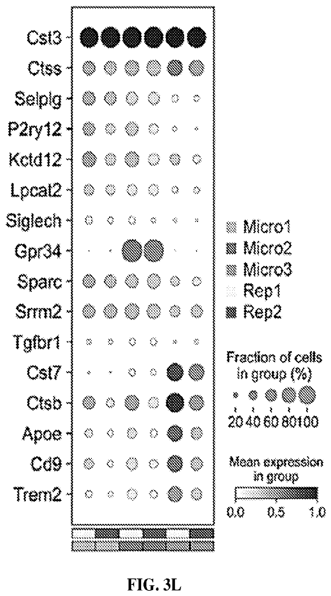

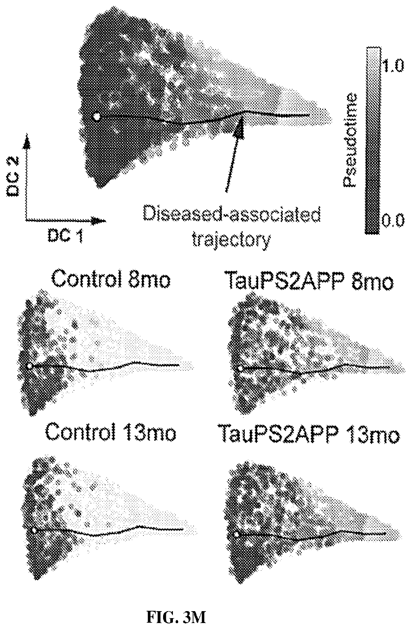

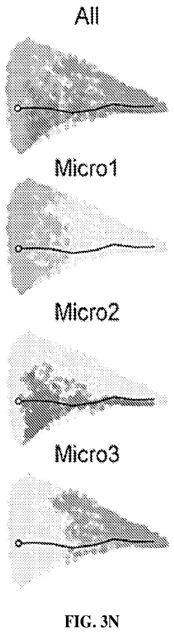

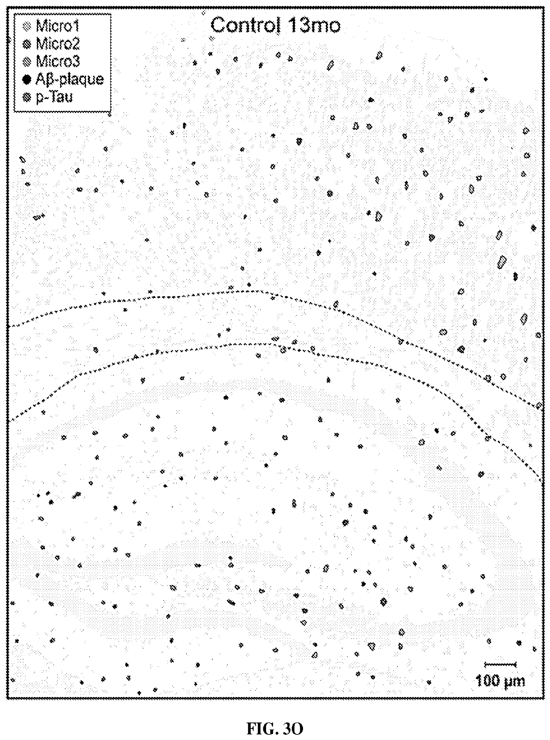

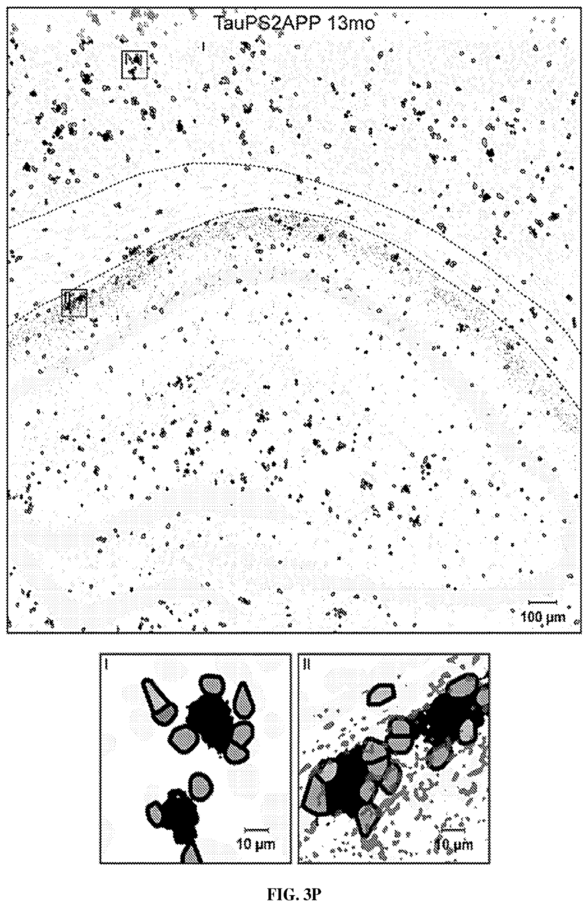

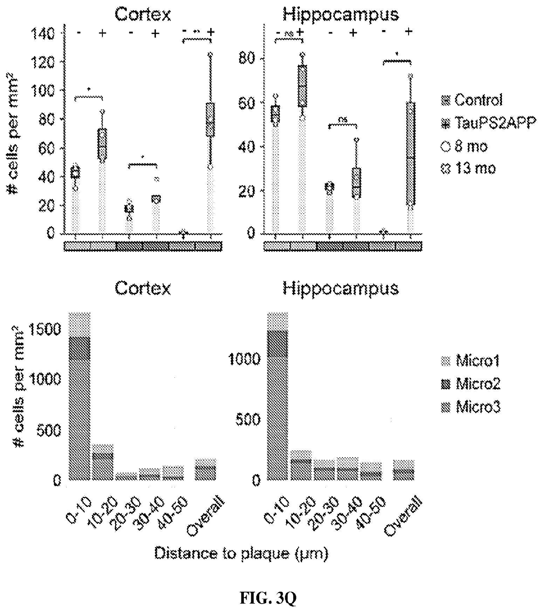

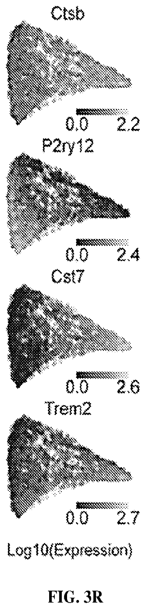

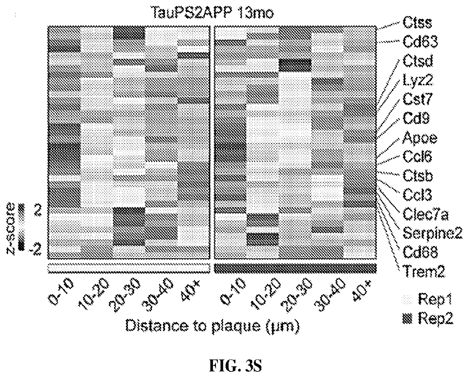

**FIGs. 3A-3S** demonstrate the unique transcriptomic response and spatial composition of a microglia population under Tau plus plaque pathology. Spatiotemporal gene expression analysis of microglia in TauPS2APP and control samples is shown. FIG. 3A provides a UMAP of the microglia cell population with subcluster annotations. A plot showing a low dimensional representation of the transcriptomics profile of 1723 microglia cells identified from FIG. 2A. Three sub-level clusters (Micro1 (n = 779), Micro2 (n = 415), and Micro3 (n = 529)) were identified by the Leiden algorithm. The Micro3 cluster was annotated as a disease-associated microglia (DAM) population by its gene markers according to previous reports and significant enrichment in diseased samples. UMAP plots with sub-level cell clusters for each sample (Control 8-month, TauPS2APP 8-month, Control 13-month, TauPS2APP 13-month) were also included. FIG. 3B shows the expression levels of representative markers across different sub-clusters (row-wise z-score). FIG. 3C provides a UMAP of the microglia cell population with pseudotime trajectory. A plot showing a low dimensional representation of the transcriptomics profile of microglia cells generated by Monocle 3 is provided. The colormap represents the pseudotime value. A corresponding trajectory was constructed and plotted on the low dimensional embedding to illustrate biological progression of the cell population. Plots for each sample are also included. The trajectory starting anchor was manually selected (based on the control sample at 8 months). FIG. 3D shows pseudotime embedding of a microglia cell population with subcluster annotations. Plots showing the low-dimensional embedding used in pseudotime computation along with subcluster annotations of microglia are provided. FIG. 3E shows pseudotime distribution. Distribution of pseudotime for three sub-populations of microglia and distribution of pseudotime for microglia in four samples is shown. Mann-Whitney-Wilcoxon test, ns: $p > 0.05$ , * $p \leq 0.05$, ** $p \leq 0.01$, *** $p \leq 0.001$, **** $p \leq 0.0001$. FIG. 3F provides a spatial map for microglia population of Control 13-month sample. Scale bar, 100 μm. FIG. 3G provides a spatial map for a microglia population of TauPS2APP 13-month sample. Scale bar, 100 μm. Two magnification sections (I, II) are shown on the top right corner. Scale bar, 10 μm. FIG. 3H shows region-wise cell density and cell type composition around plaque. A stacked bar chart showing the density (count per $mm^2$) of each microglia sub-cluster in both the cortex and subcortical region for each sample is provided (top). The shading in the bar plots correspond to the cell type legend in FIG. 3F. The plots show a significant enrichment of the DAM in both TauPS2APP samples at 8 and 13-month. A stacked bar chart showing the percentage of each microglia sub-cluster in each distance interval (0-10, 10-20, 20-30, 30-40, 40-50 μm) around the Aβ plaque is also provided (bottom). In each region, an overall cell type composition was included as a reference. FIG. 3I shows gene set enrichment analysis (GSEA) results of differentially expressed genes (DEGs). Shaded by sign of statistically significant (Nominal p-value < 0.01) enrichment score. Terms are filtered by term size: 20-1000. FIG. 3J provides a matrix plot showing a validated subset of DEGs of the microglia population from the AD vs. Control comparison. A plot is provided showing the row-wise scaled expression values of the top significantly altered (ranked by p-value) DEGs in a 2766 gene dataset and the 64 genes validation result. FIG. 3K shows subclustering of the microglia cell population. A plot showing a low dimensional representation of the transcriptomics profile of 3732 microglia cells identified from FIG. 2A (top). Three sub-level clusters (Micro1 (n = 1924), Micro2 (n = 784), and Micro3 (n = 1024)) were identified by the Leiden algorithm. The Micro3 cluster was annotated as a disease-associated microglia (DAM) population by its gene markers according to previous reports and significant enrichment in diseased samples. A diffusion map visualization of sub-level cell clusters of microglia populations for each sample (Control 8-month, TauPS2APP 8-month, Control 13-month, TauPS2APP 13-month, n = 2 under each condition) were also included. FIG. 3L shows the expression level of representative gene markers among different subclusters of cell populations. A dot plot showing both the mean gene expression (shading) of representative markers for each microglia subpopulation and the percentage of cells expressing them (dot size) is provided. Gene expression values were normalized for each column. FIG. 3M provides a diffusion map of the microglia cell population with pseudotime trajectory. A plot showing a low dimensional representation of the transcriptomics profile of microglia cells generated by Monocle 3 is provided (top). The colormap represents the pseudotime value. A corresponding trajectory was constructed and plotted on the low dimensional embedding to illustrate biological progression of the cell population. Plots for each sample are also included. The trajectory starting anchor was manually selected (based on the control sample at 8 months). Cells from different samples are highlighted separately in diffusion map embedding (bottom). FIG. 3N shows pseudotime embedding of a microglia cell population with subcluster annotations. Plots showing the low-dimensional embedding used in pseudotime computation along with subcluster annotations of microglia identified in FIG. 3K with trajectory identified in FIG. 3M are provided. FIG. 3O provides a spatial map for microglia population of Control 13-month sample. Scale bar, 100 $\mu$m. FIG. 3P provides a spatial map for a microglia population of TauPS2APP 13-month sample. Scale bar, 100 $\mu$m. Two magnification sections (I, II) are shown highlighted in black boxes. Scale bar, 10 $\mu$m. Dashed black lines mark the boundaries between cortex, corpus callosum (cc), and hippocampus. FIG. 3Q shows region-wise cell density and cell type composition around plaque. A box plot showing the density (count per mm$^2$) of each microglia sub-cluster in both the cortex and hippocampus region in control and TauPS2APP mice at two time points is provided (top). The shading in the bar plots correspond to the cell type legend in FIG. 3P. The plots show a significant enrichment of the DAM in both brain regions in TauPS2APP samples at 8 and 13-month. A stacked bar chart showing the percentage of each microglia sub-cluster in each distance interval (0-10, 10-20, 20-30, 30-40, 40-50 $\mu$m) around the A$\beta$ plaque at 13 months is also provided (bottom). In each region, the overall cell density of each subpopulation in each area was included as a reference for comparison. FIG. 3R provides a diffusion map showing the expression of four representative gene markers of microglia subtypes. The shading indicates the $\log_{10}$(mean gene expression value) of the gene in each cell. FIG. 3S provides a matrix plot showing the z-scores of spatial DEGs of microglia across multiple distance intervals (0-10, 10-20, 20-30, 30-40, 40+ $\mu$m) from plaques.

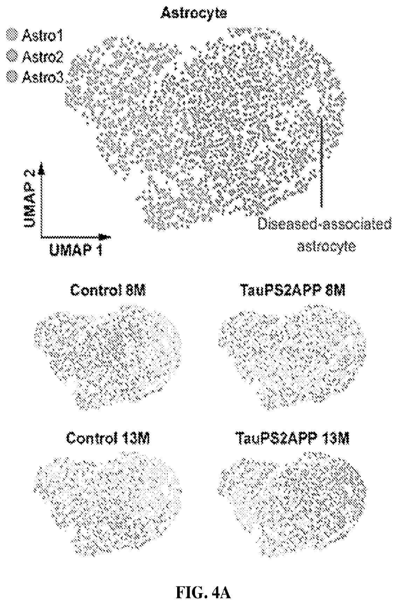

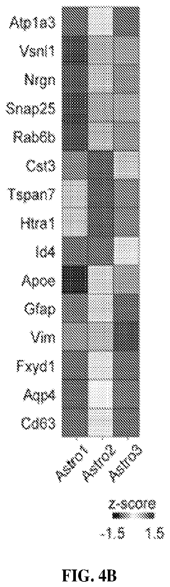

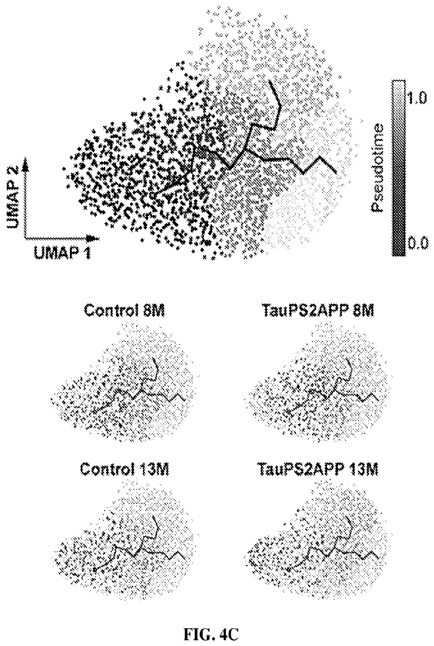

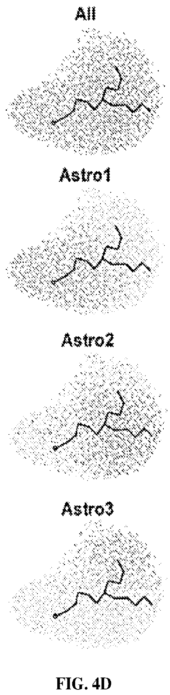

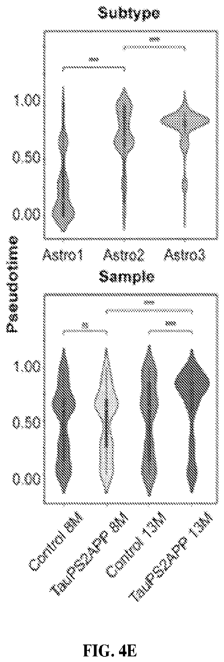

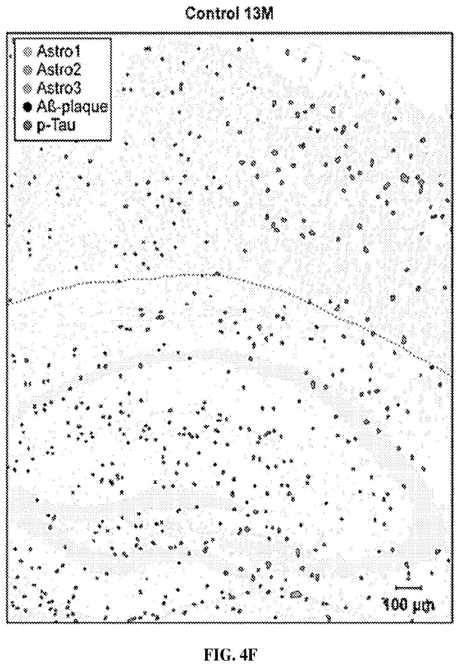

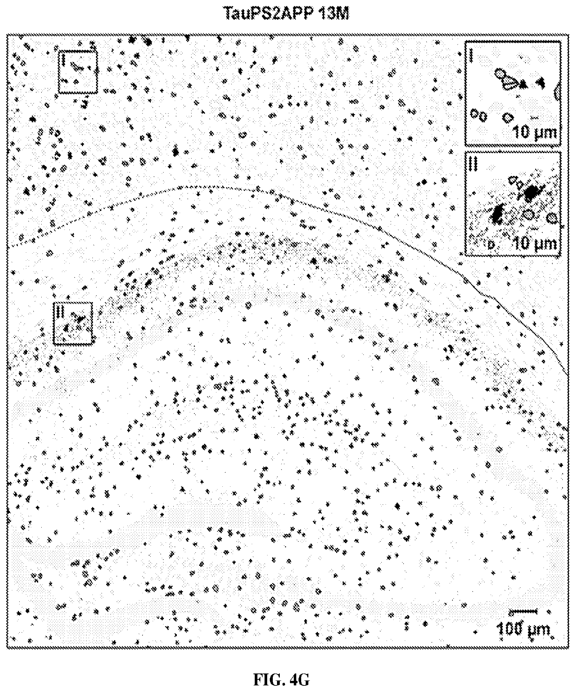

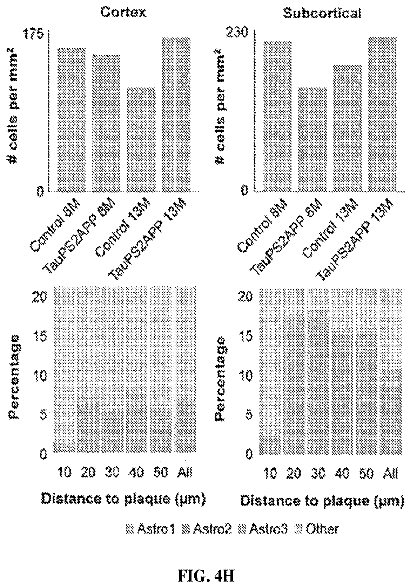

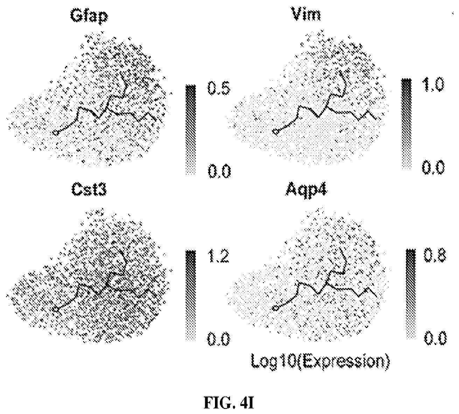

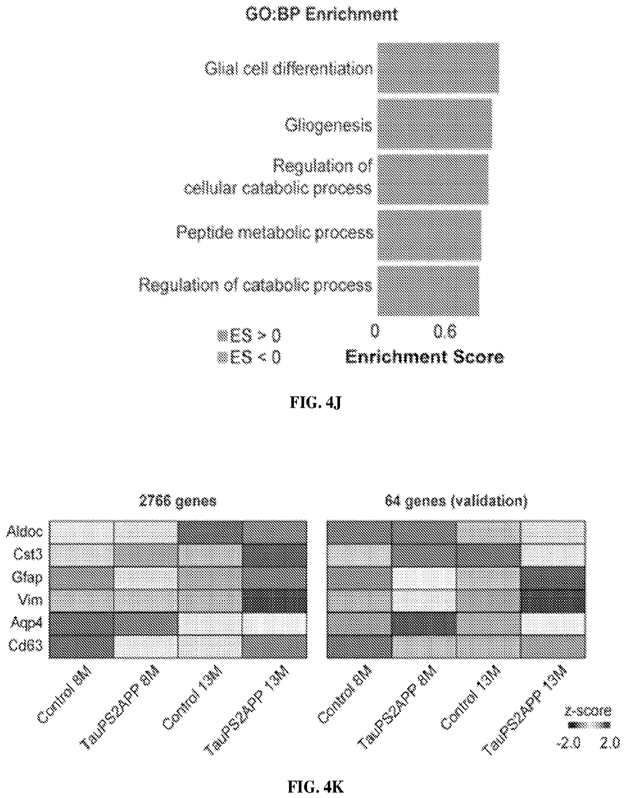

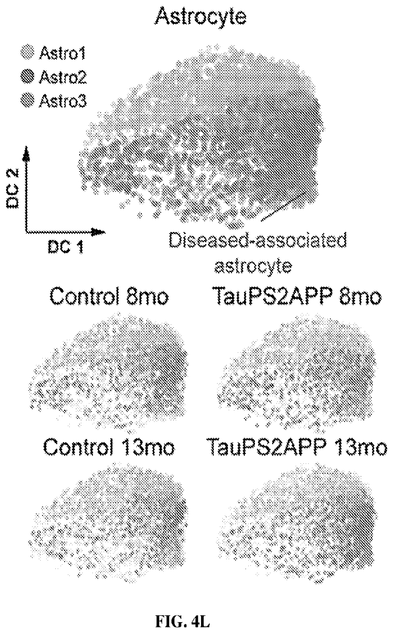

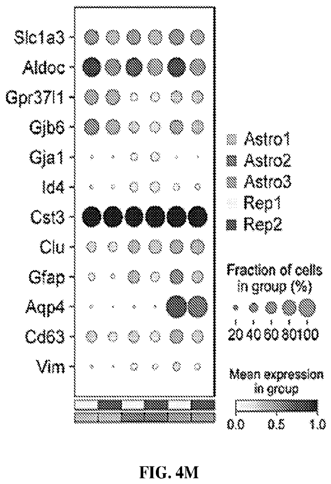

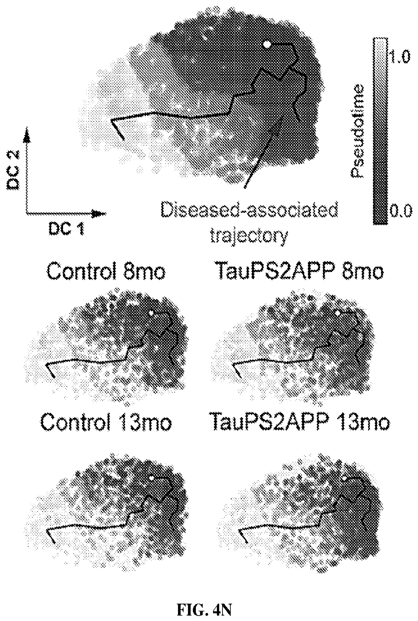

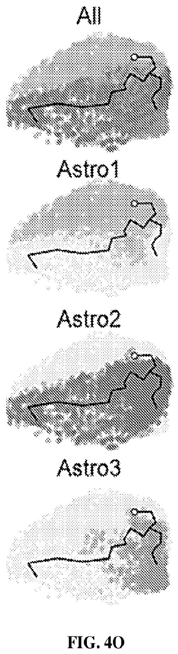

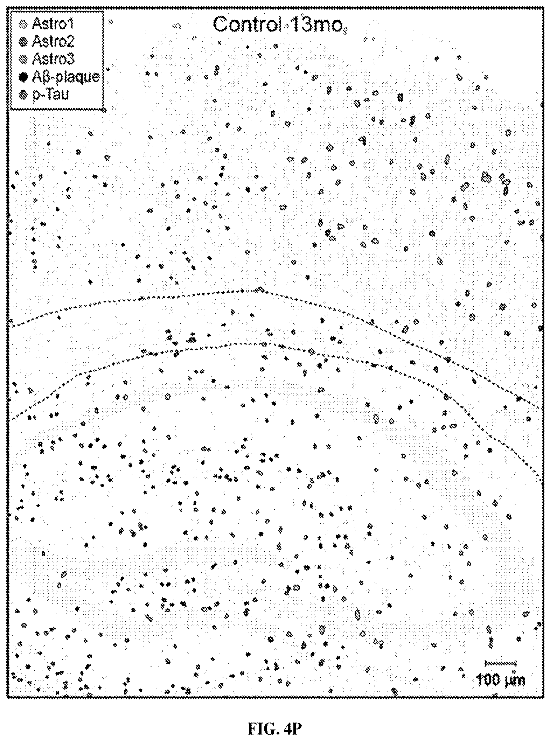

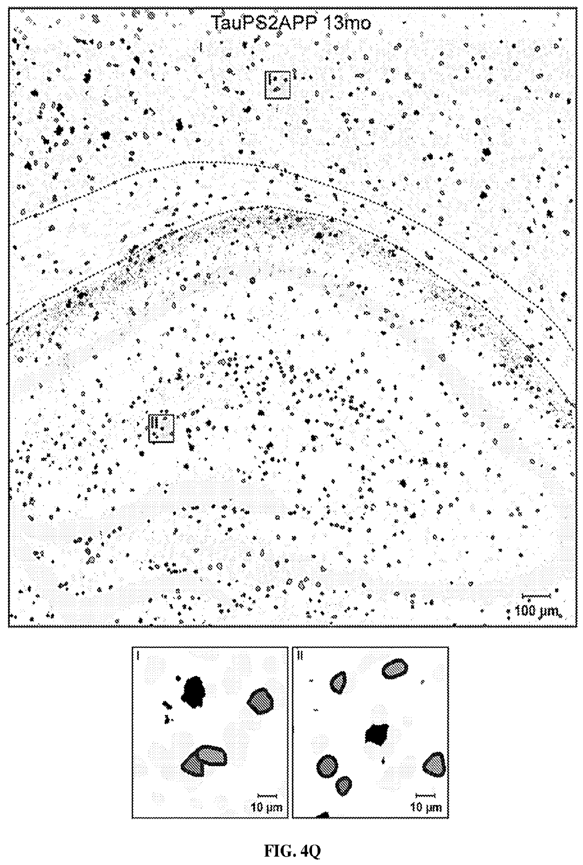

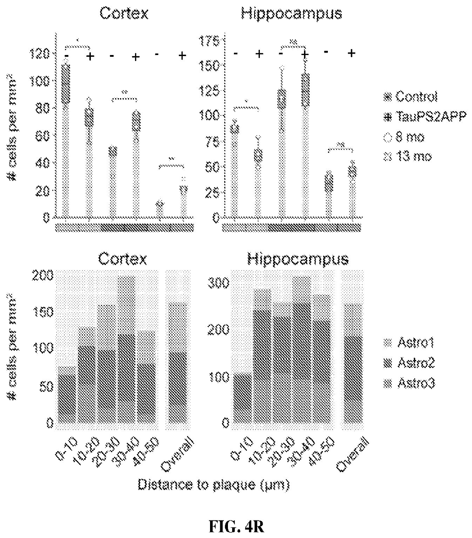

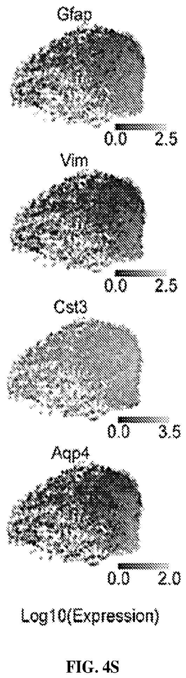

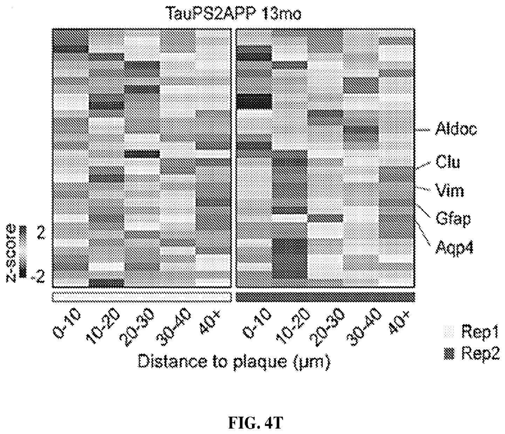

**FIGs. 4A-4T** show a disease-associated population and the spatial composition of astrocytes under Tau plus plaque pathology. Spatiotemporal gene expression analysis of astrocytes in TauPS2APP and control samples is shown. FIG. 4A provides a UMAP of an astrocyte cell population with subcluster annotations. A plot showing a low dimensional representation of the transcriptomics profile of 2884 astrocytes identified from FIG. 2A is provided. Three sub-level clusters (Astro1 (n = 1068), Astro2 (n = 1271), and Astro3 (n = 545)) were identified by the Leiden algorithm. The Astro3 cluster was annotated as disease-associated astrocyte (DAA) by its gene markers according to previous reports and significant enrichment in diseased samples. UMAP plots with sub-level cell clusters for each sample (Control 8 month, TauPS2APP 8-month, Control 13 month, TauPS2APP 13 month) were also included. FIG. 4B shows the expression levels of representative markers across different sub-clusters. Shaded by row-wise z-score. FIG. 4C provides a UMAP of an astrocyte cell population with pseudotime trajectory. A plot showing a low dimensional representation of the transcriptomics profile of astrocytes generated by Monocle 3 is provided. Colormap represents the pseudotime value. A corresponding trajectory was constructed and plotted on the low dimensional embedding to illustrate biological progression of the population. Plots for each sample were also included. FIG. 4D shows pseudotime embedding of the astrocyte cell population with subcluster annotations. Plots showing the low-dimensional embedding used in pseudotime computation with subcluster annotations of astrocytes are provided. FIG. 4E shows pseudotime distribution. Distribution of pseudotime for three sub-populations of astrocytes and distribution of pseudotime for astrocytes in four samples are shown. Mann-Whitney-Wilcoxon test, ns: $p > 0.05$, * $p \leq 0.05$, ** $p \leq 0.01$, *** $p \leq 0.001$, **** $p \leq 0.0001$. FIG. 4F provides a spatial map for the astrocyte population of Control 13-month sample. Scale bar, 100 $\mu$m. FIG. 4G provides a spatial map for the astrocyte population of TauPS2APP 13-month sample. Scale bar, 100 $\mu$m. Two magnification sections (I, II) are shown at the top right corner. Scale bar, 10 $\mu$m. FIG. 4H shows region-wise cell density and cell type composition around plaque. A stacked bar chart showing the density (count per mm$^2$) of each astrocyte sub-cluster in both the cortex and subcortical region for each sample is provided (top). The shading in the bar plots correspond to the cell type legend in FIG. 4F. The plots show a significant enrichment of the DAA in the cortex, especially at 13 months. A stacked bar chart showing the percentage of each astrocyte sub-cluster in each distance interval (0-10, 10-20, 20-30, 30-40, and 40-50 $\mu$m) around the A$\beta$ plaque is also provided (bottom). In each region, an overall cell type composition was included as a reference. FIG. 4I shows DEGs on pseudotime embedding. UMAPs showing the expression of four disease up-regulated genes on the pseudotime embedding are shown, and the shaded scale of the raw counts was adjusted by $\log_{10}$ scale. FIG. 4J shows GSEA results of differentially expressed genes (DEGs). Terms are filtered by term size (20-1000) and nominal p-value (< 0.01). FIG. 4K provides a matrix plot showing a validated subset of DEGs of the astrocyte population from the AD vs. Control comparison. A plot is provided showing the row-wise scaled expression values of the top significantly altered (ranked by p-value) DEGs in a 2766 genes dataset and the 64 genes validation result. FIG. 4L shows a subcluster of an astrocyte cell population. A plot showing a zoomed-in diffusion map visualization of the transcriptomics profile of 6,789 astrocytes identified from FIG. 2A is provided (top). Three sub-level clusters of astrocytes (Astro1 (n = 2,547), Astro2 (n = 3,278), and Astro3 (n = 964)) were identified by the Leiden algorithm. The Astro3 cluster was annotated as disease-associated astrocyte (DAA) by its gene markers according to previous reports and significant enrichment in diseased samples. Diffusion map embeddings of sub-level cell clusters of astrocytes for each sample (Control 8 month, TauPS2APP 8-month, Control 13 month, TauPS2APP 13 month, n = 2 under each condition) were also included. FIG. 4M shows the expression level of representative markers across different sub-clusters. A dot plot showing both the mean gene expression (shading) of representative markers for each astrocyte subpopulation and the percentage of cells expressing them (dot size) is provided. Gene expression values were normalized for each column. FIG. 4N provides a diffusion map of an astrocyte cell population with pseudotime trajectory. A diffusion map visualization of the transcriptomics profile of astrocytes generated by Monocle 3 is provided (top). Colormap represents the pseudotime value. A corresponding trajectory was constructed and plotted on the low dimensional embedding to illustrate biological progression of the population. Plots for each sample were also included. Trajectory starting anchor was manually selected based on the Astro1 population. Black arrow highlights bifurcation point on the trajectory related to the disease-associated gene expression changes. Cells from different samples were highlighted separately in diffusion map embedding (bottom). FIG. 4O provides a diffusion map showing different types of astrocytes identified in FIG. 4L along with trajectory identified in FIG. 4N. Pseudotime embedding of the astrocyte cell population with subcluster annotations is shown. Plots showing the low-dimensional embedding used in pseudotime computation with subcluster annotations of astrocytes are provided. FIG. 4P provides a spatial map for the astrocyte population of TauPS2APP 13-month sample. Scale bar, 100 $\mu$m. FIG. 4Q provides a spatial map for the astrocyte population of TauPS2APP 13-month sample. Scale bar, 100 $\mu$m. Two magnification sections (I, II) are shown. Scale bar, 10 $\mu$m. Dashed black lines mark the boundaries between cortex, corpus callosum (cc), and hippocampus. FIG. 4R shows region-wise cell density and cell type composition around plaque. A boxplot showing the density (count per mm$^2$) of each astrocyte sub-cluster in both the cortex and hippocampus regions for each sample (control and TauPS2APP mice) is provided at two time points (top). The shading in the bar plots correspond to the cell type legend in FIG. 4Q. The plots show a significant enrichment of the DAA in the cortex, especially at 13 months. A stacked bar chart showing the density (count per mm$^2$) of astrocyte sub-cluster in each distance interval (0-10, 10-20, 20-30, 30-40, and 40-50 $\mu$m) around the A$\beta$ plaque at 13 months is also provided (bottom). In each region, the cell density of each subpopulation in each area was included as a reference for comparison. FIG. 4S shows DEGs on pseudotime embedding. A diffusion map showing the expression of four representative gene markers of astrocyte subpopulations on the pseudotime embedding are shown, and the shaded scale of the raw counts was adjusted by log$_{10}$ transformation. FIG. 4T provides a matrix plot showing the z-scores of spatial DEGs of astrocytes across multiple distance intervals (0-10, 10-20, 20-30, 30-40, 40+ $\mu$m) from plaques.

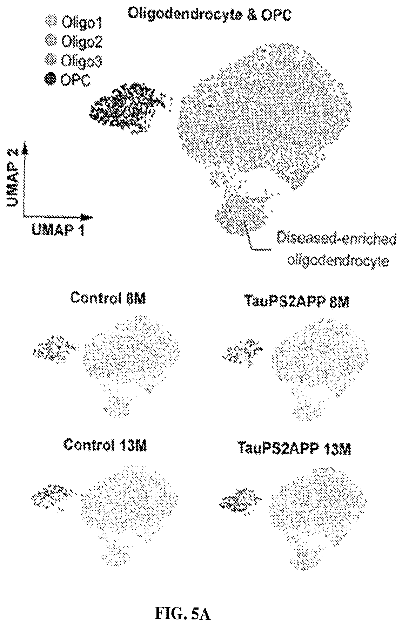

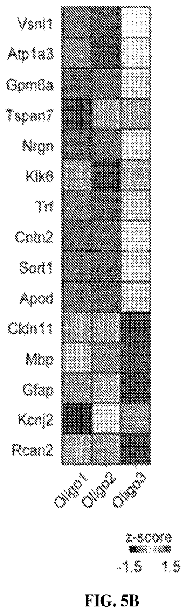

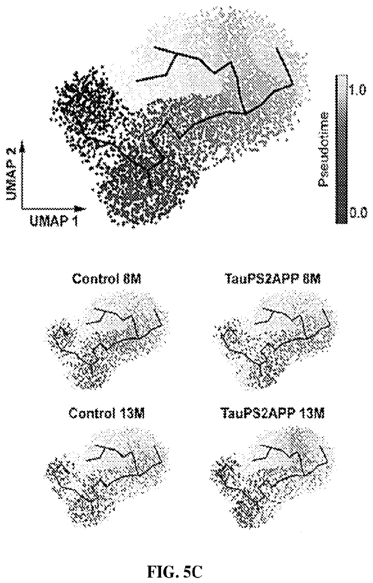

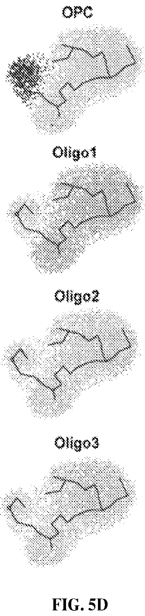

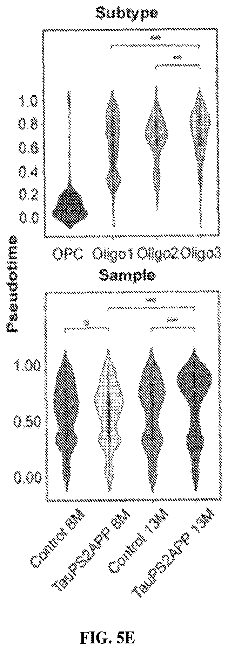

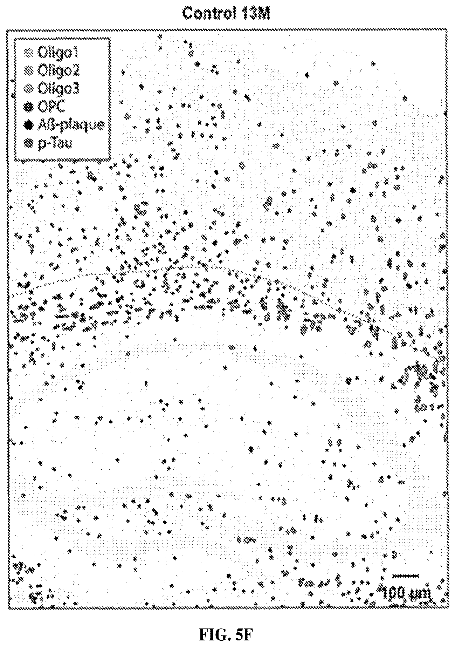

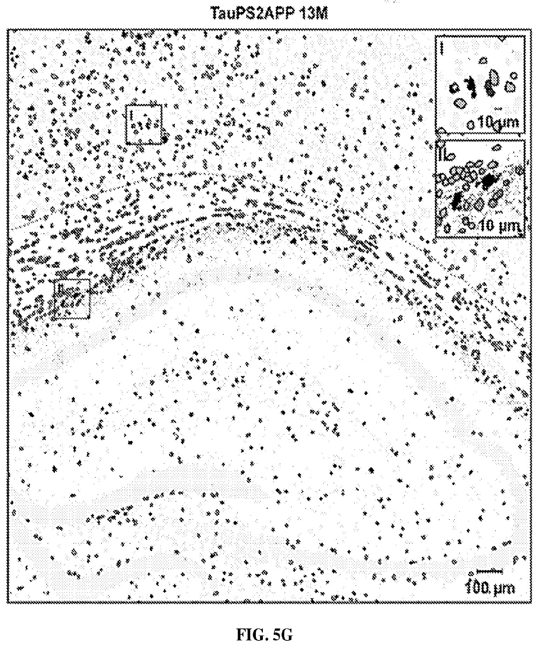

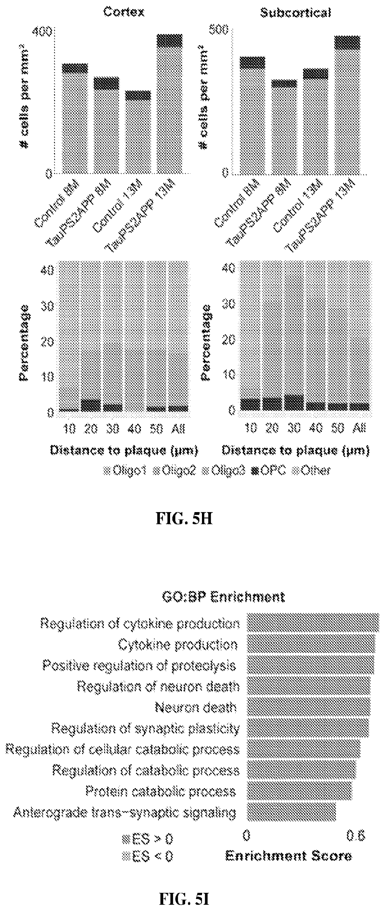

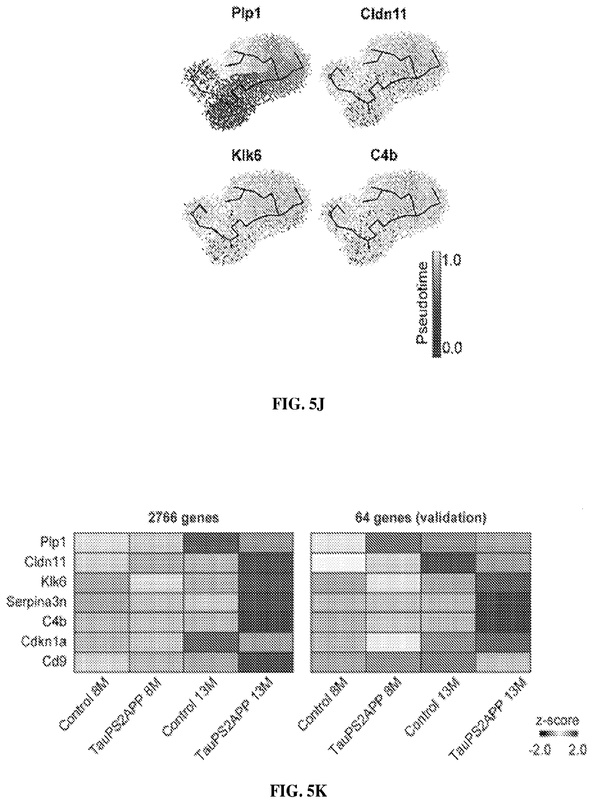

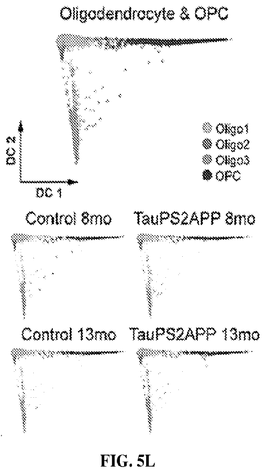

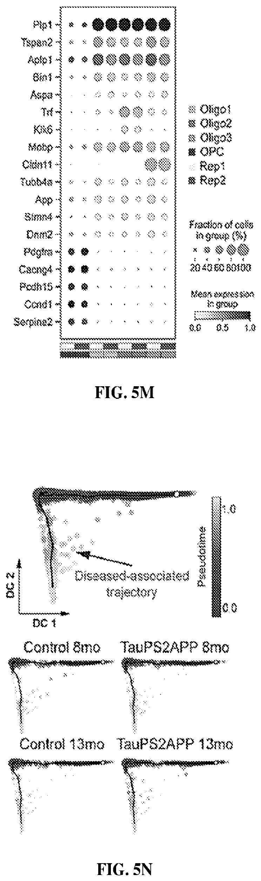

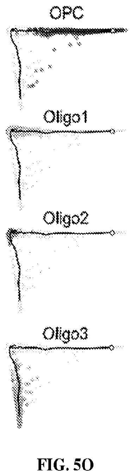

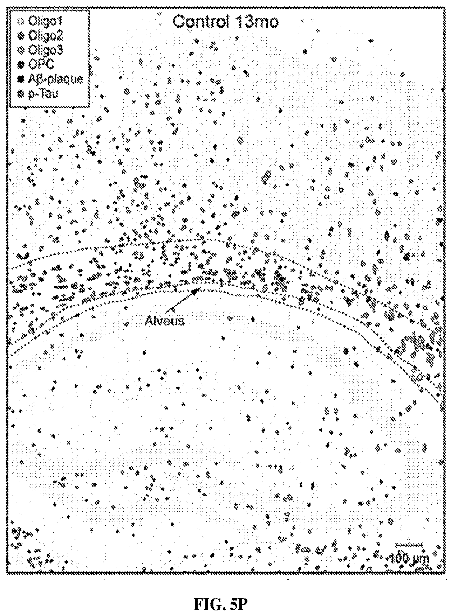

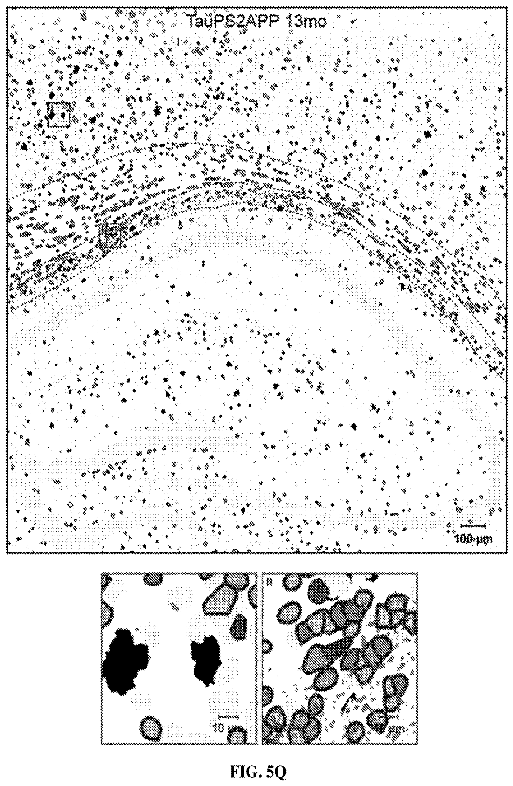

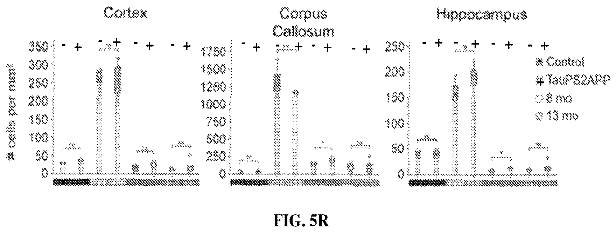

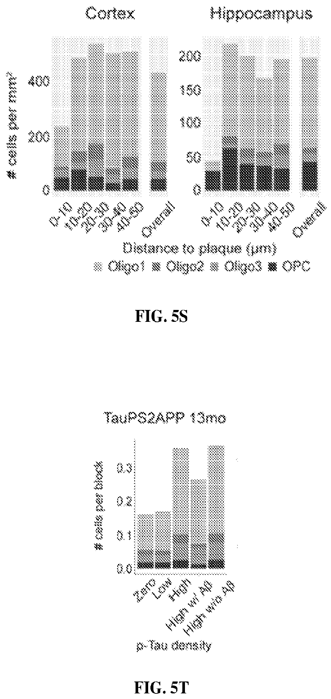

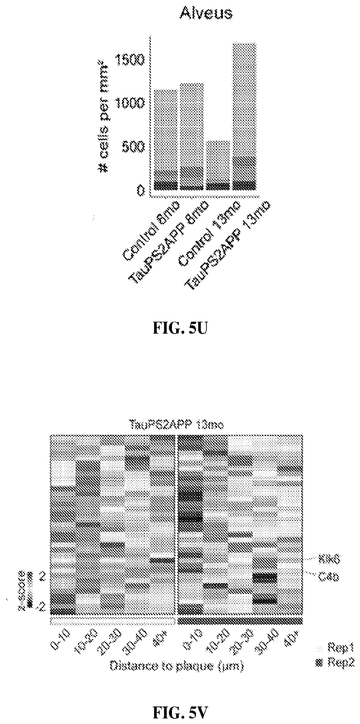

**FIGs. 5A-5V** show sub-level clustering and composition of oligodendrocytes and precursor cells under Tau plus plaque pathology. Spatiotemporal gene expression analysis of oligodendrocytes and precursor cells in TauPS2APP and control samples is shown. FIG. 5A provides a UMAP of Oligodendrocytes and precursor cell (OPC) population with subcluster annotations. A UMAP plot is provided showing a low dimensional representation of the transcriptomics profile of 4966 oligodendrocytes and 549 OPCs identified from FIG. 2A. Three sub-level clusters (Oligo1 (n = 4,295), Oligo2 (n = 181), Oligo3 (n = 490)) and OPC (n = 549) were identified by the Leiden algorithm. The Oligo3 cluster was annotated as disease-enriched oligodendrocyte. UMAP plots with sub-level cell clusters for each sample (Control 8 month, TauPS2APP 8-month, Control 13 month, TauPS2APP 13 month) were also included. FIG. 5B shows the expression levels of representative markers across different sub-clusters of oligodendrocytes (row-wise z-score). FIG. 5C provides a UMAP of an oligodendrocyte-related cell population with pseudotime trajectory. A plot showing a low dimensional representation of the transcriptomics profile of oligodendrocytes and OPCs generated by Monocle 3 is provided. Colormap represents the pseudotime value. A corresponding trajectory was constructed and plotted on the low dimensional embedding to illustrate biological progression of the population. Plots for each sample were also included. FIG. 5D shows pseudotime embedding of the oligodendrocyte-related cell population with subcluster annotations. Plots are provided showing the low-dimensional embedding used in the pseudotime computation with subcluster annotations of oligodendrocytes and precursor cells. FIG. 5E shows distribution of pseudotime for three sub-populations of oligodendrocytes and oligodendrocyte precursor cells, as well as the distribution of pseudotime for those cells in four samples. Mann-Whitney-Wilcoxon test, ns: p > 0.05, * p $\leq$ 0.05, ** p $\leq$ 0.01, *** p $\leq$ 0.001, **** p $\leq$ 0.0001). FIG. 5F provides a spatial map for the oligodendrocyte-related population of Control 13-month sample. Scale bar, 100 $\mu$m. FIG. 5G provides a spatial map for the oligodendrocyte-related population of TauPS2APP 13-month sample. Scale bar, 100 $\mu$m. Two magnification sections (I, II) are provided on the top right corner. Scale bar, 10 $\mu$m. FIG. 5H shows region-wise cell density and cell type composition around plaque. A stacked bar chart is provided showing the density (count per mm$^2$) of each oligodendrocyte sub-cluster and OPC in both the cortex and subcortical region for each sample. The shading in the bar plots corresponds to the cell type legend in FIG. 5F. A stacked bar chart is also provided showing the percentage of each oligodendrocyte sub-cluster and OPC in each distance interval (0-10, 10-20, 20-30, 30-40, and 40-50 $\mu$m) around the A$\beta$ plaque (bottom). In each region, an overall cell type composition was included as a reference. FIG. 5I shows gene set enrichment analysis (GSEA) results of differentially expressed genes (DEGs). Terms are filtered by term size 20-1000 and nominal p-value < 0.01. FIG. 5J shows pseudotime embedding of cells expressing marker genes. UMAPs showing the pseudotime distribution of cells expressing representative markers are provided. FIG. 5K provides a matrix plot showing a validated subset of DEGs of the oligodendrocyte related population from the AD vs. Control comparison. A plot is provided showing the row-wise scaled expression values of the top significantly altered (rank by p-value) DEGs in the 2766 gene dataset and the 64 genes validation result. FIG. 5L shows a subcluster of an oligodendrocyte and precursor cell (OPC) population. A zoomed-in diffusion map visualization of 11,265 oligodendrocytes and 1,269 OPCs identified from FIG. 2A is provided (top). Three subclusters of oligodendrocytes identified by the Leiden clustering are shown: Oligo1 (n = 9,594), Oligo2 (n = 910), Oligo3 (n = 761), and OPC (n = 1,269). A diffusion map embedding of subclusters of oligodendrocytes and OPCs for each sample (Control 8 month, TauPS2APP 8-month, Control 13 month, TauPS2APP 13 month) is also provided (bottom). FIG. 5M shows the expression levels of representative markers across different sub-clusters of oligodendrocytes and OPCs. A dot plot is provided showing both the mean gene expression (shading) of representative markers for each subpopulation and the percentage of cells expressing them (dot size). Gene expression values were normalized for each column. FIG. 5N shows a diffusion map pseudotime trajectory visualization of an oligodendrocyte-related cell population. A diffusion map visualization of pseudotime trajectory of oligodendrocytes and OPCs generated by Monocle 3 is provided (top). The colormap represents the pseudotime value. A corresponding pseudotime trajectory was plotted on the diffusion map embedding. Trajectory starting anchor was manually selected based on the OPC population. A black arrow highlights the bifurcation point on the trajectory related to the disease-associated gene expression changes. Cells from different samples were highlighted separately in diffusion map embedding (bottom). FIG. 5O provides a diffusion map showing different types of oligodendrocytes and OPCs identified in FIG. 5L, along with the trajectory. FIG. 5P provides a spatial cell map for the oligodendrocyte-related population of Control 13-month sample. Scale bar, 100 $\mu$m. FIG. 5Q provides a spatial cell map for the oligodendrocyte-related population of TauPS2APP 13-month sample. Scale bar, 100 $\mu$m. Two magnification sections (I, II) are provided showing zoomed-in regions highlighted in black boxes. Scale bar, 10 $\mu$m. Dashed black lines mark the boundaries between cortex, corpus callosum (cc), hippocampus, and alveus. FIG. 5R provides a boxplot showing the density (count per mm$^2$) of each oligodendrocyte sub-type and OPC in the cortex, corpus callosum, and hippocampus regions in Control and TauPS2APP mice at two time points. The plots show a significant enrichment of the Oligo2 in the corpus callosum (cc) and hippocampus region. FIG. 5S provides a stacked bar chart showing the density (count per mm$^2$) of each oligodendrocyte sub-cluster and OPC in each distance interval (0-10, 10-20, 20-30, 30-40, 40-50 $\mu$m) around the A$\beta$ plaque at 13 months. The overall cell density of each subpopulation in each area was included as the reference for comparison (all). FIG. 5T shows cell-type composition analysis of oligodendrocyte lineages in relation to p-Tau pathology. The tissue region was divided into a 20 $\mu$m x 20 $\mu$m grid in the TauPS2APP sample at 13 months ranked by p-Tau density. The blocks divided by the grid lines were ranked by the percentage of p-Tau positive pixels and grouped into 3 bins: zero (0%), low (1%-50%), high (50%-100%). The high p-Tau bin is further divided into two groups based on presence or absence of A$\beta$ plaques. A stacked bar plot showing the average number of cells per block for each oligodendrocyte related subtype is provided. FIG. 5U shows cell density and subtype composition of oligodendrocyte and OPC in the hippocampal alveus region. FIG. 5V provides a matrix plot showing the z-scores of spatial DEGs of oligodendrocytes across multiple distance intervals (0-10, 10-20, 20-30, 30-40, 40+ $\mu$m) from plaques.

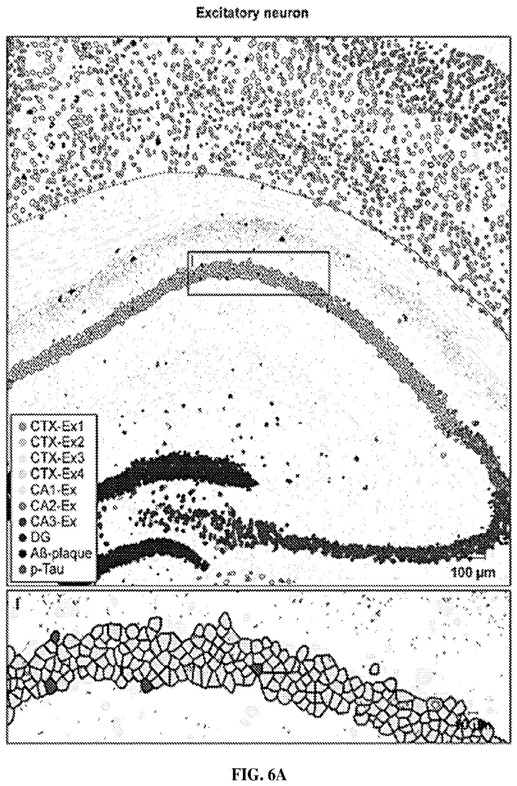

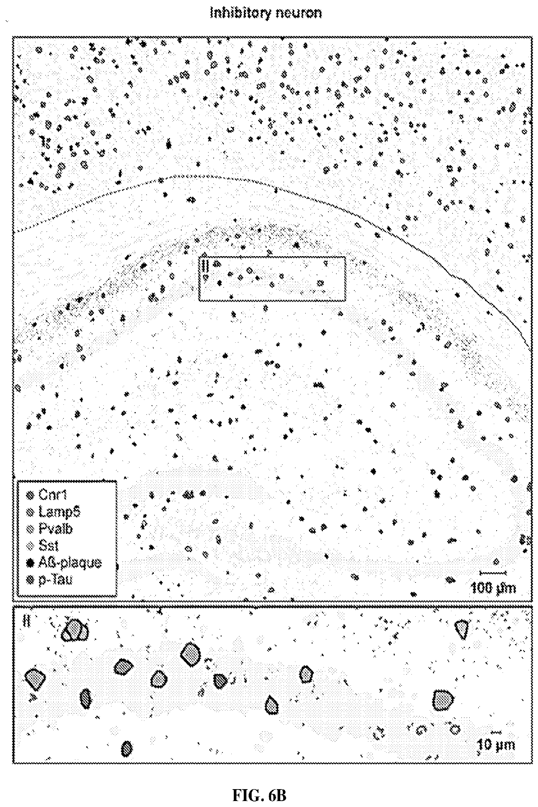

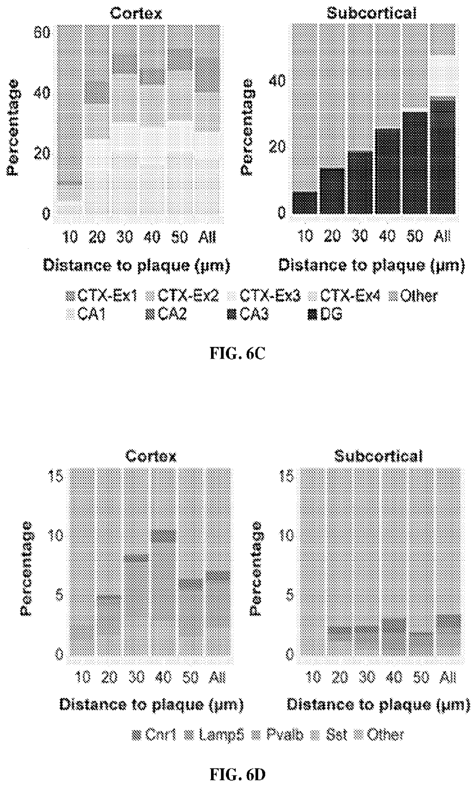

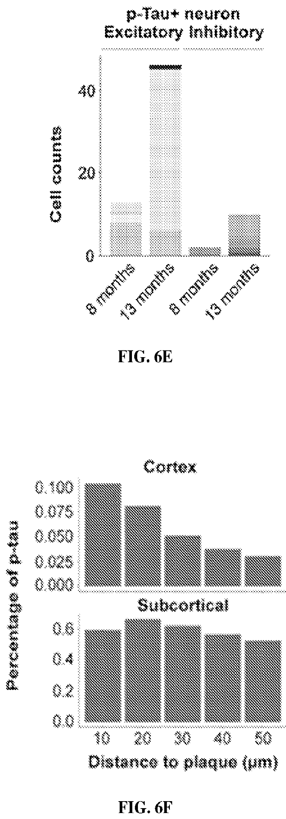

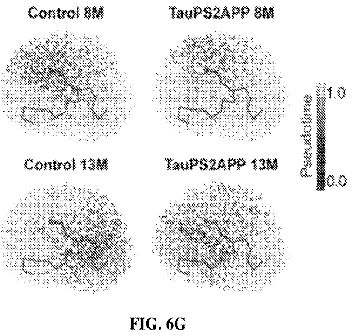

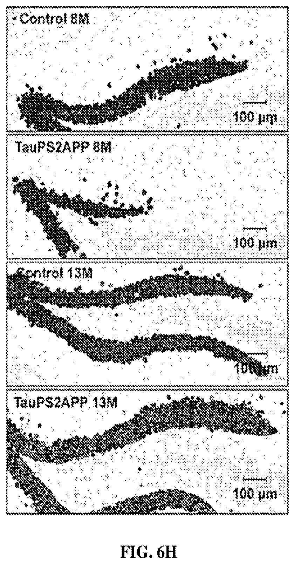

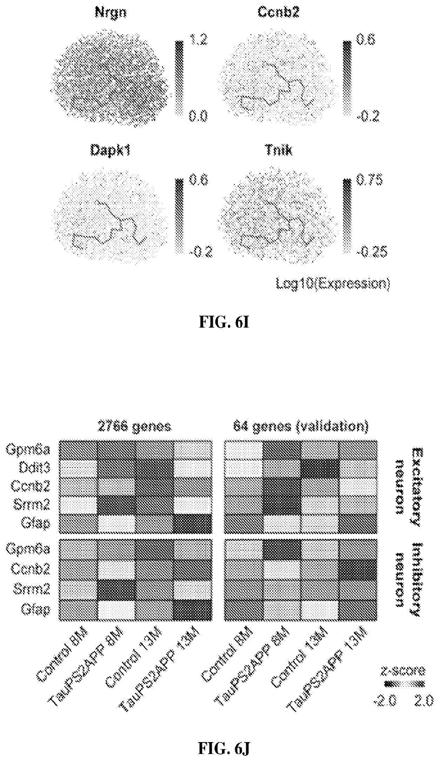

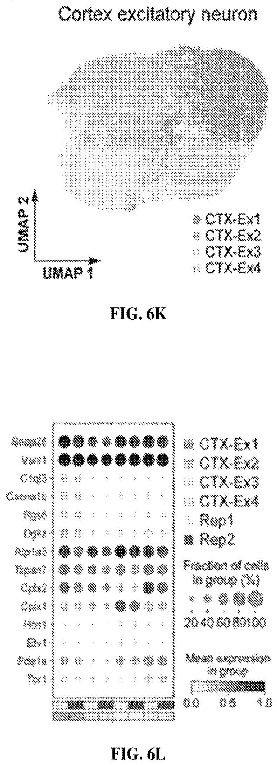

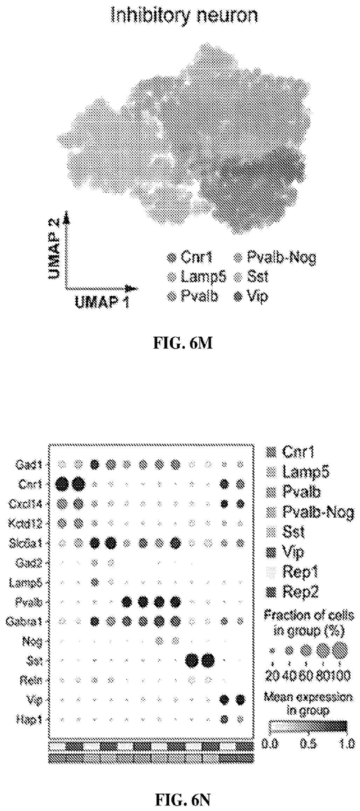

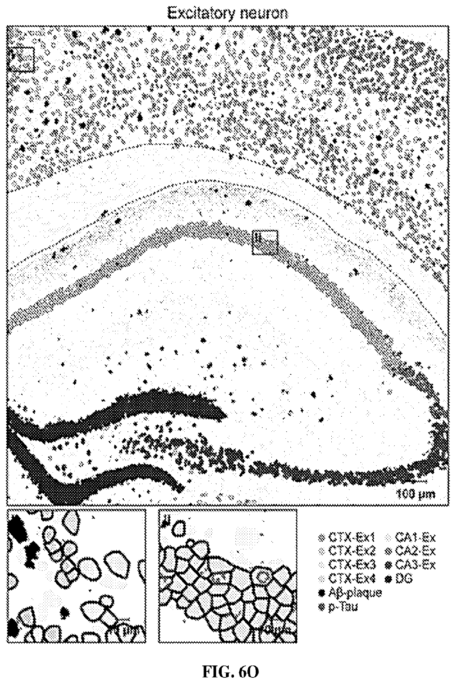

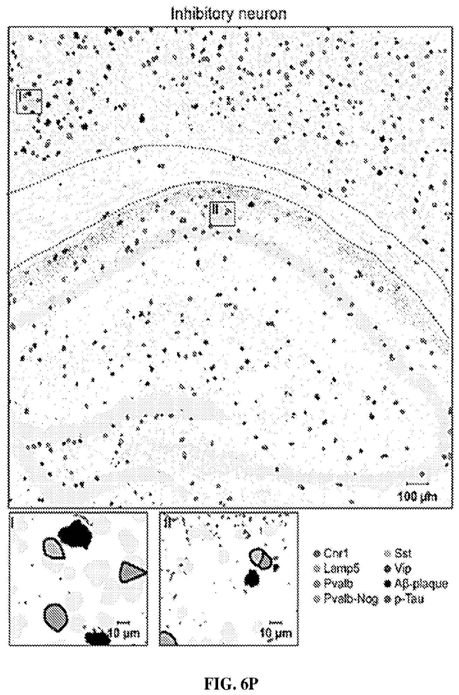

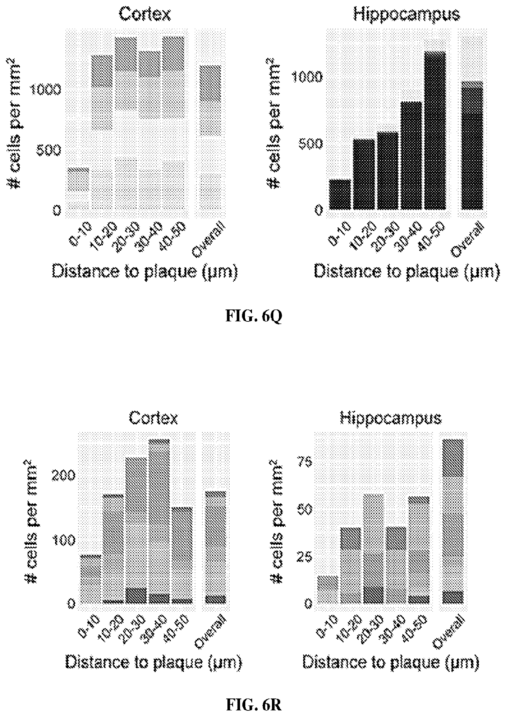

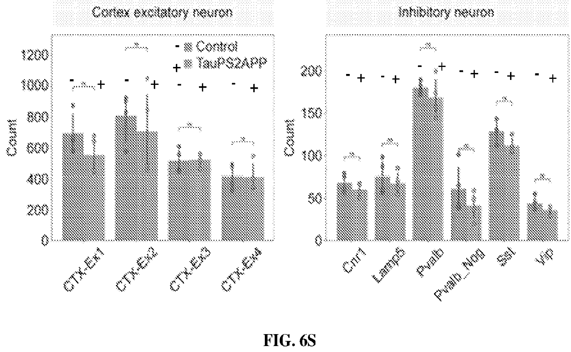

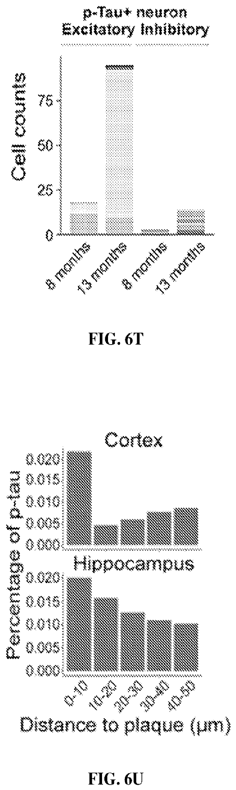

**FIGs. 6A-6U** show differential gene expression analysis and spatial information of neurons in TauPS2APP and control samples. FIG. 6A provides a spatial map for excitatory neuron population. Scale bar, 100 $\mu$m. (Total cell counts: CTX-Ex1: 2,292 , CTX-Ex2: 2,766, CTX-Ex3: 1,184, CTX-Ex4: 2,445, CA1: 2,754, CA2: 436, CA3: 1,878, DG: 4,377) of TauPS2APP 13-month sample with one magnification section of CA1 region on the bottom. Scale bar, 10 $\mu$m. p-Tau protein signals colored as gray on top of the cells. FIG. 6B provides a spatial map for an inhibitory neuron population. Scale bar, 100 $\mu$m. Cnr1: 421, Lamp5: 191, Pvalb: 864, Sst: 529 of TauPS2APP. The 13-month sample with one magnification section of the CA1 region is shown on the bottom. Scale bar, 10 $\mu$m. p-Tau protein signals are colored as gray on top of the cells. FIG. 6C shows excitatory neuron composition around plaque. A stacked bar chart is provided showing the percentage of each sub-cluster of the excitatory neuron population in each distance interval (0-10, 10-20, 20-30, 30-40, and 40-50 $\mu$m) around the A$\beta$ plaque from the cortex and subcortical region of the AD 13-month sample. In each region, an overall cell type composition was included as a reference. FIG. 6D shows inhibitory neuron composition around plaque. A stacked bar chart is provided showing the percentage of each sub-cluster of inhibitory neuron population in each distance interval (0-10, 10-20, 20-30, 30-40, and 40-50 $\mu$m) around the A$\beta$ plaque from the cortex and subcortical region of the AD 13-month sample. In each region, an overall cell type composition was included as a reference. FIG. 6E shows the cell type composition of p-Tau positive neurons. A stacked bar chart is provided showing the composition of tau positive excitatory neurons and inhibitory neurons in each AD sample under current threshold. FIG. 6F shows p-Tau signal quantification around plaques. p-Tau+ pixels (intensity > threshold) were quantified in each distance interval (0-10, 10-20, 20-30, 30-40, and 40-50 $\mu$m) around the A$\beta$ plaque from the cortex and subcortical region of the TauPS2APP 13-month sample. Values were normalized by ring area. FIG. 6G provides a UMAP of a Dentate gyrus (DG) cell population with pseudotime trajectory. Plots are provided showing low dimensional representations of the transcriptomics profile of a cell population in the dentate gyrus area of each sample generated by Monocle 3. Colormap represents the pseudotime value. A corresponding trajectory was constructed and plotted on the low dimensional embedding to illustrate the biological progression of the population. Plots for each sample were also included. FIG. 6H provides a spatial cell map shaded by pseudotime for DG population. Scale bar, 100 $\mu$m. FIG. 6I shows DEGs on pseudotime embedding. UMAPs showing the expression of four significantly altered genes from DEG analysis of DG neurons on the pseudotime embedding are provided. The shading scale of the raw counts was adjusted by $\log_{10}$ scale. FIG. 6J provides a matrix plot showing a validated subset of DEGs of the excitatory and inhibitory neuron population from the AD vs. Control comparison. A plot is provided showing the row-wise scaled expression values of the top significantly altered (ranked by p-value) DEGs in the 2,766 gene dataset and the 64 genes validation result. FIG. 6K shows subclustering of a cortex excitatory neuron cell population. UMAP visualization showing four subclusters of cortex excitatory neuron cells identified by Leiden clustering: CTX-Ex1 (n = 4,972), CTX-Ex2 (n = 6,049), CTX-Ex3 (n = 4,158), and CTX-Ex4 (n = 3,304). FIG. 6L shows the expression level of representative gene markers among different subclusters of cortex excitatory neuron cells. Dot plots showing both the mean gene expression (shading) of representative markers for each subpopulation and the percentage of cells expressing them (dot size) are provided. Gene expression values were normalized for each column. FIG. 6M shows subclustering of an inhibitory neuron cell population. UMAP visualization showing six subclusters of inhibitory cells identified by Leiden clustering: Cnr1 (n = 512), Lamp5 (n = 573), Pvalb (n = 1,392), Pvalb-Nog (n = 410), Sst (n = 959), and Vip (n = 317). FIG. 6N shows the expression level of representative gene markers among different subclusters of inhibitory neuron cells. A dot plot showing both the mean gene expression (shading) of representative markers for each subpopulation and the percentage of cells expressing them (dot size) is provided. Gene expression values were normalized for each column. FIG. 6O provides a spatial map of A$\beta$ plaque and p-Tau for an excitatory neuron population in the TauPS2APP 13-month sample (top). Scale bar, 100 $\mu$m. Total cell counts: CTX-Ex1: 2,292 , CTX-Ex2: 2,766, CTX-Ex3: 1,184, CTX-Ex4: 2,445, CA1: 2,754, CA2: 436, CA3: 1,878, DG: 4,377) of TauPS2APP 13-month sample with high magnification views of sections of CA1 region indicated in black boxes is shown on the bottom. Scale bar, 10 $\mu$m. p-Tau protein signals colored as gray on top of the cells. FIG. 6P provides a spatial map of A$\beta$ plaque and p-Tau for an inhibitory neuron population in the TauPS2APP 13-month sample (top). Scale bar, 100 $\mu$m. Cnr1: 421, Lamp5: 191, Pvalb: 864, Sst: 529 of TauPS2APP. The 13-month sample with high magnification views of sections of the CA1 region indicated in black boxes is shown on the bottom. Scale bar, 10 $\mu$m. p-Tau protein signals are colored as gray on top of the cells. FIG. 6Q shows excitatory neuron composition around plaque. A stacked bar chart is provided showing the density (count per mm$^2$) of each sub-cluster of the excitatory neuron population from different brain regions in each distance interval (0-10, 10-20, 20-30, 30-40, and 40-50 $\mu$m) around the A$\beta$ plaque from the cortex and subcortical region of the AD TauPS2APP 13-month sample. In each region, the overall cell density of each cell type subpopulation was included as a reference comparison standard. FIG. 6R shows inhibitory neuron composition around plaque. A stacked bar chart is provided showing the density (count per mm$^2$) of each sub-cluster of inhibitory neuron population from different brain regions in each distance interval (0-10, 10-20, 20-30, 30-40, and 40-50 $\mu$m) around the A$\beta$ plaque from the cortex and subcortical region of the AD TauPS2APP 13-month sample. In each region, the overall cell density of each cell type subpopulation was included as a reference comparison standard. FIG. 6S provides a barplot showing the density (count per mm$^2$) of each cortex excitatory neuron and inhibitory neuron sub-type in control and TauPS2APP mice. FIG. 6T shows p-Tau signal quantification around plaques. p-Tau+ pixels (intensity > threshold) were quantified in each distance interval (0-10, 10-20, 20-30, 30-40, and 40-50 $\mu$m) around the A$\beta$ plaque from the cortex and subcortical region of the TauPS2APP 13-month sample. Values were normalized by ring area. Y-axis values were normalized by the total p-Tau signal of each brain region. FIG. 6U shows the cell type composition of p-Tau positive neurons. A stacked bar chart is provided showing the composition of p-Tau positive excitatory neurons and inhibitory neurons in each AD sample (control and TauPS2APP) under current threshold at two time points defined by the ratio of tau positive pixels to the area of each cell.

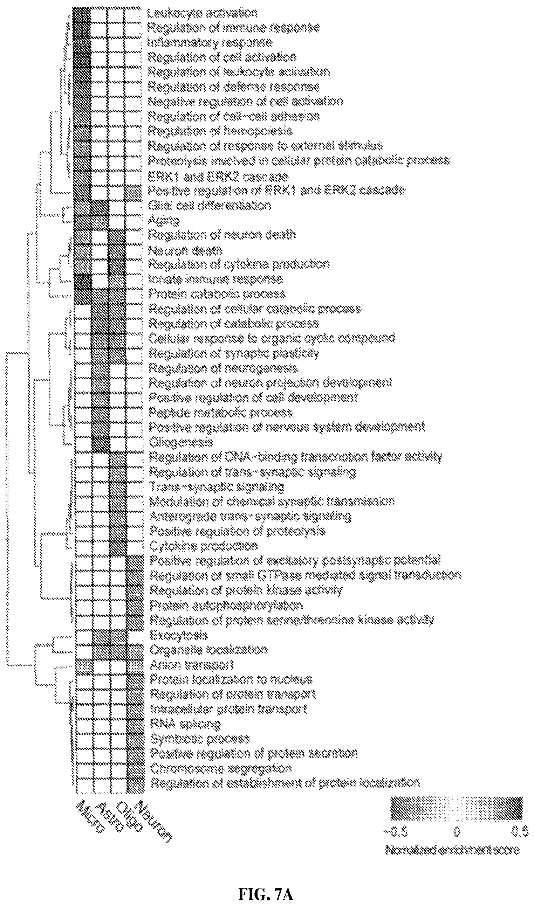

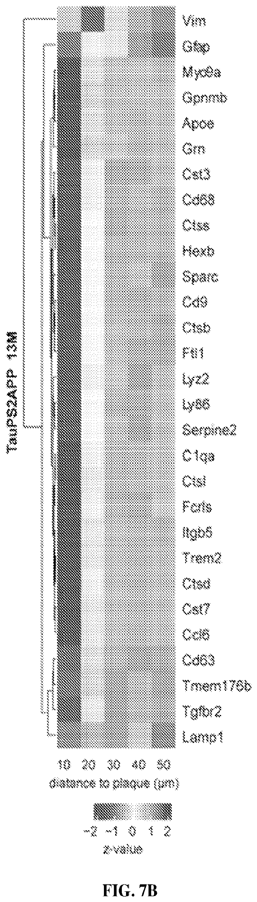

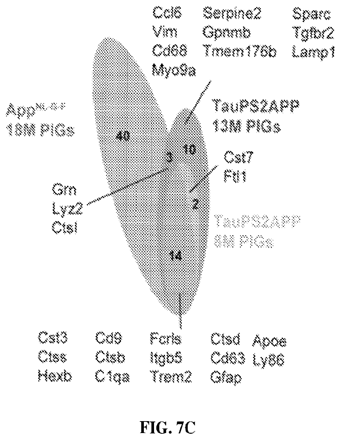

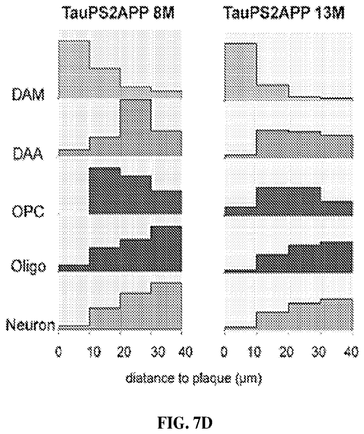

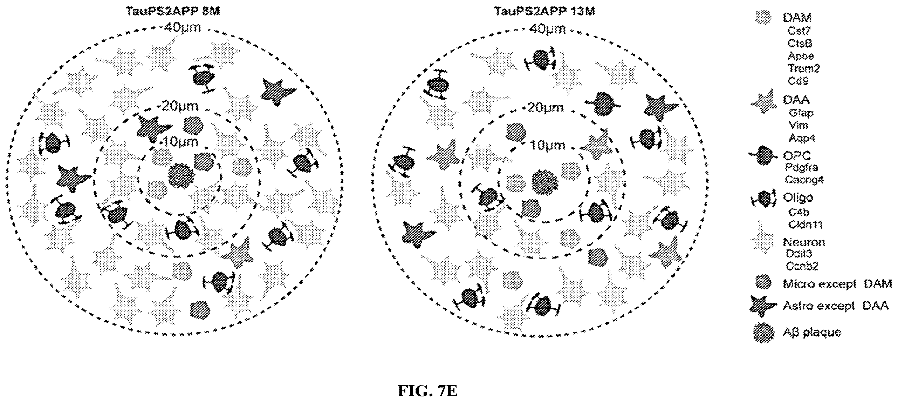

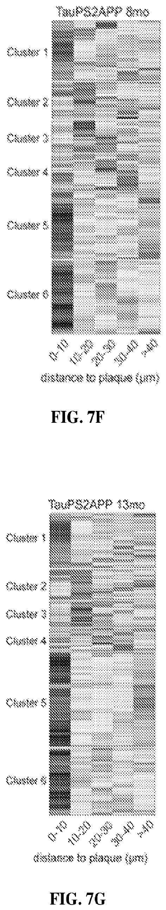

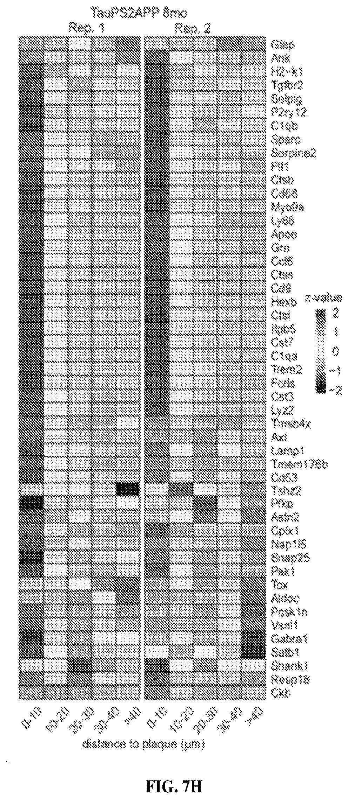

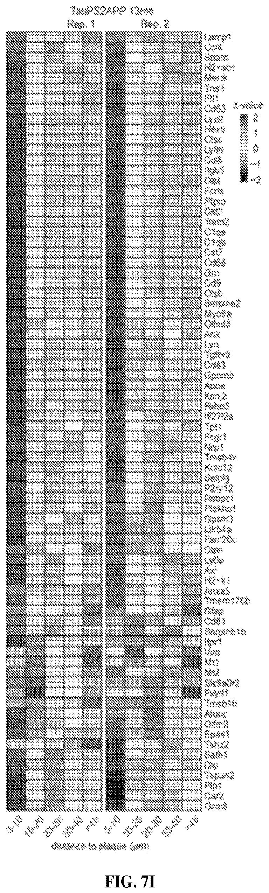

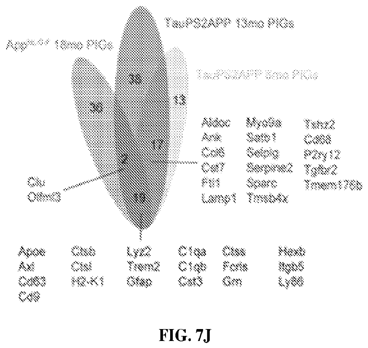

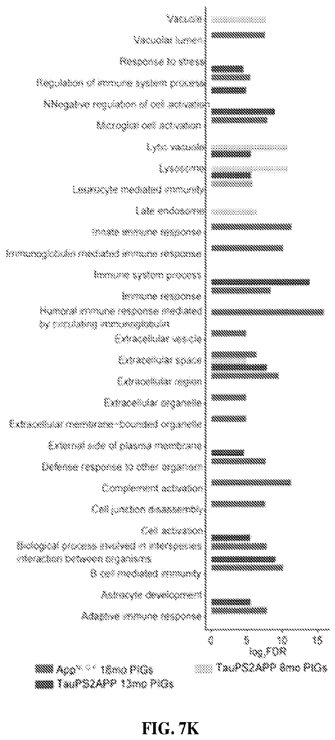

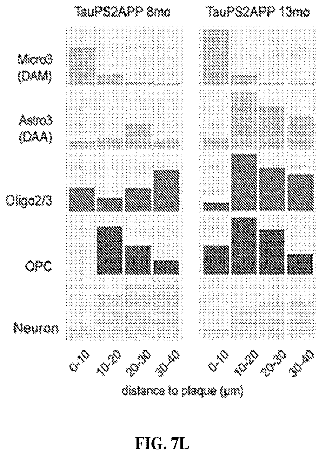

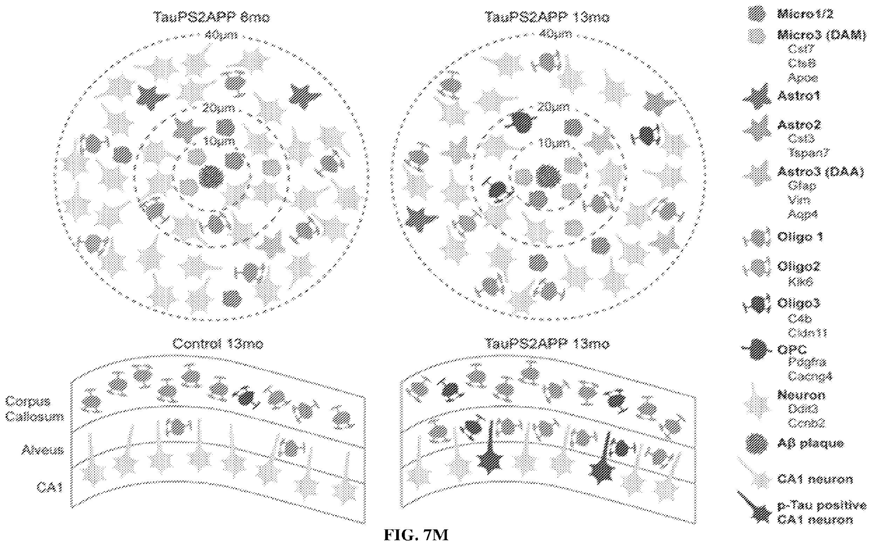

**FIGs. 7A-7M** show integrated pathway and spatial analysis of disease-associated cell types. FIG. 7A provides a GSEA heatmap showing the significant (nominal p-value < 0.05) biological process related terms enriched in DEGs of each cell type of interest in the AD 13-month sample. Terms are filtered by term size (20-1000). The shading of the tiles represents the normalized enrichment score. FIG. 7B provides a matrix plot showing genes upregulated (LogFC > 0.1) in the near-plaque regions of AD 13-month samples from cells within a 25 $\mu$m ring compared to cells outside the 25 $\mu$m ring (shaded by row-wise z-score). FIG. 7C provides a Venn diagram highlighting the overlap of plaque-induced genes (PIGs) in the AD 13-month sample with PIGs in the AD 8-month sample and previously reported PIGs in 18-month AppNL-G-F mice. FIG. 7D provides spatial histograms of disease-associated microglia (DAM), disease-associated astrocyte (DAA), Oligodendrocyte, OPC, and neuron cell populations around A$\beta$ plaque in the AD 8-month sample (left) and 13-month sample (right). On the histogram, cells were counted in 10 $\mu$m bins in a 2D max-projection from the edge of each plaque. FIG. 7E provides a schematic diagram of DAM, DAA, Oligodendrocyte, OPC, Microglia (except DAM), Astrocyte (except DAA), and neuron cell populations around Aβ plaque in 8-month AD mouse (left) and 13-month mouse brain. The number of cells in the schematic diagram is the approximate value of the calculated average cell number of each cell type in each ring. FIGs. 7F-7G provide a matrix plot showing the gene clustering results in each distance interval (0-10, 10-20, 20-30, 30-40, >40 μm) around the Aβ plaque in TauPS2APP 8-month sample (FIG. 7F) and TauPS2APP 13-month sample (FIG. 7G). Shaded by row-wise z-score. FIGs. 7H-7I provide matrix plots showing the Plaque Induced Genes (Enriched in 0-40 interval, adjusted p-value < 0.01) in each distance interval (0-10, 10-20, 20-30, 30-40, >40 μm) around the Aβ plaque in TauPS2APP 8-month sample (FIG. 7H) and TauPS2APP 13-month sample (FIG. 7I). Colored by row-wise z-score. FIG. 7J shows a Venn diagram highlighting the overlap of SDEGs in the TauPS2APP 8- and 13-month samples with SDEGs in TauPS2APP and previously reported PIGs in 18-month $App_{NL-G-F}$ mice. FIG. 7K shows the significantly enriched GO terms of SDEGs in the TauPS2APP 8- and 13-month samples and previously reported PIGs. FIG. 7L provides spatial histograms of Micro3 disease-associated microglia (DAM), Astro3 disease-associated astrocyte (DAA), Oligo2/3 Oligodendrocyte, OPC, and neuronal cell populations around Aβ plaque in the AD TauPS2APP 8-month sample (left) and 13-month sample (right). On the histogram, cells were counted in 10 μm bins in a 2D max-projection from the edge of each plaque. FIG. 7M provides a schematic diagram of different cell types (e.g., DAM, DAA, Oligodendrocyte, OPC, Microglia (except DAM), Astrocyte (except DAA), and neuron cell populations) around Aβ plaque and oligodendrocyte subtypes in hippocampal alveus in 8-month AD mouse (left) and 13-month mouse brain. The number of cells in the schematic diagram is the approximate ratio of cell number of each cell type in each ring.

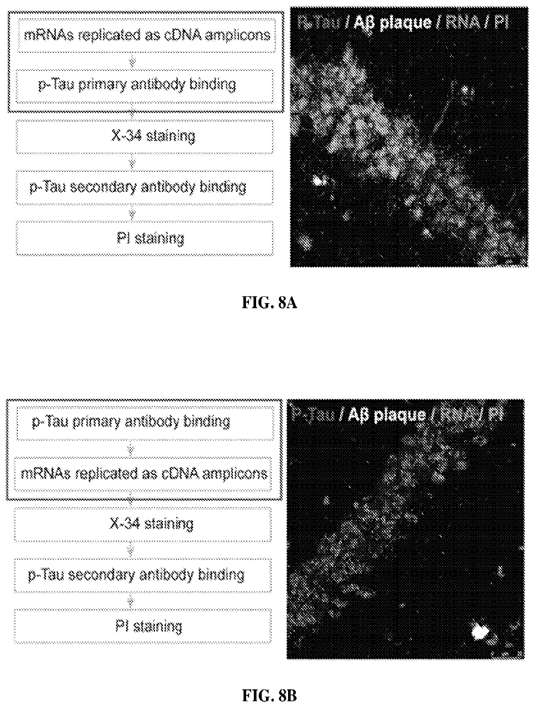

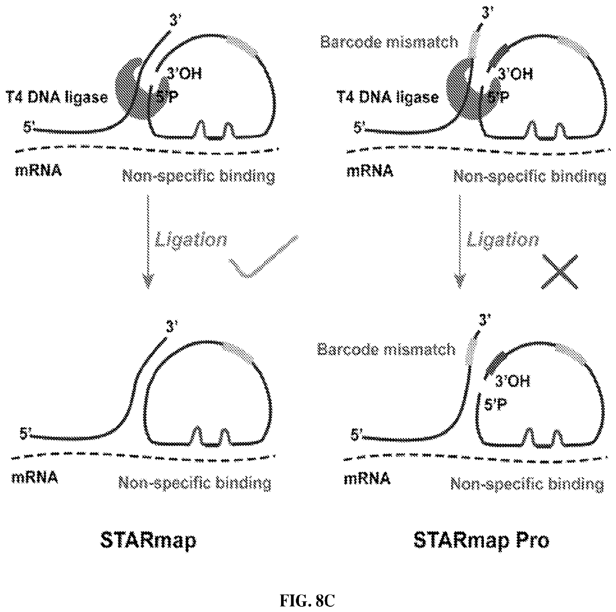

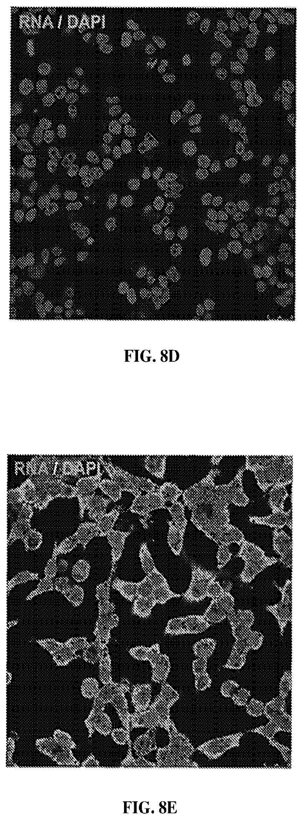

**FIGs. 8A-8E** show the development of the STARmap Pro method. FIG. 8A shows the STARmap Pro procedure, in which p-Tau primary antibody staining was performed after mRNA *in situ* hybridization and amplification. The imaging results showed strong signals from both cDNA amplicons and proteins. FIG. 8B shows an alternative procedure where p-Tau primary antibody staining was conducted before mRNA *in situ* hybridization and amplification. The imaging results showed much weaker signal from cDNA amplicons, suggesting RNA degradation was occurring during antibody incubation and washing steps. FIG. 8C provides a schematic diagram explaining the enhanced specificity of STARmap Pro compared with the previous STARmap method. In the original STARmap method design, DNA probes of all genes share an identical DNA sequence at the ligation junction, so the padlock probes could still be circularized and amplified non-specifically when there was a primer probe nearby. This could potentially lead to false signals when using the previous STARmap method. In STARmap Pro, an additional 5-nt barcode is placed at the ligation site and can prevent the ligation of non-specific binding probes and thus improve specificity. FIGs. 8D and 8E show imaging results of STARmap Pro using SNAIL probes with (FIG. 8D) and without (FIG. 8E) barcode mismatch near the ligation site. DAPI staining of cell nuclei and fluorescent DNA probe staining of all cDNA amplicons are shown according to the legend provided.

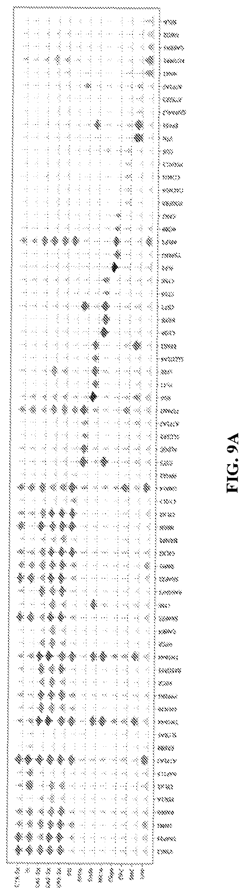

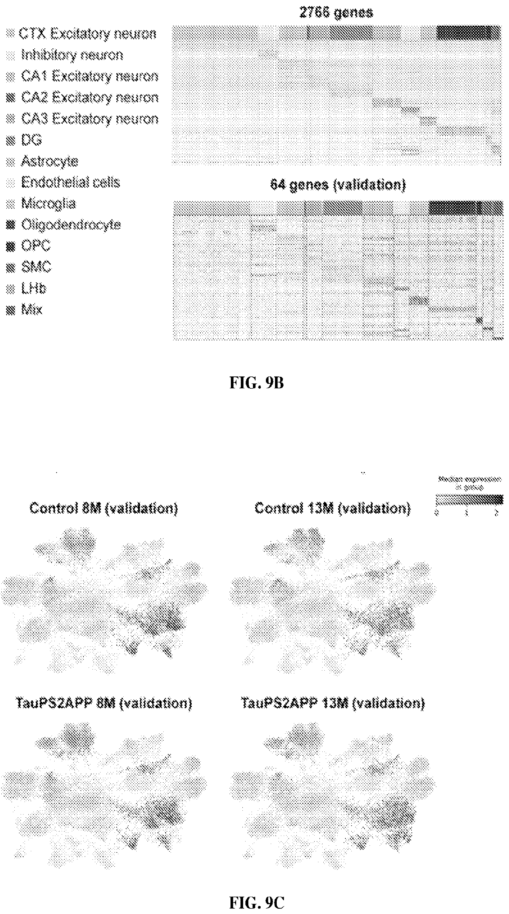

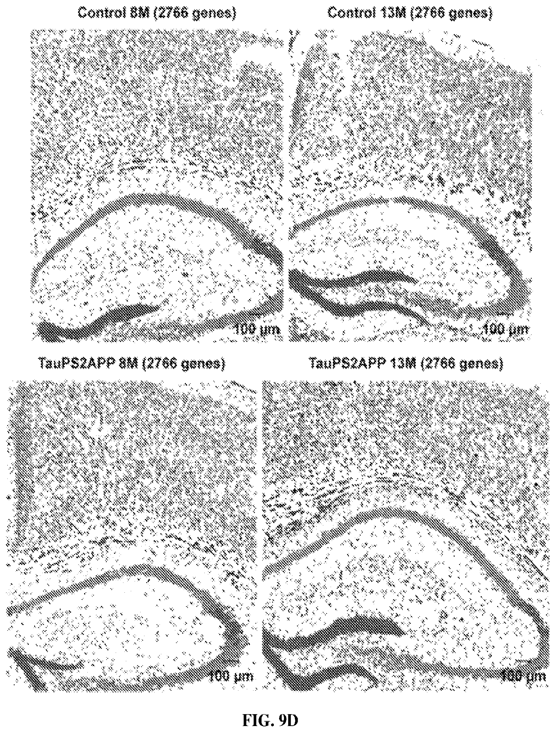

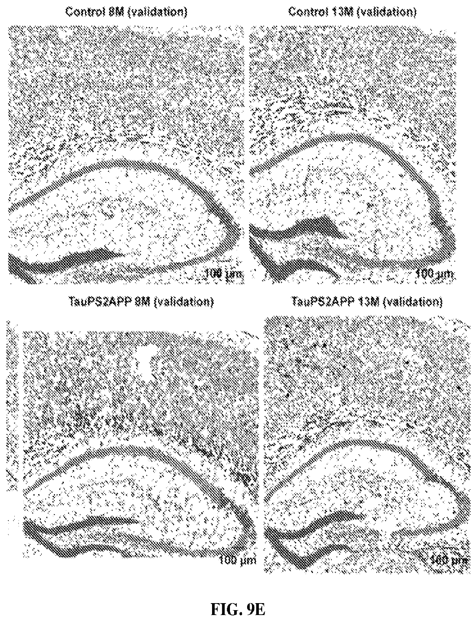

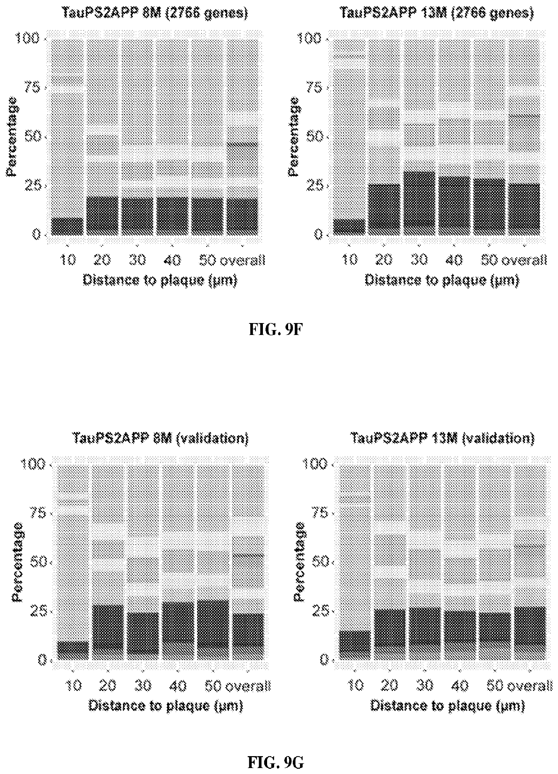

**FIGs. 9A-9G** show top-level cell type classification results of all samples. FIG. 9A provides a stacked violin plot for representative gene markers aligned with each top-level cell type of the 2,766 gene dataset. FIG. 9B provides gene expression heatmaps for representative markers aligned with each top-level cell type of 2,766- and 64-gene datasets. The 64-gene data successfully recapitulated the top-level clustering results. Expression for each gene is z-scored across all genes in each cell. FIG. 9C provides a Uniform Manifold Approximation (UMAP) plot visualizing a non-linear dimensionality reduction for the transcriptomic profiles of 36,625 cells from four (4) samples of the validation dataset. A plot is provided showing the 13 top-level clusters (Cortex excitatory neuron (CTX-Ex, 8,640 cells), Inhibitory neuron (In, 2,858 cells), CA1 excitatory neuron (CA1-Ex, 2,967 cells), CA2 excitatory neuron (CA2-Ex, 331 cells), CA3 excitatory neuron (CA3-Ex, 1751 cells), Dentate Gyrus (DG, 4,560 cells), Astrocyte (Astro, 3,423 cells), Endothelial cells (Endo, 1,661 cells), Microglia (Micro, 2,183 cells), Oligodendrocytes (Oligo, 5,268 cells), Oligodendrocyte precursor cells (OPC, 711 cells), Smooth muscle cells (SMC, 1,146 cells), and Mixed unidentified cells (Mix, 1126 cells). FIGs. 9D and 9E show spatial atlas of the top-level cell types in the cortex and hippocampus regions of four (4) samples in the 2,766-gene dataset (FIG. 9D) and 64-gene dataset (FIG. 9E). Scale bars, 100 μm. FIGs. 9F and 9G show cell type composition around Aβ plaque at different distance intervals in both 8- and 13-month samples of the 2,766-gene dataset (F) and 64-gene validation dataset (G). A stacked bar plot is provided showing the percentage of each top-level cell type in each distance interval (0-10, 10-20, 20-30, 30-40, and 40-50 μm) around the Aβ plaque. In each region, an overall cell type composition was included as a reference.

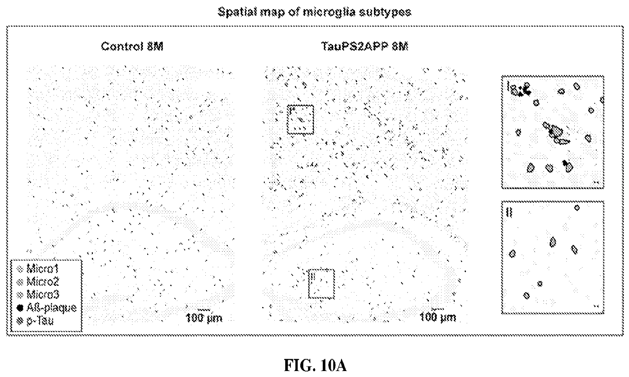

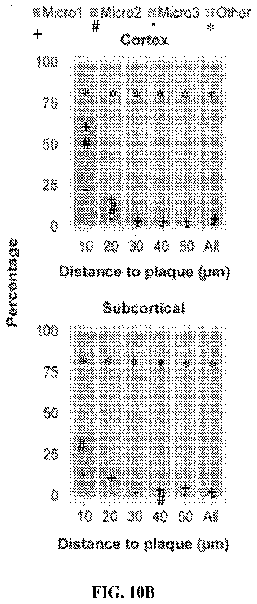

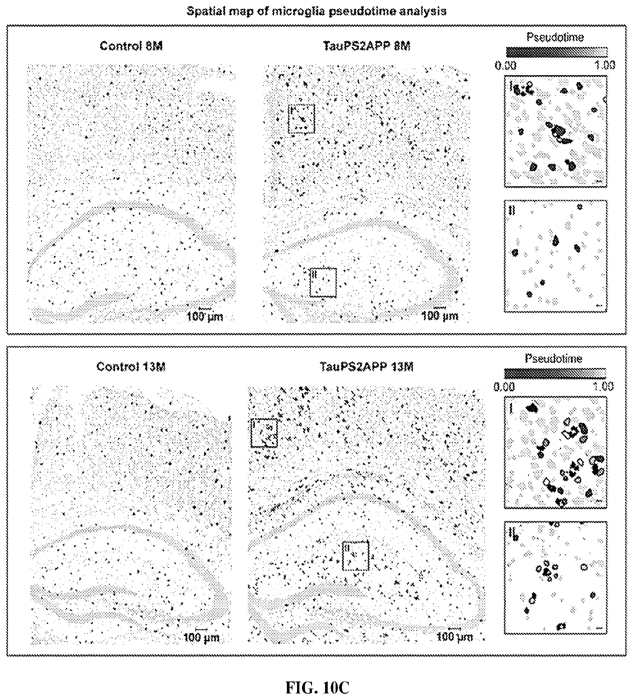

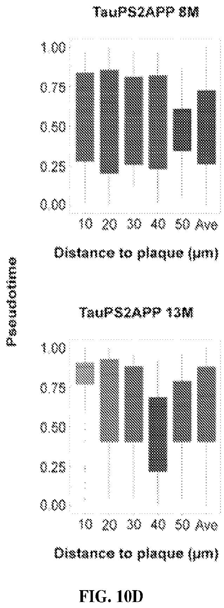

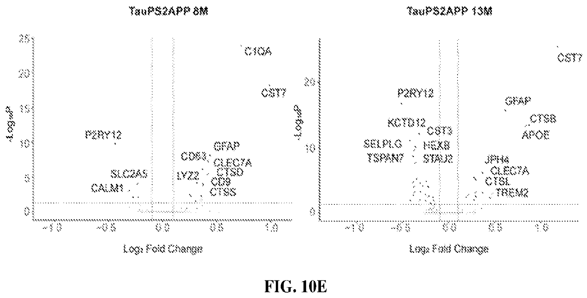

**FIGs. 10A-10E** show additional gene expression and spatial information of a Microglia population. FIG. 10A provides a spatial map of microglia subtypes in the 8-month control and TauPS2APP samples. Scale bar, 100 μm. Two magnification sections (I, II) are shown on the right side. Scale bar, 10 μm. FIG. 10B shows cell type composition around Aβ plaque in different distance intervals for the TauPS2APP sample in the 8-month sample. A stacked bar plot is provided showing the percentage of each microglia sub population in each distance interval (0-10, 10-20, 20-30, 30-40, and 40-50 μm) around the Aβ plaque. In each region, an overall cell type composition was included as a reference. FIG. 10C provides a spatial map of microglia colored by pseudotime. Scale bar, 100 μm. Two magnification sections (I, II) are shown on the right side. Scale bar, 10 μm. FIG. 10D shows pseudotime values of microglia in relation to plaque. Box plots are provided showing the distribution of the pseudotime for microglia in each distance interval (0-10, 10-20, 20-30, 30-40, and 40-50 $\mu$m) around the A$\beta$ plaque. A distribution of all the microglia was included as a reference. Box shading represents median pseudotime. FIG. 10E provides volcano plots for microglia differential expression. Plots show gene expression of microglia across AD and control samples in the 8- and 13-month samples (y-axis: -log adjusted p-value, x-axis: average log fold change). Differentially expressed genes (adjusted p-value < 0.05, absolute value of logFC > 0.1) have a positive fold-change value (up-regulated) or a negative fold-change value (down-regulated).

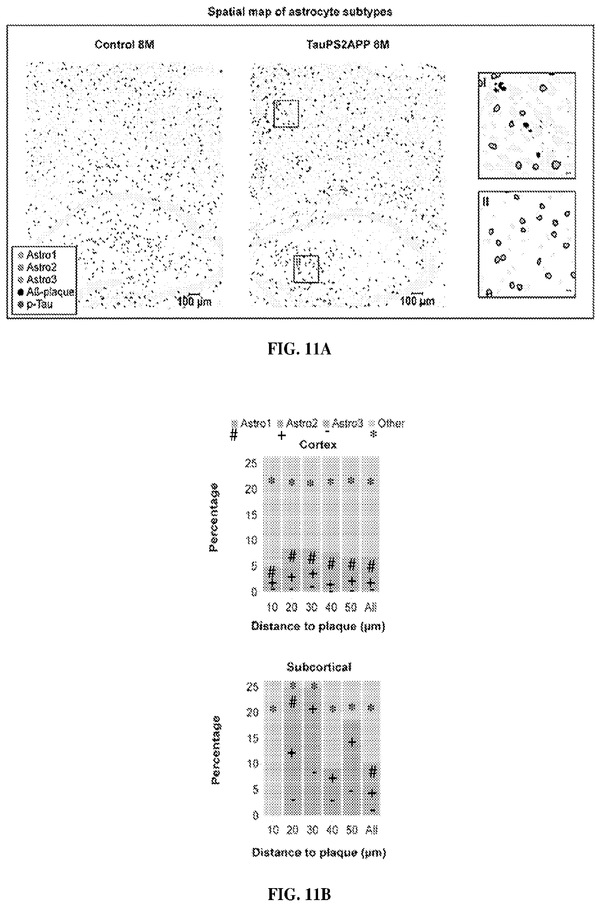

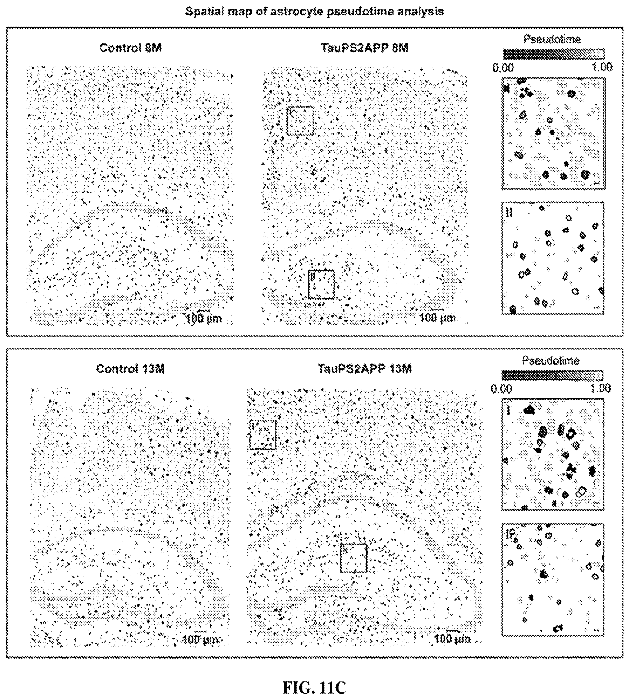

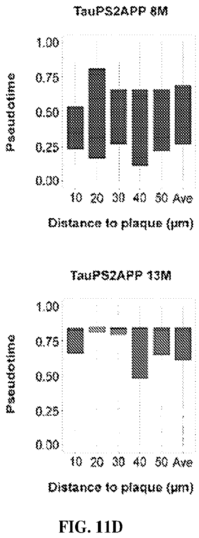

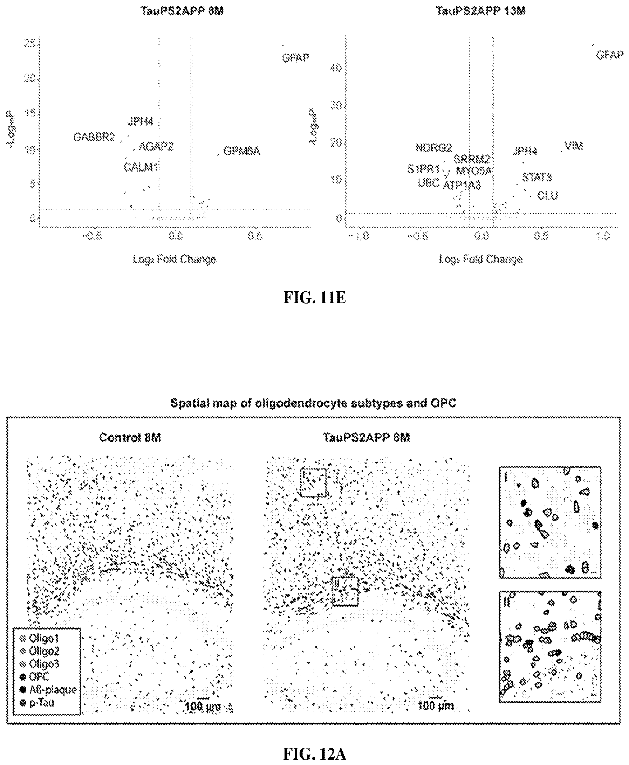

**FIGs. 11A-11E** show additional gene expression and spatial information for an astrocyte cell population. FIG. 11A provides a spatial map of astrocyte subtypes in Control and TauPS2APP samples in the 8-month sample. Scale bar, 100 $\mu$m. Two magnification sections (I, II) are shown on the right side. Scale bar, 10 $\mu$m. FIG. 11B shows cell type composition around A$\beta$ plaque in different distance intervals for the TauPS2APP 8-month sample. A stacked bar plot is provided showing the percentage of each astrocyte subpopulation at each distance interval (0-10, 10-20, 20-30, 30-40, and 40-50 $\mu$m) around the A$\beta$ plaque. In each region, an overall cell type composition was included as a reference. FIG. 11C provides a spatial map of astrocytes shaded by pseudotime for the astrocyte cell population. Scale bar, 100 $\mu$m. Two magnification sections (I, II) are shown on the right side. Scale bar, 10 $\mu$m. FIG. 11D shows pseudotime in relation to plaque. Box plots are provided showing the distribution of the pseudotime for astrocytes at each distance interval (0-10, 10-20, 20-30, 30-40, and 40-50 $\mu$m) around the A$\beta$ plaque. A distribution of all the astrocytes was included as a reference. FIG. 11E provides volcano plots showing differential gene expression in astrocytes. Plots are provided showing gene expression of astrocytes across AD and control 8- and 13-month samples (y-axis: -log adjusted p-value, x-axis: average log fold change). Differentially expressed genes (adjusted p-value < 0.05, absolute value of logFC > 0.1) have positive fold-change values (up-regulated) or negative fold-change values (down-regulated).

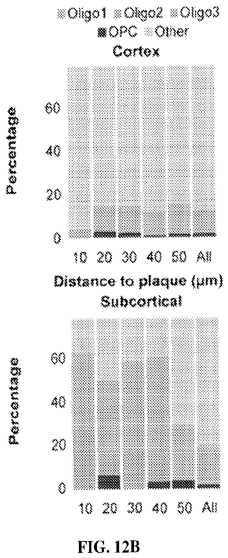

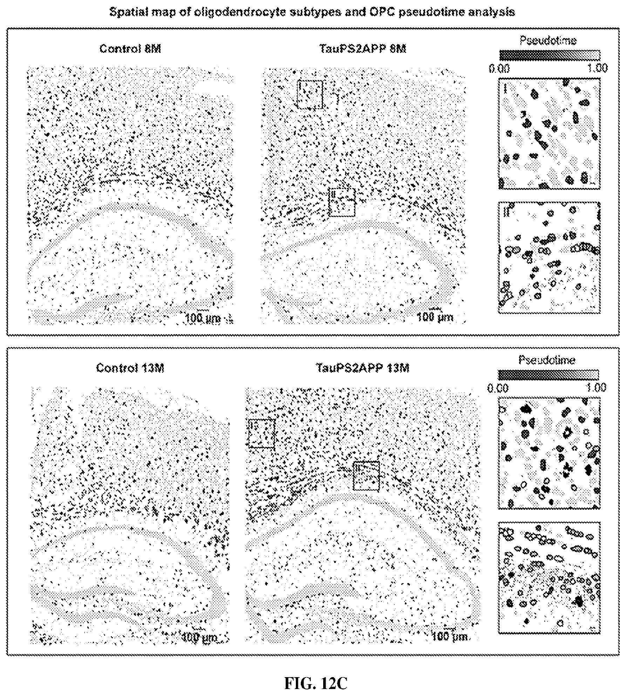

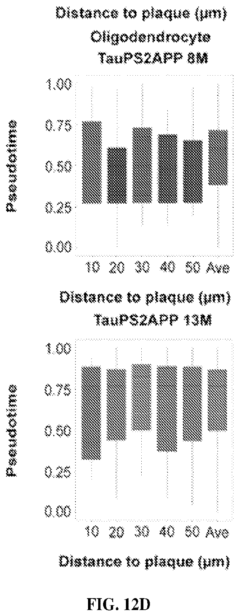

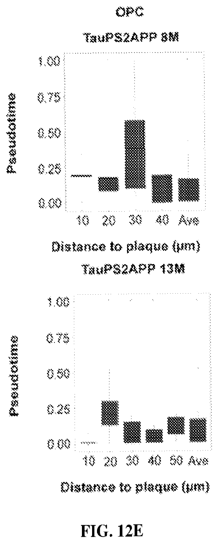

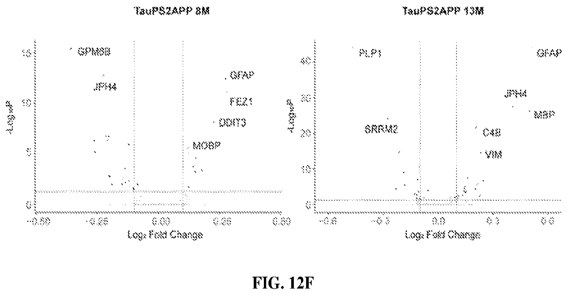

**FIGs. 12A-12F** show additional gene expression and spatial information of oligodendrocyte and OPC cell populations. FIG. 12A provides a cell-resolved spatial map for the oligodendrocyte and OPC population of both Control and TauPS2APP 8-month samples. Scale bar, 100 $\mu$m. Two magnification sections (I, II) are shown on the right side. Scale bar, 10 $\mu$m. FIG. 12B shows cell type composition around A$\beta$ plaque in different distance intervals for the TauPS2APP 8-month sample. A stacked bar plot is provided showing the percentage of each oligodendrocyte subpopulation and OPC at each distance interval (0-10, 10-20, 20-30, 30-40, and 40-50 $\mu$m) around the A$\beta$ plaque. In each region, an overall cell type composition was included as a reference. FIG. 12C provides a spatial map shaded by pseudotime for the oligodendrocyte-related cell population. Scale bar, 100 $\mu$m. Two magnification sections (I, II) are shown on the right side. Scale bar, 10 $\mu$m. FIG. 12D shows pseudotime in relation to plaque. Box plots are provided showing the distribution of the pseudotime for oligodendrocytes at each distance interval (0-10, 10-20, 20-30, 30-40, and 40-50 $\mu$m) around the A$\beta$ plaque. A distribution of all oligodendrocytes was included as a reference. FIG. 12E shows pseudotime in relation to plaque. Box plots are provided showing the distribution of the pseudotime for OPCs at each distance interval (0-10, 10-20, 20-30, 30-40, and 40-50 $\mu$m) around the A$\beta$ plaque. A distribution of all OPCs was included as a reference. FIG. 12E provides volcano plots showing differential expression in oligodendrocytes. Plots are provided showing gene expression of oligodendrocytes across AD and control 8- and 13-month samples (y-axis: -log adjusted p-value, x-axis: average log fold change). Differentially expressed genes (adjusted p-value < 0.05, absolute value of logFC > 0.1) have positive fold-change values (up-regulated) or negative fold-change values (down-regulated).

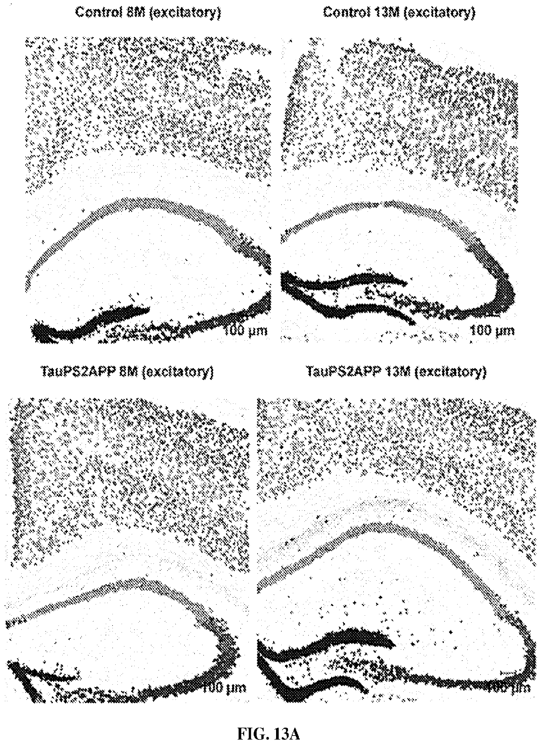

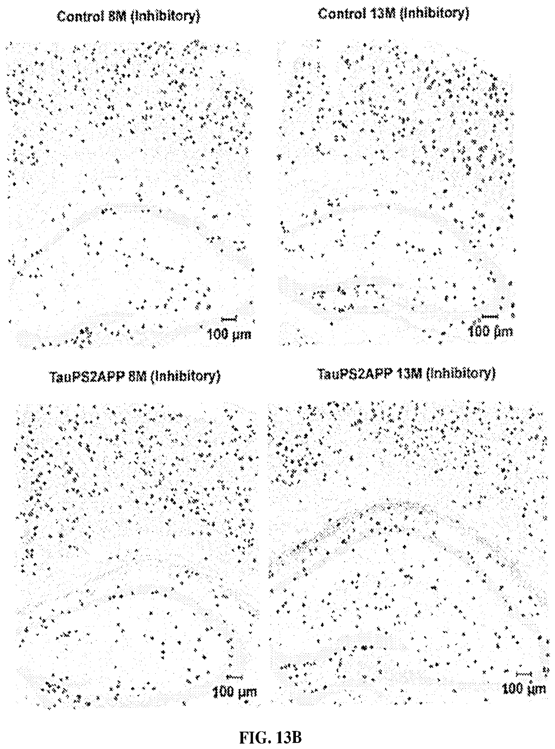

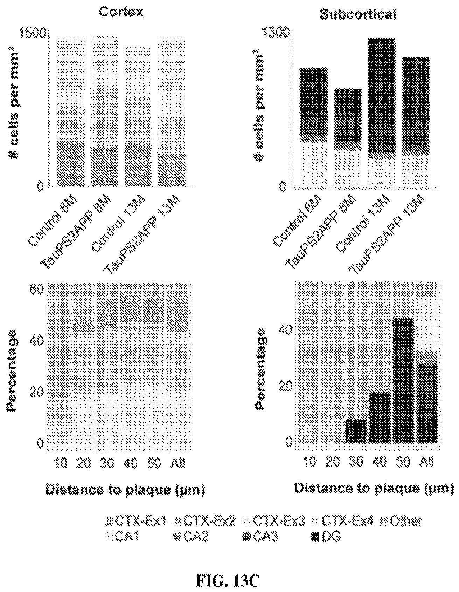

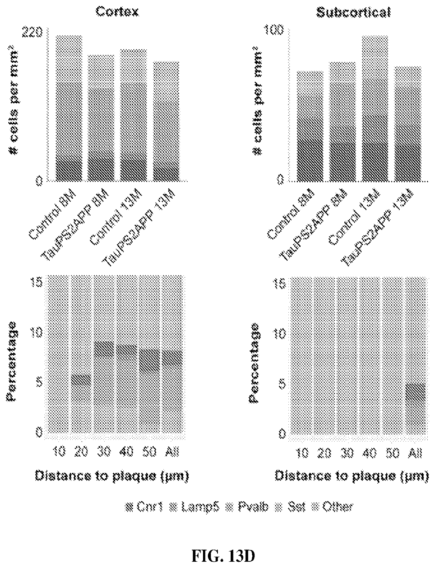

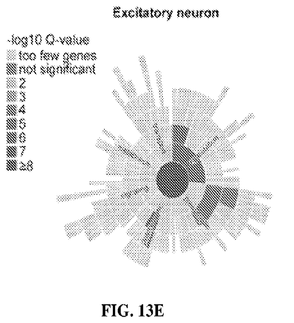

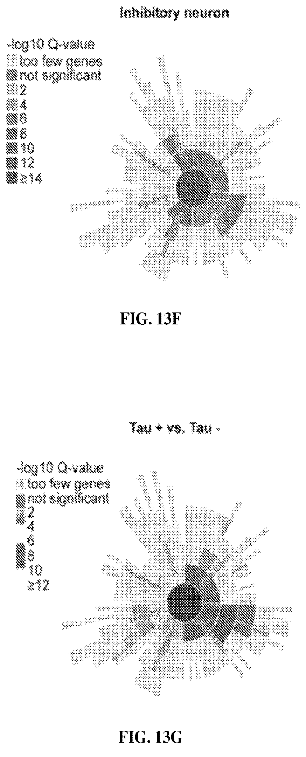

**FIGs. 13A-13G** show additional gene expression and spatial information for a population of neurons. FIG. 13A provides spatial maps for excitatory neuron populations of four samples. Scale bar, 100 $\mu$m. FIG. 13B provides spatial maps for inhibitory neuron populations of four samples. Scale bar, 100 $\mu$m. FIG. 13C shows region-wise cell density and cell type composition around plaque of excitatory neurons. A stacked bar chart showing the density (count per mm$^2$) of each excitatory neuron sub-cluster in both the cortex and subcortical region for each sample is provided (top). The shading in the bar plots corresponds to the cell type legend in FIG. 13A. A stacked bar chart showing the percentage of each excitatory neuron sub-cluster at each distance interval (0-10, 10-20, 20-30, 30-40, and 40-50 $\mu$m) around the A$\beta$ plaque is also provided (bottom). In each region, an overall cell type composition was included as a reference. FIG. 13D shows region-wise cell density and cell type composition around plaque of inhibitory neurons. A stacked bar chart showing the density (count per mm$^2$) of each inhibitory neuron sub-cluster in both the cortex and subcortical region for each sample is provided (top). The shading in the bar plots correspond to the cell type legend in FIG. 13B. A stacked bar chart showing the percentage of each inhibitory neuron sub-cluster at each distance interval (0-10, 10-20, 20-30, 30-40, and 40-50 $\mu$m) around the A$\beta$ plaque is also provided (bottom). In each region, an overall cell type composition was included as a reference. FIGs. 13E-13G show synaptic gene ontology term enrichment of DEGs from excitatory neurons, inhibitory neurons, and CA1 cells with Tau pathology using SynGO. Shading of the sunburst plot represents enrichment -log$_{10}$ Q-value at 1% FDR.

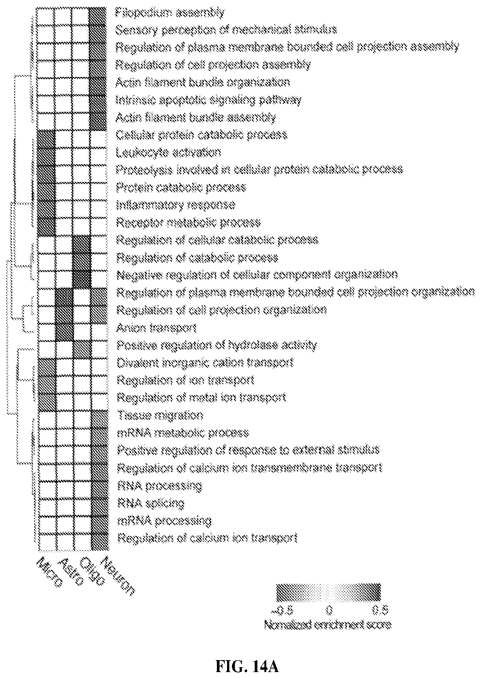

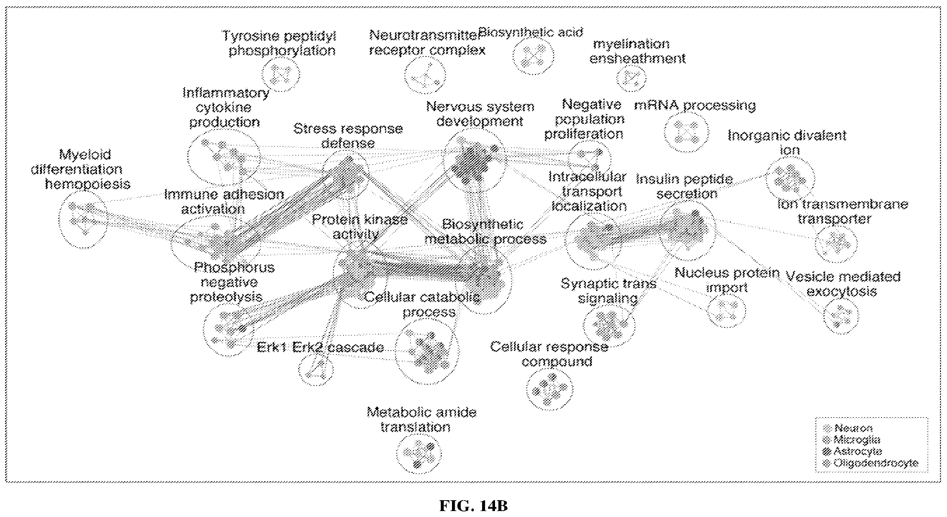

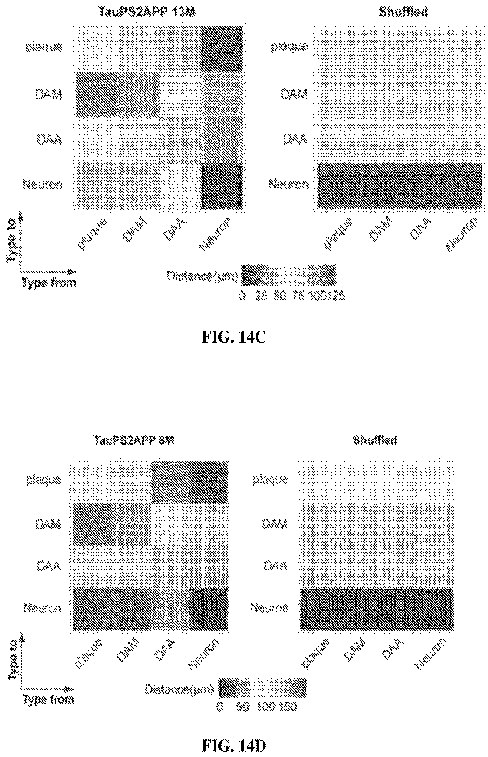

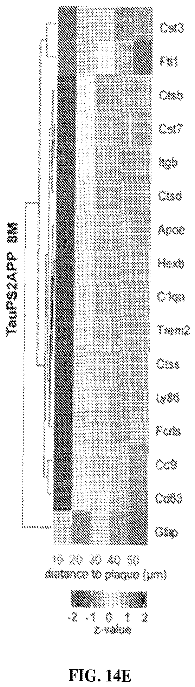

**FIGs. 14A-14E** show pathway and spatial analysis of AD mouse brain. FIG. 14A provides a gene ontology heatmap showing the biological process related terms enriched in upregulated genes of each cell type of interest in the AD 8-month sample. Terms are filtered by term size: 20-1000. The shading of the tiles represents enrichment $-\log_{10}$(FDR) value. FIG. 14B provides functional enrichment maps generated from the differentially expressed genes (abs(LogFC) > 0.1, Wilcox test p value < 0.05) in neuronal cells (excitatory, inhibitory neurons) and non-neuronal cells (microglia, astrocytes, and oligodendrocytes) using Cytoscape with EnrichmentMap and AutoAnnotate apps. Each circle represents a GO term and is shaded by cell types to account for common and different contributions of DEGs from each cell cluster. Ellipses delineate constellations of GO terms, clustered by the Autoannotate app. FIG. 14C (left) provides a heatmap showing the average nearest-neighbor distance between relevant cell types and Aβ plaque in the AD 13-month sample. The average nearest-neighbor distance is calculated by: 1) For every cell and plaque, calculate the Euclidean distance to each other object, then find the nearest cell of each cell type or plaque and save the distance. 2) For every comparison of interest (*i.e.,* Micro vs. Plaque), calculate the mean of its distance distribution. FIG. 14C (right) shows the same distances as FIG. 14C (left) but using shuffled (randomized) cell type labels. FIG. 14D (left) provides a heatmap showing the average nearest-neighbor distance between relevant cell types and Aβ plaque in the AD 8-month sample. The calculation is the same with that used in FIG. 14C. FIG. 14D (right) shows the same distances as FIG. 14D (left) but using shuffled (randomized) cell type labels. FIG. 14E provides a matrix plot showing genes upregulated in the near plaque regions of AD 8-month samples from cells within a 25 μm ring compared to cells outside the 25 μm ring.

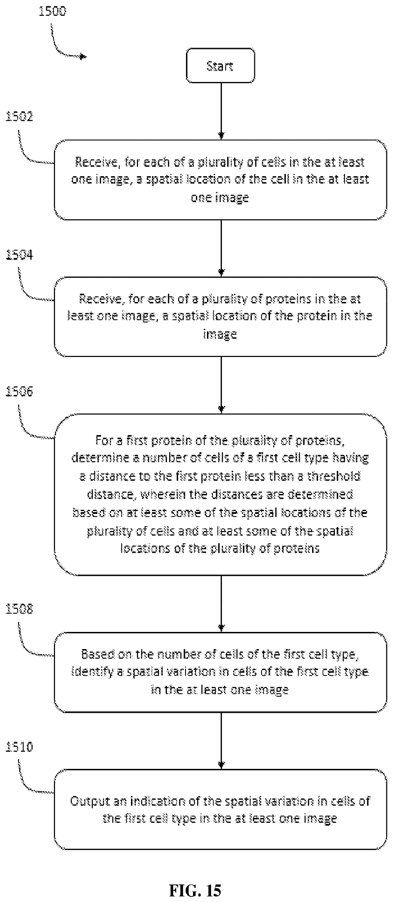

**FIG. 15** is a flow diagram of one embodiment of a method related to identifying spatial variations of cell types in at least one image.

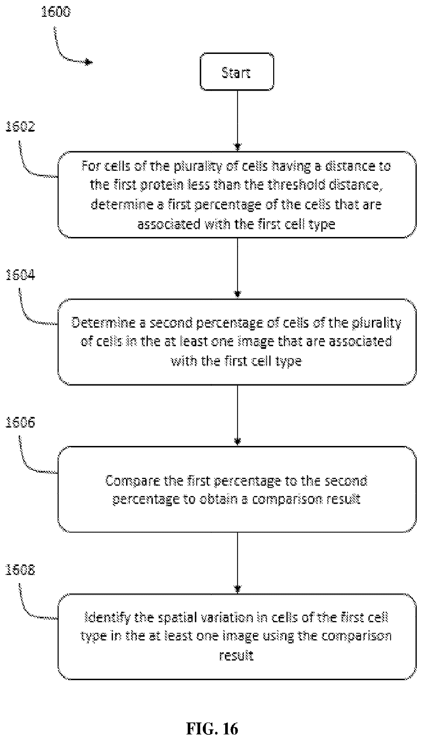

**FIG. 16** is a flow diagram of one embodiment of a method related to identifying a spatial variation in cells of a first cell type in at least one image.

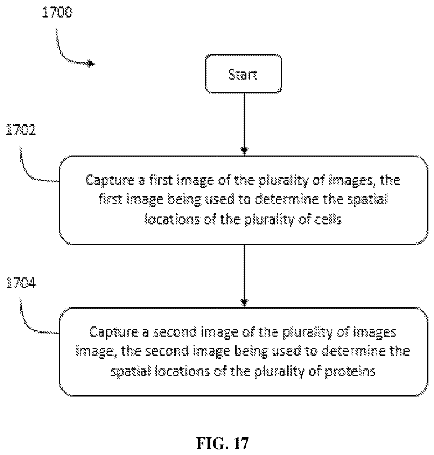

**FIG. 17** is a flow diagram of one embodiment of a method related to capturing at least one image using a camera.

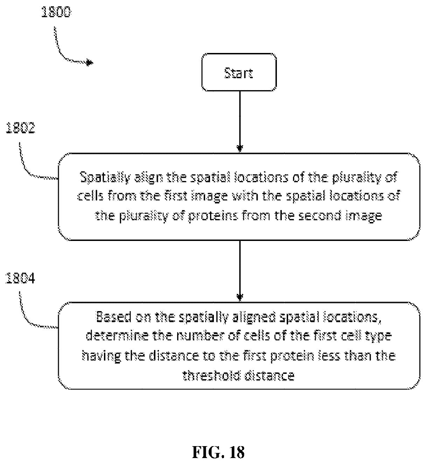

**FIG. 18** is a flow diagram of one embodiment of spatially aligning spatial locations from a plurality of images.

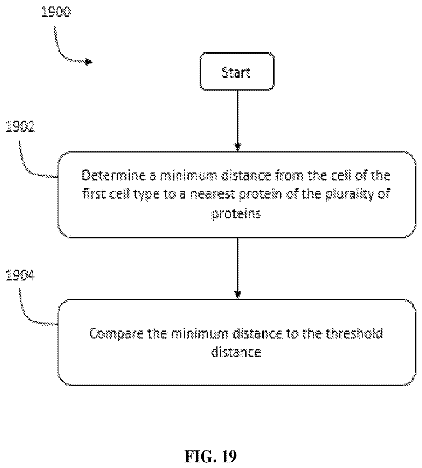

**FIG. 19** is a flow diagram of one embodiment of a method related to determining a number of cells of a cell type having a distance to a first protein less than a threshold distance.

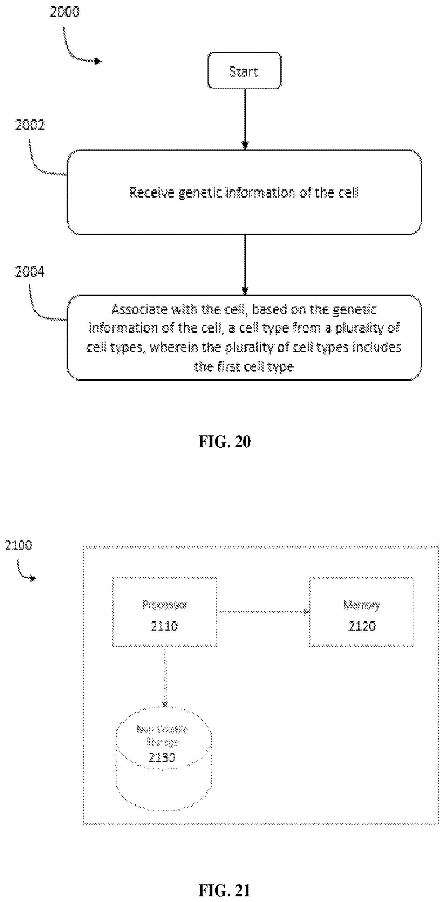

**FIG. 20** is a flow diagram of one embodiment of a method related to determining the cell type of a cell.

**FIG. 21** is a block diagram of a computer system on which various functions or methods may be implemented.


## DEFINITIONS

Unless defined otherwise, all technical and scientific terms used herein have the meaning commonly understood by a person skilled in the art to which this invention belongs. The following references provide one of skill with a general definition of many of the terms used in this invention: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991). As used herein, the following terms have the meanings ascribed to them unless specified otherwise.

The terms "administer," "administering," and "administration" refer to implanting, absorbing, ingesting, injecting, inhaling, or otherwise introducing a treatment or therapeutic agent, or a composition of treatments or therapeutic agents, in or on a subject.

The term "amplicon" as used herein refers to a nucleic acid (*e.g.,* DNA or RNA) that is the product of an amplification reaction (*i.e.,* the production of one or more copies of a genetic fragment or target sequence) or replication reaction. Amplicons can be formed artificially using, for example, PCR or other polymerization reactions. The term "concatenated amplicons" refers to multiple amplicons that are joined together to form a single nucleic acid molecule. Concatenated amplicons can be formed, for example, by rolling circle amplification (RCA), in which a circular oligonucleotide is amplified to produce multiple linear copies of the oligonucleotide as a single nucleic acid molecule comprising multiple amplicons that are concatenated.

An "antibody" refers to a glycoprotein belonging to the immunoglobulin superfamily. The terms antibody and immunoglobulin are used interchangeably. With some exceptions, mammalian antibodies are typically made of basic structural units each with two large heavy chains and two small light chains. There are several different types of antibody heavy chains, and several different kinds of antibodies, which are grouped together into different isotypes based on which heavy chain they possess. Five different antibody isotypes are known in mammals (IgG, IgA, IgE, IgD, and IgM, which perform different roles, and help direct the appropriate immune response for each different type of foreign object they encounter. In some embodiments, an antibody used herein binds to a protein of interest (*e.g.,* any protein of interest expressed in a cell). The term "antibody" as used herein also encompasses antibody fragments and nanobodies, as well as variants of antibodies and variants of antibody fragments and nanobodies.

The term "cancer" refers to a class of diseases characterized by the development of abnormal cells that proliferate uncontrollably and have the ability to infiltrate and destroy normal body tissues. *See e.g.,* Stedman's Medical Dictionary, 25th ed.; Hensyl ed.; Williams & Wilkins: Philadelphia, 1990. Exemplary cancers include, but are not limited to, acoustic neuroma; adenocarcinoma; adrenal gland cancer; anal cancer; angiosarcoma (*e.g.,* lymphangiosarcoma, lymphangioendotheliosarcoma, hemangiosarcoma); appendix cancer; benign monoclonal gammopathy; biliary cancer (*e.g.,* cholangiocarcinoma); bladder cancer; breast cancer (*e.g.,* adenocarcinoma of the breast, papillary carcinoma of the breast, mammary cancer, medullary carcinoma of the breast); brain cancer (*e.g.,* meningioma, glioblastomas, glioma (*e.g.,* astrocytoma, oligodendroglioma), medulloblastoma); bronchus cancer; carcinoid tumor; cervical cancer (*e.g.,* cervical adenocarcinoma); choriocarcinoma; chordoma; craniopharyngioma; colorectal cancer (*e.g.,* colon cancer, rectal cancer, colorectal adenocarcinoma); connective tissue cancer; epithelial carcinoma; ependymoma; endotheliosarcoma (*e.g.,* Kaposi's sarcoma, multiple idiopathic hemorrhagic sarcoma); endometrial cancer (*e.g.,* uterine cancer, uterine sarcoma); esophageal cancer (*e.g.,* adenocarcinoma of the esophagus, Barrett's adenocarcinoma); Ewing's sarcoma; ocular cancer (*e.g.,* intraocular melanoma, retinoblastoma); familiar hypereosinophilia; gall bladder cancer; gastric cancer (*e.g.,* stomach adenocarcinoma); gastrointestinal stromal tumor (GIST); germ cell cancer; head and neck cancer (*e.g.,* head and neck squamous cell carcinoma, oral cancer (*e.g.,* oral squamous cell carcinoma), throat cancer (*e.g.,* laryngeal cancer, pharyngeal cancer, nasopharyngeal cancer, oropharyngeal cancer)); hematopoietic cancers (*e.g.,* leukemia such as acute lymphocytic leukemia (ALL) (*e.g.,* B-cell ALL, T-cell ALL), acute myelocytic leukemia (AML) (*e.g.,* B-cell AML, T-cell AML), chronic myelocytic leukemia (CML) (*e.g.,* B-cell CML, T-cell CML), and chronic lymphocytic leukemia (CLL) (*e.g.,* B-cell CLL, T-cell CLL); lymphoma such as Hodgkin lymphoma (HL) (*e.g.,* B-cell HL, T-cell HL) and non-Hodgkin lymphoma (NHL) (*e.g.,* B-cell NHL such as diffuse large cell lymphoma (DLCL) (*e.g.,* diffuse large B-cell lymphoma), follicular lymphoma, chronic lymphocytic leukemia/small lymphocytic lymphoma (CLL/SLL), mantle cell lymphoma (MCL), marginal zone B-cell lymphomas (*e.g.,* mucosa-associated lymphoid tissue (MALT) lymphomas, nodal marginal zone B-cell lymphoma, splenic marginal zone B-cell lymphoma), primary mediastinal B-cell lymphoma, Burkitt lymphoma, lymphoplasmacytic lymphoma (*i.e.,* Waldenström's macroglobulinemia), hairy cell leukemia (HCL), immunoblastic large cell lymphoma, precursor B-lymphoblastic lymphoma and primary central nervous system (CNS) lymphoma; and T-cell NHL such as precursor T-lymphoblastic lymphoma/leukemia, peripheral T-cell lymphoma (PTCL) (*e.g.,* cutaneous T-cell lymphoma (CTCL) (*e.g.,* mycosis fungoides, Sezary syndrome), angioimmunoblastic T-cell lymphoma, extranodal natural killer T-cell lymphoma, enteropathy type T-cell lymphoma, subcutaneous panniculitis-like T-cell lymphoma, and anaplastic large cell lymphoma); a mixture of one or more leukemia/lymphoma as described above; and multiple myeloma (MM)), heavy chain disease (*e.g.*, alpha chain disease, gamma chain disease, mu chain disease); hemangioblastoma; hypopharynx cancer; inflammatory myofibroblastic tumors; immunocytic amyloidosis; kidney cancer (*e.g.,* nephroblastoma *a.k.a.* Wilms' tumor, renal cell carcinoma); liver cancer (*e.g.,* hepatocellular cancer (HCC), malignant hepatoma); lung cancer (*e.g.,* bronchogenic carcinoma, small cell lung cancer (SCLC), non-small cell lung cancer (NSCLC), adenocarcinoma of the lung); leiomyosarcoma (LMS); mastocytosis (*e.g.,* systemic mastocytosis); muscle cancer; myelodysplastic syndrome (MDS); mesothelioma; myeloproliferative disorder (MPD) (*e.g.,* polycythemia vera (PV), essential thrombocytosis (ET), agnogenic myeloid metaplasia (AMM) *a.k.a.* myelofibrosis (MF), chronic idiopathic myelofibrosis, chronic myelocytic leukemia (CML), chronic neutrophilic leukemia (CNL), hypereosinophilic syndrome (HES)); neuroblastoma; neurofibroma (*e.g.,* neurofibromatosis (NF) type 1 or type 2, schwannomatosis); neuroendocrine cancer (*e.g.,* gastroenteropancreatic neuroendoctrine tumor (GEP-NET), carcinoid tumor); osteosarcoma (*e.g.,* bone cancer); ovarian cancer (*e.g.,* cystadenocarcinoma, ovarian embryonal carcinoma, ovarian adenocarcinoma); papillary adenocarcinoma; pancreatic cancer (*e.g.,* pancreatic adenocarcinoma, intraductal papillary mucinous neoplasm (IPMN), Islet cell tumors); penile cancer (*e.g.,* Paget's disease of the penis and scrotum); pinealoma; primitive neuroectodermal tumor (PNT); plasma cell neoplasia; paraneoplastic syndromes; intraepithelial neoplasms; prostate cancer (*e.g.,* prostate adenocarcinoma); rectal cancer; rhabdomyosarcoma; salivary gland cancer; skin cancer (*e.g.,* squamous cell carcinoma (SCC), keratoacanthoma (KA), melanoma, basal cell carcinoma (BCC)); small bowel cancer (*e.g.,* appendix cancer); soft tissue sarcoma (*e.g.,* malignant fibrous histiocytoma (MFH), liposarcoma, malignant peripheral nerve sheath tumor (MPNST), chondrosarcoma, fibrosarcoma, myosarcoma); sebaceous gland carcinoma; small intestine cancer; sweat gland carcinoma; synovioma; testicular cancer (*e.g.,* seminoma, testicular embryonal carcinoma); thyroid cancer (*e.g.,* papillary carcinoma of the thyroid, papillary thyroid carcinoma (PTC), medullary thyroid cancer); urethral cancer; vaginal cancer; and vulvar cancer (*e.g.,* Paget's disease of the vulva).

A "cell," as used herein, may be present in a population of cells (*e.g.,* in a tissue, a sample, a biopsy, an organ, or an organoid). In some embodiments, a population of cells is composed of a plurality of different cell types. Cells for use in the methods of the present disclosure can be present within an organism, a single cell type derived from an organism, or a mixture of cell types. Included are naturally occurring cells and cell populations, genetically engineered cell lines, cells derived from transgenic animals, cells from a subject, etc. Virtually any cell type and size can be accommodated in the methods and systems described herein. Suitable cells include bacterial, fungal, plant, and animal cells. In some embodiments, the cells are mammalian cells (*e.g.,* complex cell populations such as naturally occurring tissues). In some embodiments, the cells are from a human. In certain embodiments, the cells are collected from a subject (*e.g.,* a human) through a medical procedure such as a biopsy. Alternatively, the cells may be a cultured population (*e.g.,* a culture derived from a complex population, or a culture derived from a single cell type where the cells have differentiated into multiple lineages). The cells may also be provided *in situ* in a tissue sample.

Cell types contemplated for use in the methods of the present disclosure include, but are not limited to, stem and progenitor cells (*e.g.,* embryonic stem cells, hematopoietic stem cells, mesenchymal stem cells, neural crest cells, *etc.*), endothelial cells, muscle cells, myocardial cells, smooth and skeletal muscle cells, mesenchymal cells, epithelial cells, hematopoietic cells, lymphocytes such as T-cells (*e.g.,* Thl T cells, Th2 T cells, ThO T cells, cytotoxic T cells) and B cells (*e.g.,* pre-B cells), monocytes, dendritic cells, neutrophils, macrophages, natural killer cells, mast cells, adipocytes, immune cells, neurons, hepatocytes, and cells involved with particular organs (*e.g.,* thymus, endocrine glands, pancreas, brain, neurons, glia, astrocytes, dendrocytes, and genetically modified cells thereof). The cells may also be transformed or neoplastic cells of different types (*e.g.,* carcinomas of different cell origins, lymphomas of different cell types, *etc.*) or cancerous cells of any kind (*e.g.,* from any of the cancers disclosed herein). Cells of different origins (*e.g.,* ectodermal, mesodermal, and endodermal) are also contemplated for use in the methods of the present disclosure. In some embodiments, the cells are microglia, astrocytes, oligodendrocytes, excitatory neurons, or inhibitory neurons. In some embodiments, cells of multiple cell types are present within the same sample.

As used herein, the term "detecting agent" refers to any agent that can be used for detecting the presence or location of any protein or peptide of interest. In some embodiments, the methods disclosed herein include a step of contacting one or more cells with one or more detecting agents. Each detecting agent used in the methods disclosed herein binds to a protein or peptide of interest. Detecting agents that can be used in the methods described herein include, but are not limited to, proteins, peptides, nucleic acids, and small molecules. In certain embodiments, the detecting agents are antibodies that bind to a protein of interest. In certain embodiments, the detecting agents include antibody fragments, antibody variants, and nanobodies. In certain embodiments, the detecting agents include aptamers. In certain embodiments, the detecting agents include receptors, or fragments thereof. In some embodiments, the detecting agents include small molecule dyes *(e.g.,* the small molecule X-34).

As used herein, the term "gene" refers to a nucleic acid fragment that expresses a protein, including regulatory sequences preceding (5' non-coding sequences) and following (3' non-coding sequences) the coding sequence.

As used herein, "gene expression" refers to the process by which information from a gene is used in the synthesis of a gene product. Gene products include proteins and RNA transcripts (*e.g.,* messenger RNA, transfer RNA, or small nuclear RNA). Gene expression includes transcription and translation. Transcription is the process by which a segment of DNA is transcribed into RNA by an RNA polymerase. Translation is the process by which an RNA is translated into a peptide or protein by a ribosome. The term "genetic information," as used herein, refers to one or more genes and/or one or more RNA transcripts (*e.g.,* any number of genes and/or RNA transcripts).

"Neurodegenerative diseases" refer to a type of neurological disease marked by the loss of nerve cells, including, but not limited to, Alzheimer's disease, Parkinson's disease, amyotrophic lateral sclerosis, tauopathies (including frontotemporal dementia), and Huntington's disease. In some embodiments, a neurodegenerative disease is Alzheimer's disease. Causes of Alzheimer's disease are poorly understood but in the majority of cases are thought to include a genetic basis. The disease is characterized by loss of neurons and synapses in the cerebral cortex, resulting in atrophy of the affected regions. Biochemically, Alzheimer's disease is characterized as a protein misfolding disease caused by plaque accumulation of abnormally folded amyloid beta protein and tau protein in the brain. Symptoms of Alzheimer's disease include, but are not limited to, difficulty remembering recent events, problems with language, disorientation, mood swings, loss of motivation, self-neglect, and behavioral issues. Ultimately, bodily functions are gradually lost, and Alzheimer's disease eventually leads to death. Treatment is currently aimed at treating cognitive problems caused by the disease (*e.g.,* with acetylcholinesterase inhibitors or NMDA receptor antagonists), psychosocial interventions (*e.g.,* behavior-oriented or cognition-oriented approaches), and general caregiving. There are no treatments currently available to stop or reverse the progression of the disease completely.

The terms "polynucleotide", "nucleotide sequence", "nucleic acid", "nucleic acid molecule", "nucleic acid sequence", and "oligonucleotide" refer to a series of nucleotide bases (also called "nucleotides") in DNA and RNA and mean any chain of two or more nucleotides. The polynucleotides can be chimeric mixtures or derivatives or modified versions thereof, and single-stranded or double-stranded. The oligonucleotide can be modified at the base moiety, sugar moiety, or phosphate backbone, for example, to improve stability of the molecule, its hybridization parameters, *etc.*

A "protein," "peptide," or "polypeptide" comprises a polymer of amino acid residues linked together by peptide bonds. The term refers to proteins, polypeptides, and peptides of any size, structure, or function. Typically, a protein will be at least three amino acids long. A protein may refer to an individual protein or a collection of proteins. Inventive proteins preferably contain only natural amino acids, although non-natural amino acids (*i.e.,* compounds that do not occur in nature but that can be incorporated into a polypeptide chain) and/or amino acid analogs as are known in the art may alternatively be employed. Also, one or more of the amino acids in a protein may be modified, for example, by the addition of a chemical entity such as a carbohydrate group, a hydroxyl group, a phosphate group, a farnesyl group, an isofarnesyl group, a fatty acid group, a linker for conjugation or functionalization, or other modification. A protein may also be a single molecule or may be a multi-molecular complex. A protein may be a fragment of a naturally occurring protein or peptide. A protein may be naturally occurring, recombinant, synthetic, or any combination of these.

"Pseudotime," as used herein, refers to a method of modeling differential expression of genes in a cell and is further described in Van den Berge, K. et al. Trajectory-based differential expression analysis for single-cell sequencing data. Nature Communications 2020, 11, 1-13, which is incorporated herein by reference.

A "transcript" or "RNA transcript" is the product resulting from RNA polymerase-catalyzed transcription of a DNA sequence. When the RNA transcript is a complimentary copy of the DNA sequence, it is referred to as the primary transcript, or it may be an RNA sequence derived from post-transcriptional processing of the primary transcript and is referred to as the mature RNA. "Messenger RNA (mRNA)" refers to the RNA that is without introns and can be translated into polypeptides by the cell. "cRNA" refers to complementary RNA, transcribed from a recombinant cDNA template. "cDNA" refers to DNA that is complementary to and derived from an mRNA template.

The term "sample" or "biological sample" refers to any sample including tissue samples (such as tissue sections, surgical biopsies, and needle biopsies of a tissue); cell samples (*e.g.,* cytological smears (such as Pap or blood smears) or samples of cells obtained by microdissection); or cell fractions, fragments, or organelles (such as obtained by lysing cells and separating the components thereof by centrifugation or otherwise). Other examples of biological samples include, but are not limited to, blood, serum, urine, semen, fecal matter, cerebrospinal fluid, interstitial fluid, mucous, tears, sweat, pus, biopsied tissue (*e.g.,* obtained by a surgical biopsy or needle biopsy), nipple aspirates, milk, vaginal fluid, saliva, swabs (such as buccal swabs), or any material containing biomolecules that is derived from a first biological sample. In some embodiments, a biological sample is a surgical biopsy taken from a subject, for example, a biopsy of any of the tissues described herein. In certain embodiments, a biological sample is a tumor biopsy (*e.g.,* from a subject diagnosed with, suspected of having, or thought to have cancer). In some embodiments, the sample is brain tissue. In some embodiments, the tissue is cardiac tissue. In some embodiments, the tissue is muscle tissue.

A "subject" to which administration is contemplated refers to a human (*i.e.,* male or female of any age group, *e.g.,* pediatric subject (*e.g.,* infant, child, or adolescent) or adult subject (*e.g.,* young adult, middle-aged adult, or senior adult)) or non-human animal. In some embodiments, the non-human animal is a mammal (*e.g.,* primate (*e.g.,* cynomolgus monkey or rhesus monkey) or mouse). The term "patient" refers to a subject in need of treatment of a disease. In some embodiments, the subject is human. In some embodiments, the patient is human. The human may be a male or female at any stage of development. A subject or patient "in need" of treatment of a disease or disorder includes, without limitation, those who exhibit any risk factors or symptoms of a disease or disorder (*e.g.,* Alzheimer's disease). In some embodiments, a subject is a non-human experimental animal (*e.g.,* a mouse).

A "therapeutically effective amount" of a treatment or therapeutic agent is an amount sufficient to provide a therapeutic benefit in the treatment of a condition or to delay or minimize one or more symptoms associated with the condition. A therapeutically effective amount of a treatment or therapeutic agent means an amount of the therapy, alone or in combination with other therapies, that provides a therapeutic benefit in the treatment of the condition. The term "therapeutically effective amount" can encompass an amount that improves overall therapy, reduces or avoids symptoms, signs, or causes of the condition, and/or enhances the therapeutic efficacy of another therapeutic agent.

As used herein, a "tissue" is a group of cells and their extracellular matrix from the same origin. Together, the cells carry out a specific function. The association of multiple tissue types together forms an organ. The cells may be of different cell types. In some embodiments, a tissue is an epithelial tissue. Epithelial tissues are formed by cells that cover an organ surface (*e.g.,* the surface of the skin, airways, soft organs, reproductive tract, and inner lining of the digestive tract). Epithelial tissues perform protective functions and are also involved in secretion, excretion, and absorption. Examples of epithelial tissues include, but are not limited to, simple squamous epithelium, stratified squamous epithelium, simple cuboidal epithelium, transitional epithelium, pseudostratified epithelium, columnar epithelium, and glandular epithelium. In some embodiments, a tissue is a connective tissue. Connective tissues are fibrous tissues made up of cells separated by non-living material (*e.g.,* an extracellular matrix). Connective tissues provide shape to organs and hold organs in place. Connective tissues include fibrous connective tissue, skeletal connective tissue, and fluid connective tissue. Examples of connective tissues include, but are not limited to, blood, bone, tendon, ligament, adipose, and areolar tissues. In some embodiments, a tissue is a muscular tissue. Muscular tissue is an active contractile tissue formed from muscle cells. Muscle tissue functions to produce force and cause motion. Muscle tissue includes smooth muscle (*e.g.,* as found in the inner linings of organs), skeletal muscle (*e.g.,* as typically attached to bones), and cardiac muscle (*e.g.,* as found in the heart, where it contracts to pump blood throughout an organism). In some embodiments, a tissue is a nervous tissue. Nervous tissue includes cells comprising the central nervous system and peripheral nervous system. Nervous tissue forms the brain, spinal cord, cranial nerves, and spinal nerves (*e.g.,* motor neurons). In certain embodiments, a tissue is brain tissue. In certain embodiments, a tissue is placental tissue. In some embodiments, a tissue is heart tissue.

The terms "treatment," "treat," and "treating" refer to reversing, alleviating, delaying the onset of, or inhibiting the progress of a disease described herein (*e.g.,* Alzheimer's disease). In some embodiments, treatment may be administered after one or more signs or symptoms of the disease have developed or have been observed (*e.g.,* prophylactically (as may be further described herein) or upon suspicion or risk of disease). In other embodiments, treatment may be administered in the absence of signs or symptoms of the disease. For example, treatment may be administered to a susceptible subject prior to the onset of symptoms (*e.g.,* in light of a history of symptoms in the subject, or family members of the subject). Treatment may also be continued after symptoms have resolved, for example, to delay or prevent recurrence. In some embodiments, treatment may be administered after using the methods disclosed herein and observing an alteration in gene and/or protein expression of one or more nucleic acids and/or proteins of interest in a cell or tissue in comparison to a healthy cell or tissue.

The terms "tumor" and "neoplasm" are used herein refers to an abnormal mass of tissue wherein the growth of the mass surpasses and is not coordinated with the growth of a normal tissue. A tumor may be "benign" or "malignant," depending on the following characteristics: degree of cellular differentiation (including morphology and functionality), rate of growth, local invasion, and metastasis. A "benign neoplasm" is generally well differentiated, has characteristically slower growth than a malignant neoplasm, and remains localized to the site of origin. In addition, a benign neoplasm does not have the capacity to infiltrate, invade, or metastasize to distant sites. Exemplary benign neoplasms include, but are not limited to, lipoma, chondroma, adenomas, acrochordon, senile angiomas, seborrheic keratoses, lentigos, and sebaceous hyperplasias. In some cases, certain "benign" tumors may later give rise to malignant neoplasms, which may result from additional genetic changes in a subpopulation of the tumor's neoplastic cells, and these tumors are referred to as "pre-malignant neoplasms." An exemplary pre-malignant neoplasm is a teratoma. In contrast, a "malignant neoplasm" is generally poorly differentiated (anaplasia) and has characteristically rapid growth accompanied by progressive infiltration, invasion, and destruction of the surrounding tissue. Furthermore, a malignant neoplasm generally has the capacity to metastasize to distant sites. The term "metastasis," "metastatic," or "metastasize" refers to the spread or migration of cancerous cells from a primary or original tumor to another organ or tissue and is typically identifiable by the presence of a "secondary tumor" or "secondary cell mass" of the tissue type of the primary or original tumor and not of that of the organ or tissue in which the secondary (metastatic) tumor is located. For example, a prostate cancer that has migrated to bone is said to be metastasized prostate cancer and includes cancerous prostate cancer cells growing in bone tissue.

## DETAILED DESCRIPTION OF CERTAIN EMBODIMENTS

The aspects described herein are not limited to specific embodiments, systems, compositions, methods, or configurations, and as such can, of course, vary. The terminology used herein is for the purpose of describing particular aspects only and, unless specifically defined herein, is not intended to be limiting.

The present disclosure provides methods and systems for mapping gene and protein expression in a cell (*i.e.,* mapping gene and protein expression within the same cell simultaneously). The present disclosure also provides methods for diagnosing a disease or disorder (*e.g.,* Alzheimer's disease or cancer) in a subject. Methods of screening for or testing a candidate agent capable of modulating gene and/or protein expression are also provided by the present disclosure. The present disclosure also provides methods for treating a disease or disorder, such as a neurological disorder (*e.g.,* Alzheimer's disease), in a subject in need thereof. Pairs of oligonucleotide probes, which may be useful for performing the methods described herein, are also described by the present disclosure, as well as kits comprising any of the oligonucleotide probes described herein. Additionally, the present disclosure provides methods, an apparatus, a system, and a non-transitory computer-readable storage medium for identifying spatial variations of cell types in at least one image.

*Methods for Mapping Gene and Protein Expression in a Cell*

In one aspect, the present disclosure provides methods for mapping gene and protein expression in a cell (see, for example, FIGs. 1A and 1B). In the methods disclosed herein, a cell may be contacted with one or more pairs of oligonucleotide probes, which are described further herein and may be used to amplify the transcripts (*e.g.,* by rolling circle amplification) to produce one or more concatenated amplicons. The cell may then be contacted with one or more detecting agents (*e.g.,* an antibody), wherein each detecting agent binds to a protein of interest in the cell. The one or more concatenated amplicons and the one or more detecting agents may then be embedded in a polymeric matrix, and the one or more amplicons may be sequenced to determine the identity of the transcripts (*e.g.,* through SEDAL sequencing (Sequencing with Error-reduction by Dynamic Annealing and Ligation) as described further herein) and their location within the polymer matrix. The location of the detecting agents (*e.g.,* antibodies) bound to one or more proteins of interest within the polymeric matrix may also be determined by imaging (*e.g.,* through confocal microscopy), allowing the location of the transcripts and the proteins of interest within the cell to be mapped. Using the locations of the transcripts and proteins of interest, individual cells, subcellular locations, and organelles can be identified. The method may be useful for comparing, for example, a cell (or multiple cells) from diseased and healthy tissue samples.

In some embodiments, the present disclosure provides a method for mapping gene and protein expression in a cell comprising the steps of:

a) contacting the cell with one or more pairs of oligonucleotide probes, wherein each pair of oligonucleotide probes comprises a first oligonucleotide probe and a second oligonucleotide probe, wherein

i) the first oligonucleotide probe comprises a portion that is complementary to the second oligonucleotide probe, a portion that is complementary to a nucleic acid of interest, a first barcode sequence, and a second barcode sequence; and

ii) the second oligonucleotide probe comprises a portion that is complementary to the nucleic acid of interest, a portion that is complementary to the first oligonucleotide probe, and a barcode sequence, wherein the barcode sequence of the second oligonucleotide probe is complementary to the second barcode sequence of the first oligonucleotide probe;

b) ligating the 5' end and the 3' end of the first oligonucleotide probe together to produce a circular oligonucleotide;
c) performing rolling circle amplification to amplify the circular oligonucleotide using the second oligonucleotide probe as a primer to produce one or more concatenated amplicons;
d) contacting the cell with one or more detecting agents, wherein each detecting agent binds to a protein of interest;
e) embedding the one or more concatenated amplicons and the one or more detecting agents in a polymeric matrix;
f) contacting the one or more concatenated amplicons embedded in the polymeric matrix with a third oligonucleotide probe comprising a sequence that is complementary to the first barcode sequence of the first oligonucleotide probe; and
g) imaging the one or more concatenated amplicons embedded in the polymeric matrix and the one or more detecting agents embedded in the polymeric matrix to determine the location of the nucleic acids of interest and the proteins of interest within the cell and, optionally, map gene and protein expression. In some embodiments, the expression of one nucleic acid and protein of interest is mapped using the methods described herein. In some embodiments, any of the methods described herein may be used to map the expression of a plurality of nucleic acids of interest and proteins of interest within the same cell (or multiple cells, *e.g.,* in a tissue sample).

The use of any type of cell in the methods disclosed herein is contemplated by the present disclosure (*e.g.,* any of the cell types described herein). In some embodiments, the cell is a mammalian cell. In certain embodiments, the cell is a human cell. In some embodiments, the cells are cells from the nervous system. In some embodiments, the cell is a cancer cell. The present disclosure also contemplates performing the methods described herein on multiple cells simultaneously. In some embodiments, the method is performed on multiple cells of the same cell type. In some embodiments, the method is performed on multiple cells comprising cells of different cell types. The cell types in which gene and protein expression may be mapped using the methods disclosed herein include, but are not limited to, stem cells, progenitor cells, neuronal cells, astrocytes, dendritic cells, endothelial cells, microglia, oligodendrocytes, muscle cells, myocardial cells, mesenchymal cells, epithelial cells, immune cells, and hepatic cells. In certain embodiments, the cells are microglia, astrocytes, oligodendrocytes, excitatory neurons, and/or inhibitory neurons. In certain embodiments, the cell or cells are permeabilized cells (*e.g.,* the cells are permeabilized prior to the step of contacting with one or more pairs of oligonucleotide probes). In certain embodiments, the cell or cells are present within an intact tissue (*e.g.,* of any of the tissue types described herein). In certain embodiments, the intact tissue is a fixed tissue sample. In some embodiments, the intact tissue comprises multiple cell types (*e.g.,* microglia, astrocytes, oligodendrocytes, excitatory neurons, and/or inhibitory neurons). In certain embodiments, the tissue is cardiac tissue, lymph node tissue, liver tissue, muscle tissue, bone tissue, eye tissue, or ear tissue.

The nucleic acid(s) of interest for which gene expression is profiled in the methods described herein may be transcripts that have been expressed from the genomic DNA of the cell. In some embodiments, the nucleic acid of interest is DNA. In some embodiments, the nucleic acid of interest is RNA. In certain embodiments, the nucleic acid of interest is mRNA. The methods described herein may be used to profile gene expression in a cell for one nucleic acid of interest at a time, or for multiple nucleic acids of interest simultaneously. In some embodiments, gene expression in a cell, or multiple cells, is mapped for more than 100, more than 200, more than 500, more than 1000, more than 2000, more than 3000, more than 5000, more than 10,000, more than 15,000, more than 20,000, more than 25,000, or more than 30,000 nucleic acids of interest simultaneously. In certain embodiments, gene expression in a cell, or multiple cells, is profiled for up to one million nucleic acids of interest simultaneously.

The methods disclosed herein also contemplate the use of a first oligonucleotide probe and a second oligonucleotide probe, provided as a pair of oligonucleotide probes. The first oligonucleotide probe used in the methods described herein (also referred to herein as the "padlock" probe) includes a first barcode sequence and a second barcode sequence, each made up of a specific sequence of nucleotides. In some embodiments, the first barcode sequence of the first oligonucleotide probe is about 5 to about 15, about 6 to about 14, about 7 to about 13, about 8 to about 12, or about 9 to about 11 nucleotides in length. In some embodiments, the first barcode sequence of the first oligonucleotide probe is 5, 6, 7, 8, 9, 10, 11, 12, 13, 14, or 15 nucleotides in length. In certain embodiments, the first barcode sequence of the first oligonucleotide probe is 10 nucleotides in length. In some embodiments, the second barcode sequence of the first oligonucleotide probe is about 5 to about 15, about 6 to about 14, about 7 to about 13, about 8 to about 12, or about 9 to about 11 nucleotides in length. In some embodiments, the second barcode sequence of the first oligonucleotide probe is 5, 6, 7, 8, 9, 10, 11, 12, 13, 14, or 15 nucleotides in length. In certain embodiments, the second barcode sequence of the first oligonucleotide probe is 10 nucleotides in length. The second barcode sequence provides an additional site of complementarity between the first and the second oligonucleotide probe, providing advantages over previous oligonucleotide probe designs. The second barcode sequence of the first oligonucleotide probe may increase the specificity of the detection of the nucleic acid of interest in the methods described herein (*i.e.,* as compared to if the method were performed using a first oligonucleotide probe that did not comprise a second barcode sequence). The second barcode sequence of the first oligonucleotide probe may also play a role in reducing non-specific amplification of the nucleic acid of interest in the methods described herein.

The barcodes of the oligonucleotide probes described herein may comprise gene-specific sequences used to identify nucleic acids of interest. The use of the barcodes on the oligonucleotide probes described herein is further described in, for example, International Patent Application Publication No. WO 2019/199579, and Wang et al., Science 2018, 361, 380, both of which are incorporated by reference herein in their entireties.

The first oligonucleotide probe also comprises a portion that is complementary to the second oligonucleotide probe and a portion that is complementary to a nucleic acid of interest. In some embodiments, the portion of the first oligonucleotide probe that is complementary to the second oligonucleotide probe is split between the 5' end and the 3' end of the first oligonucleotide probe. In some embodiments, the portion of the first oligonucleotide probe that is complementary to a nucleic acid of interest is about 10, about 11, about 12, about 13, about 14, about 15, about 16, about 17, about 18, about 19, about 20, about 21, about 22, about 23, about 24, about 25, about 26, about 27, about 28, about 29, or about 30 nucleotides long. In some embodiments, the first oligonucleotide probe is about 20, about 21, about 22, about 23, about 24, about 25, about 26, about 27, about 28, about 29, about 30, about 31, about 32, about 33, about 34, about 35, about 36, about 37, about 38, about 39, or about 40 nucleotides long. In some embodiments, the first oligonucleotide probe comprises the structure:

5'-[portion complementary to second probe]-[portion complementary to nucleic acid of interest]-[first barcode sequence]-[second barcode sequence]-3'

wherein ]-[ comprises an optional linker (*e.g.,* an optional nucleotide linker). In some embodiments, ]-[ represents a direct linkage between two portions of the first oligonucleotide probe (*i.e.,* a phosphodiester bond).

The second oligonucleotide probe used in the methods disclosed herein (also referred to herein as the "primer" probe) includes a barcode sequence made up of a specific sequence of nucleotides. In some embodiments, the barcode sequence of the second oligonucleotide probe is about 5 to about 15, about 6 to about 14, about 7 to about 13, about 8 to about 12, or about 9 to about 11 nucleotides in length. In some embodiments, the barcode sequence of the second oligonucleotide probe is 5, 6, 7, 8, 9, 10, 11, 12, 13, 14, or 15 nucleotides in length. In certain embodiments, the barcode sequence of the second oligonucleotide probe is 10 nucleotides in length.

The second oligonucleotide probe also comprises a portion that is complementary to a nucleic acid of interest and a portion that is complementary to a portion of the first oligonucleotide probe. In some embodiments, the first and the second oligonucleotide probes are complementary to and bind different portions of the nucleic acid of interest. In some embodiments, the portion of the second oligonucleotide probe that is complementary to a nucleic acid of interest is about 10, about 11, about 12, about 13, about 14, about 15, about 16, about 17, about 18, about 19, about 20, about 21, about 22, about 23, about 24, about 25, about 26, about 27, about 28, about 29, or about 30 nucleotides long. In some embodiments, the second oligonucleotide probe is about 20, about 21, about 22, about 23, about 24, about 25, about 26, about 27, about 28, about 29, about 30, about 31, about 32, about 33, about 34, about 35, about 36, about 37, about 38, about 39, or about 40 nucleotides long. In some embodiments, the second oligonucleotide probe comprises the structure:

5'-[portion complementary to nucleic acid of interest]-[portion complementary to first probe]-[barcode sequence]-3'

wherein ]-[ comprises an optional nucleotide linker. In some embodiments, ]-[ represents a direct linkage between two portions of the second oligonucleotide probe.

The methods disclosed herein also include the use of a third oligonucleotide probe. In some embodiments, the third oligonucleotide probe comprises a detectable label (*i.e.,* any label that can be used to visualize the location of the third oligonucleotide probe, for example, through imaging). In certain embodiments, the detectable label is fluorescent (*e.g.,* a fluorophore). As described herein, the third oligonucleotide probe is complementary to the second barcode sequence of the first oligonucleotide probe. In some embodiments, the second barcode sequence of the first oligonucleotide probe is a gene-specific sequence used to identify a nucleic acid of interest. In some embodiments, the step of contacting the one or more concatenated amplicons embedded in the polymeric matrix with the third oligonucleotide probe is performed to identify the nucleic acid of interest. This method for identifying a nucleic acid of interest is known as sequencing with error-reduction by dynamic annealing and ligation (SEDAL sequencing) and is described further in Wang, X. et al., Three-dimensional intact-tissue sequencing of single-cell transcriptional states. Science 2018, 36, eaat5691, and International Patent Application Publication No. WO 2019/199579, each of which is incorporated herein by reference.

The third oligonucleotide probe used in the methods described herein (*e.g.,* as used in SEDAL sequencing) may be read out using any suitable imaging technique known in the art. For example, in embodiments where the third oligonucleotide probe comprises a fluorophore, the fluorophore may be read out using imaging to identify the nucleic acid of interest. As discussed above, the third oligonucleotide probe comprises a sequence complementary to the second barcode sequence of the first oligonucleotide probe, which is used to detect a specific nucleic acid of interest. By imaging the location of the third oligonucleotide probe comprising a fluorophore, the location of that specific nucleic acid of interest within the sample can be determined. In some embodiments, the step of imaging comprises fluorescent imaging. In certain embodiments, the step of imaging comprises confocal microscopy. In certain embodiments, the step of imaging comprises epifluorescence microscopy. In some embodiments, the locations of the nucleic acids of interest and the proteins of interest are determined in the same round of imaging. In some embodiments, the locations of the nucleic acids of interest and the proteins of interest are determined in separate rounds of imaging. In certain embodiments, two rounds of imaging are performed. In certain embodiments, three rounds of imaging are performed. In certain embodiments, four rounds of imaging are performed. In certain embodiments, five or more rounds of imaging are performed.

In some embodiments, the methods disclosed herein include a step of contacting the cell with one or more detecting agents, wherein each detecting agent binds to a protein of interest. In some embodiments, the detecting agent is a protein, peptide, nucleic acid, or small molecule. In certain embodiments, the detecting agent is an antibody. In certain embodiments, the detecting agent is an antibody fragment, an antibody variant, or a nanobody. In certain embodiments, the detecting agent is an aptamer. In certain embodiments, the detecting agent is a receptor, or a fragment thereof. The use of any antibody that binds to a protein of interest is contemplated by the present disclosure. Antibodies that may be used in the methods described herein also include antibody fragments, as well as variants of full-length antibodies or antibody fragments. In some embodiments, the one or more detecting agents are antibodies that each comprise a unique detectable label (*e.g.,* a fluorophore). In some embodiments, the method further comprises contacting the one or more antibodies with a secondary detecting agent. For example, each antibody that binds to a protein of interest may be contacted with a different secondary detecting agent. In some embodiments, the secondary detecting agent is a secondary antibody (*i.e.,* an antibody that binds to one of the antibodies bound to a protein of interest). In certain embodiments, the secondary antibody comprises a detectable label. In some embodiments, the detectable label of the secondary antibody is a fluorophore. In some embodiments, the one or more detecting agents are antibodies that each bind to a protein of interest, and each antibody that binds to a protein of interest is conjugated to a unique oligonucleotide sequence. The one or more antibodies can then be contacted with an oligonucleotide conjugated to a detectable label (*e.g.,* a fluorophore) that is complementary to the oligonucleotide sequence conjugated to the one or more antibodies, allowing the locations of the one or more antibodies to be visualized (for example, by confocal microscopy or any other means for detecting fluorescence).

In some embodiments, the detecting agent that binds to a protein of interest is a small molecule dye. In certain embodiments, the small molecule dye is X-34. For example, X-34 (a fluorescent, amyloid-specific dye commonly used as a highly fluorescent marker for beta-sheet structures) may be used to detect the presence of Aβ plaques within a cell to determine pathological changes associated with Alzheimer's disease. The use of X-34 in detecting Aβ plaques is well known in the art and is described, for example, in Styren, S. D. et al. X-34, a fluorescent derivative of Congo red: a novel histochemical stain for Alzheimer's disease pathology. J. Histochem. Cytochem. 2000, 48(9), 1223-1232, which is incorporated herein by reference. In some embodiments, the step of contacting each of the one or more detecting agents embedded in the polymeric matrix with a secondary detecting agent is performed after the step of performing rolling circle amplification to amplify the circular oligonucleotide. In some embodiments, the step of contacting the cell with one or more detecting agents is performed prior to the step of embedding.

The methods provided herein may be used to map gene and protein expression in a cell at subcellular resolution. For example, any of the methods provided herein may be performed at a subcellular resolution of 200 nm, 150 nm, 100 nm, 50 nm, 40 nm, 30 nm, 20 nm, or 10 nm. In certain embodiments, any of the methods provided herein are performed at a subcellular resolution of 200 nm.

The use of various polymeric matrices is contemplated by the present disclosure, and any polymeric matrix in which the one or more concatenated amplicons and detecting agents can be embedded is suitable for use in the methods described herein. In some embodiments, the polymeric matrix is a hydrogel (*i.e.,* a network of crosslinked polymers that are hydrophilic). In some embodiments, the hydrogel is a polyvinyl alcohol hydrogel, a polyethylene glycol hydrogel, a sodium polyacrylate hydrogel, an acrylate polymer hydrogel, or a polyacrylamide hydrogel. In certain embodiments, the hydrogel is a polyacrylamide hydrogel. Such a hydrogel may be prepared, for example, by incubating the sample in a buffer comprising acrylamide and bis-acrylamide, removing the buffer, and incubating the sample in a polymerization mixture (comprising, *e.g.*, ammonium persulfate and tetramethylethylenediamine).

In some embodiments, the step of performing rolling circle amplification to amplify the circular oligonucleotide to produce one or more concatenated amplicons further comprises providing nucleotides modified with reactive chemical groups (*e.g., 5*-(*3*-aminoallyl)-dUTP). In some embodiments, the nucleotides modified with reactive chemical groups make up about 5%, about 6%, about 7%, about 8%, about 9%, or about 10% of the nucleotides used in the amplification reaction. For example, the step of performing rolling circle amplification to amplify the circular oligonucleotide to produce one or more concatenated amplicons may further comprise providing amine-modified nucleotides. During the amplification process, the amine-modified nucleotides are incorporated into the one or more concatenated amplicons as they are produced. The resulting amplicons are functionalized with primary amines, which can be further reacted with another compatible chemical moiety (*e.g., N*-hydroxysuccinimide) to facilitate the step of embedding the concatenated amplicons in the polymer matrix. In some embodiments, the step of embedding the one or more concatenated amplicons in a polymer matrix comprises reacting the amine-modified nucleotides of the one or more concatenated amplicons with acrylic acid *N*-hydroxysuccinimide ester and co-polymerizing the one or more concatenated amplicons and the polymer matrix.

In certain embodiments, the present disclosure provides a method for mapping gene expression in a cell comprising the steps of:

a) contacting the cell with one or more pairs of oligonucleotide probes, wherein each pair of oligonucleotide probes comprises a first oligonucleotide probe and a second oligonucleotide probe, wherein

i) the first oligonucleotide probe comprises a portion that is complementary to the second oligonucleotide probe, a portion that is complementary to a nucleic acid of interest, a first barcode sequence, and a second barcode sequence; and
ii) the second oligonucleotide probe comprises a portion that is complementary to the nucleic acid of interest, a portion that is complementary to the first oligonucleotide probe, and a barcode sequence, wherein the barcode sequence of the second oligonucleotide probe is complementary to the second barcode sequence of the first oligonucleotide probe;

b) ligating the 5' end and the 3' end of the first oligonucleotide probe together to produce a circular oligonucleotide;
c) performing rolling circle amplification to amplify the circular oligonucleotide using the second oligonucleotide probe as a primer to produce one or more concatenated amplicons;
d) embedding the one or more concatenated amplicons in a polymeric matrix;
e) contacting the one or more concatenated amplicons embedded in the polymeric matrix with a third oligonucleotide probe comprising a sequence that is complementary to the first barcode sequence of the first oligonucleotide probe; and
f) imaging the one or more concatenated amplicons embedded in the polymeric matrix to determine the location of the nucleic acids of interest within the cell and, optionally, map gene and protein expression.

In certain embodiments, the present disclosure provides a method for mapping gene and protein expression in a cell comprising the steps of:

a) contacting the cell with one or more pairs of oligonucleotide probes, wherein each pair of oligonucleotide probes comprises a first oligonucleotide probe and a second oligonucleotide probe, wherein

i) the first oligonucleotide probe comprises a portion that is complementary to the second oligonucleotide probe, a portion that is complementary to a nucleic acid of interest, and a barcode sequence; and
ii) the second oligonucleotide probe comprises a portion that is complementary to the nucleic acid of interest and a portion that is complementary to the first oligonucleotide probe;

b) ligating the 5' end and the 3' end of the first oligonucleotide probe together to produce a circular oligonucleotide;
c) performing rolling circle amplification to amplify the circular oligonucleotide using the second oligonucleotide probe as a primer to produce one or more concatenated amplicons;
d) contacting the cell with one or more detecting agents, wherein each detecting agent binds to a protein of interest;
e) embedding the one or more concatenated amplicons and the one or more detecting agents in a polymeric matrix;
f) contacting the one or more concatenated amplicons embedded in the polymeric matrix with a third oligonucleotide probe comprising a sequence that is complementary to the first barcode sequence of the first oligonucleotide probe; and
g) imaging the one or more concatenated amplicons embedded in the polymeric matrix and the one or more detecting agents embedded in the polymeric matrix to determine the location of the nucleic acids of interest and the proteins of interest within the cell and, optionally, map gene and protein expression.

*Methods for Diagnosing a Disease or Disorder in a Subject*

In another aspect, the present disclosure provides methods for diagnosing a disease or disorder in a subject. For example, the methods for profiling gene and protein expression described herein may be performed in a cell or multiple cells from a sample taken from a subject (e.g., a subject who is thought to have or is at risk of having a disease or disorder, or a subject who is healthy or thought to be healthy). The expression of various nucleic acids and proteins of interest in the cell can then be compared to the expression of the same nucleic acids and proteins of interest in a non-diseased cell or a cell from a non-diseased tissue sample (*e.g.,* a cell from a healthy individual, or multiple cells from a population of healthy individuals). Any alteration in the expression of the nucleic acid of interest and/or protein of interest (or multiple nucleic acids of interest and/or proteins of interest, *e.g.*, a specific disease signature) relative to expression in a non-diseased cell may indicate that the subject has the disease or disorder. Gene and protein expression in one or more non-diseased cells may be profiled alongside expression in a diseased cell as a control experiment. Gene and protein expression in one or more non-diseased cells may have also been profiled previously, and expression in a diseased cell may be compared to this reference data for a non-diseased cell.

In some embodiments, a method for diagnosing a disease or disorder in a subject comprises the steps of:

a) contacting a cell taken from the subject with one or more pairs of oligonucleotide probes, wherein each pair of oligonucleotide probes comprises a first oligonucleotide probe and a second oligonucleotide probe, wherein

i) the first oligonucleotide probe comprises a portion that is complementary to the second oligonucleotide probe, a portion that is complementary to a nucleic acid of interest, a first barcode sequence, and a second barcode sequence; and

ii) the second oligonucleotide probe comprises a portion that is complementary to the nucleic acid of interest, a portion that is complementary to the first oligonucleotide probe, and a barcode sequence, wherein the barcode sequence of the second oligonucleotide probe is complementary to the second barcode sequence of the first oligonucleotide probe;

b) ligating the 5' end and the 3' end of the first oligonucleotide probe together to produce a circular oligonucleotide;
c) performing rolling circle amplification to amplify the circular oligonucleotide using the second oligonucleotide probe as a primer to produce one or more concatenated amplicons;
d) contacting the cell with one or more detecting agents, wherein each detecting agent binds to a protein of interest;
e) embedding the one or more concatenated amplicons and the one or more detecting agents in a polymeric matrix;
f) contacting the one or more concatenated amplicons embedded in the polymeric matrix with a third oligonucleotide probe comprising a sequence that is complementary to the first barcode sequence of the first oligonucleotide probe; and
g) imaging the one or more concatenated amplicons embedded in the polymeric matrix and the one or more detecting agents embedded in the polymeric matrix to determine the location of the nucleic acids of interest and the proteins of interest within the cell and, optionally, map gene and protein expression;

wherein an alteration in the expression of the nucleic acids of interest and/or the proteins of interest relative to expression in one or more non-diseased cells indicates that the subject has the disease or disorder.

In some embodiments, gene and protein expression in one or more non-diseased cells is profiled simultaneously using the methods disclosed herein as a control experiment. In some embodiments, the gene and protein expression data in one or more non-diseased cells that is compared to expression in a diseased cell comprises reference data from when the method was performed on one or more non-diseased cells previously.

Diagnosis of any disease or disorder is contemplated by the methods described herein. In some embodiments, the disease or disorder is a genetic disease, a proliferative disease, an inflammatory disease, an autoimmune disease, a liver disease, a spleen disease, a lung disease, a hematological disease, a neurological disease, a gastrointestinal (GI) tract disease, a genitourinary disease, an infectious disease, a musculoskeletal disease, an endocrine disease, a metabolic disorder, an immune disorder, a central nervous system (CNS) disorder, a neurological disorder, an ophthalmic disease, or a cardiovascular disease. In certain embodiments, the disease or disorder is a neurodegenerative disorder. In certain embodiments, the disease or disorder is Alzheimer's disease. In certain embodiments, the disease or disorder is cancer.

In some embodiments, the cell is present in a tissue. In some embodiments, the tissue is a tissue sample from a subject. In some embodiments, the subject is a non-human experimental animal (*e.g.*, a mouse). In some embodiments, the subject is a domesticated animal. In some embodiments, the subject is a human. In some embodiments, the tissue sample comprises a fixed tissue sample. In certain embodiments, the tissue sample is a biopsy (*e.g.,* bone, bone marrow, breast, gastrointestinal tract, lung, liver, pancreas, prostate, brain, nerve, renal, endometrial, cervical, lymph node, muscle, or skin biopsy). In certain embodiments, the biopsy is a tumor biopsy. In certain embodiments, the tissue is brain tissue. In certain embodiments, the tissue is from the central nervous system.

The use of any type of cell in the methods for diagnosing a disease or disorder in a subject disclosed herein is contemplated by the present disclosure. In some embodiments, the cell is a mammalian cell. In certain embodiments, the cell is a human cell. In some embodiments, the cell is a cancer cell. The present disclosure also contemplates performing the methods described herein on multiple cells simultaneously. In some embodiments, the method is performed on multiple cells of the same cell type. In some embodiments, the method is performed on multiple cells of different cell types. The cell types in which gene and protein expression may be mapped using the methods disclosed herein include, but are not limited to, stem cells, progenitor cells, neuronal cells, astrocytes, dendritic cells, endothelial cells, microglia, oligodendrocytes, muscle cells, myocardial cells, mesenchymal cells, epithelial cells, immune cells, and hepatic cells. In certain embodiments, the cells are microglia, astrocytes, oligodendrocytes, excitatory neurons, and/or inhibitory neurons.

Various nucleic acids of interest and proteins of interest may be profiled using the methods disclosed herein. For example, the expression of any nucleic acid or protein of interest that is known or thought to be associated with a disease or disorder (*e.g.,* Alzheimer's disease) may be mapped using the methods disclosed herein and used in the diagnosis of the disease or disorder. In some embodiments, the nucleic acids of interest are selected from the group consisting of *Vsnll, Snap25, Dnm1, Slc6a1, Aldoc, Bsg, Ctss, Plp1, Cst7, Ctsb, Apoe, Trem2, C1qa, P2ry12, Gfap, Vim, Aqp4, Clu, Plp1, Mbp, C4b, Ccnb2, Gpm6a, Ddit3, Dapk1, Myo5a, Tspan7,* and *Rhoc.* In some embodiments, the proteins of interest comprise amyloid beta (Aβ) peptides. In certain embodiments, the Aβ peptides are present in the form of Aβ plaques. In some embodiments, the proteins of interest comprise tau protein. In certain embodiments, the tau protein is present in the cell in the form of inclusion bodies (p-Tau). The presence of Aβ plaques and/or p-Tau may be used, for example, to diagnose a neurodegenerative disease (*e.g.,* Alzheimer's disease) in a subject.

The use of various detecting agents to detect the one or more proteins of interest is contemplated by the present disclosure. In some embodiments, when the one or more proteins of interest comprise Aβ peptides, the Aβ peptides are detected using a small molecule detecting agent (*e.g.,* a small molecule fluorescent dye). In certain embodiments, the small molecule detecting agent is X-34, as has been described herein and, for example, in Styren, S. D. et al. X-34, a fluorescent derivative of Congo red: a novel histochemical stain for Alzheimer's disease pathology. J. Histochem. Cytochem. 2000, 48(9), 1223-1232, which is incorporated herein by reference. In some embodiments, when the proteins of interest comprise tau protein or p-Tau, the tau protein is detected using a p-Tau primary antibody. In certain embodiments, the method further comprises detecting the p-Tau primary antibody with a secondary antibody. In some embodiments, the secondary antibody is conjugated to a detectable label (*e.g.,* a fluorophore).

Using the methods disclosed herein for diagnosing a disease or disorder in a subject, an alteration in the expression of the nucleic acids of interest and/or the proteins of interest being examined, relative to expression in one or more non-diseased cells, may indicate that the subject has the disease or disorder. In some embodiments, an alteration in the expression of the nucleic acids of interest and/or proteins of interest is used to identify cell types in close proximity to plaques. For example, a subject may have or be suspected of having Alzheimer's disease if certain cell types are identified in close proximity to plaques. In some embodiments, the plaques are Aβ plaques. In certain embodiments, the identification of disease-associated microglia cell types (comprising, *e.g.,* high expression of *C1qa, Trem2, Cst7, Ctsb,* and/or *Apoe*) in close proximity to plaques indicates that the subject has or is at risk of having Alzheimer's disease. In certain embodiments, identification of disease-associated astrocyte cell types (comprising, *e.g.,* high expression of *Gfap, Vim,* and/or *Apoe*) in close proximity to plaques indicates that the subject has or is at risk of having Alzheimer's disease. In certain embodiments, the identification of oligodendrocyte precursor cell types (comprising, *e.g.,* high expression of *Cldn11, Klk6, Serpina3n,* and/or *C4b*) in close proximity to plaques indicates that the subject has or is at risk of having Alzheimer's disease. Cells that are in close proximity to plaques may include cells that are within about 10 μm, within about 20 μm, within about 30 μm, within about 40 μm, or within about 50 μm of a plaque.

The methods disclosed herein can also be used to map proteins that have been modified with a post-translational modification of interest (*e.g.,* phosphorylation, glycosylation, ubiquitination, nitrosylation, methylation, acetylation, lipidation, *etc.*). Studying the location of such modified-proteins relative to specific cell types could provide a useful tool, for example, for cancer research, diagnosis, and treatment because protein modifications, such as phosphorylation, play important roles during cancer development and progression.

*Methods of Screening for an Agent Capable of Modulating Gene and/or Protein Expression*

In another aspect, the present disclosure provides methods for screening for an agent capable of modulating gene and/or protein expression of a nucleic acid or protein of interest, or of multiple nucleic acids and/or proteins of interest. For example, the methods for mapping gene and protein expression described herein may be performed in a cell in the presence of one or more candidate agents. The expression of various nucleic acids and/or proteins of interest in the cell (*e.g.,* a normal cell, or a diseased cell) can then be compared to the expression of the same nucleic acids and/or proteins of interest in a cell that was not exposed to the one or more candidate agents. Any alteration in the expression of the nucleic acid(s) and/or protein(s) of interest relative to expression in the cell that was not exposed to the candidate agent(s) may indicate that expression of the nucleic acid(s) and/or proteins of interest is modulated by the candidate agent(s). In some embodiments, a particular signature (*e.g.,* of nucleic acid and protein expression) that is known to be associated with treatment of the disease may be used to identify a candidate agent capable of modulating gene and/or protein expression in a desired manner. The methods described herein may also be used to identify drugs that have certain side effects, for example, by looking for specific nucleic acid and protein expression signatures when one or more cells is treated with a candidate agent or known drug.

In some embodiments, the present disclosure provides a method for screening for an agent capable of modulating gene and/or protein expression comprising the steps of:

a) contacting a cell that is being treated or has been treated with the candidate agent with one or more pairs of oligonucleotide probes, wherein each pair of oligonucleotide probes comprises a first oligonucleotide probe and a second oligonucleotide probe, wherein:

i) the first oligonucleotide probe comprises a portion that is complementary to the second oligonucleotide probe, a portion that is complementary to a nucleic acid of interest, a first barcode sequence, and a second barcode sequence; and
ii) the second oligonucleotide probe comprises a portion that is complementary to the nucleic acid of interest, a portion that is complementary to the first oligonucleotide probe, and a barcode sequence, wherein the barcode sequence of the second oligonucleotide probe is complementary to the second barcode sequence of the first oligonucleotide probe;

b) ligating the 5' end and the 3' end of the first oligonucleotide probe together to produce a circular oligonucleotide;
c) performing rolling circle amplification to amplify the circular oligonucleotide using the second oligonucleotide probe as a primer to produce one or more concatenated amplicons;
d) contacting the cell with one or more detecting agents, wherein each detecting agent binds to a protein of interest;
e) embedding the one or more concatenated amplicons and the one or more detecting agents in a polymeric matrix;
f) contacting the one or more concatenated amplicons embedded in the polymeric matrix with a third oligonucleotide probe comprising a sequence that is complementary to the first barcode sequence of the first oligonucleotide probe; and
g) imaging the one or more concatenated amplicons embedded in the polymeric matrix and the one or more detecting agents embedded in the polymeric matrix to determine the location of the nucleic acids of interest and the proteins of interest in the cell and, optionally, map gene and protein expression;

wherein an alteration in the expression of the nucleic acids of interest and/or the proteins of interest in the presence of the candidate agent relative to expression in the absence of the candidate agent indicates that the candidate agent modulates gene and/or protein expression.

In some embodiments, the candidate agent is a small molecule, a protein, a peptide, a nucleic acid, a lipid, or a carbohydrate. In certain embodiments, the small molecule is an anti-cancer therapeutic agent. In some embodiments, the small molecule comprises a known drug. In some embodiments, the small molecule comprises an FDA-approved drug. In certain embodiments, the protein is an antibody. In certain embodiments, the protein is an antibody fragment or an antibody variant. In certain embodiments, the protein is a receptor. In certain embodiments, the protein is a cytokine. In certain embodiments, the nucleic acid is an mRNA, an antisense RNA, an miRNA, an siRNA, an RNA aptamer, a double stranded RNA (dsRNA), a short hairpin RNA (shRNA), or an antisense oligonucleotide (ASO). Any candidate agent may be screened using the methods described herein. In particular, any candidate agents thought to be capable of modulating gene and/or protein expression may be screened using the methods described herein. In some embodiments, modulation of gene and/or protein expression by the candidate agent is associated with reducing, relieving, or eliminating the symptoms of a disease or disorder, or preventing the development or progression of the disease or disorder. In some embodiments, the disease or disorder modulated by the candidate agent is a genetic disease, a proliferative disease, an inflammatory disease, an autoimmune disease, a liver disease, a spleen disease, a lung disease, a hematological disease, a neurological disease, a gastrointestinal (GI) tract disease, a genitourinary disease, an infectious disease, a musculoskeletal disease, an endocrine disease, a metabolic disorder, an immune disorder, a central nervous system (CNS) disorder, a neurological disorder, an ophthalmic disease, or a cardiovascular disease. In certain embodiments, the disease or disorder is Alzheimer's disease. In certain embodiments, the disease or disorder is cancer.

*Methods for Treating a Disease or Disorder in a Subject*

In another aspect, the present disclosure provides methods for treating a disease or disorder in a subject. For example, the methods for profiling gene expression and protein expression described herein may be performed in a cell from a sample taken from a subject (*e.g.,* a subject who is thought to have or is at risk of having a disease or disorder). The expression of various nucleic acids and/or proteins of interest in the cell can then be compared to the expression of the same nucleic acids and/or proteins of interest in a cell from a non-diseased tissue sample. A treatment for the disease or disorder may then be administered to the subject if any alteration in the expression of the nucleic acids and/or proteins of interest relative to expression in a non-diseased cell is observed. Gene and protein expression in one or more non-diseased cells may be profiled alongside expression in a diseased cell as a control experiment. Gene and protein expression in one or more non-diseased cells may have also been profiled previously, and expression in a diseased cell may be compared to this reference data for a non-diseased cell.

In some embodiments, the present disclosure provides a method for treating a disease or disorder in a subject comprising the steps of:

a) contacting a cell taken from the subject with one or more pairs of oligonucleotide probes, wherein each pair of oligonucleotide probes comprises a first oligonucleotide probe and a second oligonucleotide probe, wherein:

i) the first oligonucleotide probe comprises a portion that is complementary to the second oligonucleotide probe, a portion that is complementary to a nucleic acid of interest, a first barcode sequence, and a second barcode sequence; and
ii) the second oligonucleotide probe comprises a portion that is complementary to the nucleic acid of interest, a portion that is complementary to the first oligonucleotide probe, and a barcode sequence, wherein the barcode sequence of the second oligonucleotide probe is complementary to the second barcode sequence of the first oligonucleotide probe;

b) ligating the 5' end and the 3' end of the first oligonucleotide probe together to produce a circular oligonucleotide;
c) performing rolling circle amplification to amplify the circular oligonucleotide using the second oligonucleotide probe as a primer to produce one or more concatenated amplicons;
d) contacting the cell with one or more detecting agents, wherein each detecting agent binds to a protein of interest;
e) embedding the one or more concatenated amplicons and the one or more detecting agents in a polymeric matrix;
f) contacting the one or more concatenated amplicons embedded in the polymeric matrix with a third oligonucleotide probe comprising a sequence that is complementary to the first barcode sequence of the first oligonucleotide probe;
g) imaging the one or more concatenated amplicons embedded in the polymeric matrix and the one or more detecting agents embedded in the polymeric matrix to determine the location of the nucleic acids of interest and the proteins of interest in the cell; and
h) administering a treatment for the disease or disorder to the subject if an alteration in the expression of the nucleic acids of interest and/or the proteins of interest relative to expression in one or more non-diseased cells is observed.

In some embodiments, gene and protein expression in one or more non-diseased cells is profiled simultaneously using the methods disclosed herein as a control experiment. In some embodiments, the gene and protein expression data in one or more non-diseased cells that is compared to expression in a diseased cell comprises reference data from a time the method was performed on a non-diseased cell previously.

Any suitable treatment for a disease or disorder may be administered to the subject. In some embodiments, the treatment comprises administering a therapeutic agent. In some embodiments, the treatment comprises surgery. In some embodiments, the treatment comprises imaging. In some embodiments, the treatment comprises performing further diagnostic methods. In some embodiments, the treatment comprises radiation therapy. In some embodiments, the therapeutic agent is a small molecule, a protein, a peptide, a nucleic acid, a lipid, or a carbohydrate. In certain embodiments, the small molecule is an anti-cancer therapeutic agent. In some embodiments, the small molecule is a known drug. In some embodiments, the small molecule is an FDA-approved drug. In certain embodiments, the protein is an antibody. In certain embodiments, the protein is an antibody fragment or antibody variant. In certain embodiments, the protein is a receptor, or a fragment or variant thereof. In certain embodiments, the protein is a cytokine. In certain embodiments, the nucleic acid is an mRNA, an antisense RNA, an miRNA, an siRNA, an RNA aptamer, a double stranded RNA (dsRNA), a short hairpin RNA (shRNA), or an antisense oligonucleotide (ASO).

Treatment of any disease or disorder is contemplated by the methods described herein. In some embodiments, the disease or disorder is a genetic disease, a proliferative disease, an inflammatory disease, an autoimmune disease, a liver disease, a spleen disease, a lung disease, a hematological disease, a neurological disease, a gastrointestinal (GI) tract disease, a genitourinary disease, an infectious disease, a musculoskeletal disease, an endocrine disease, a metabolic disorder, an immune disorder, a central nervous system (CNS) disorder, a neurological disorder, an ophthalmic disease, or a cardiovascular disease. In certain embodiments, the disease or disorder is a neurodegenerative disease. In certain embodiments, the disease or disorder is Alzheimer's disease. In certain embodiments, the disease or disorder is cancer.

In some embodiments, the subject is a human. In some embodiments, the sample comprises a biological sample. In some embodiments, the sample comprises a tissue sample. In certain embodiments, the tissue sample is a biopsy (e.g., bone, bone marrow, breast, gastrointestinal tract, lung, liver, pancreas, prostate, brain, nerve, renal, endometrial, cervical, lymph node, muscle, or skin biopsy). In certain embodiments, the biopsy is a tumor biopsy. In certain embodiments, the biopsy is a solid tumor biopsy. In some embodiments, the tissue sample is a brain tissue sample. In certain embodiments, the tissue sample is a central nervous system tissue sample.

*Oligonucleotide Probes*

The present disclosure also provides oligonucleotide probes for use in the methods and systems described herein.

In one aspect, the present disclosure provides a plurality of oligonucleotide probes comprising a first oligonucleotide probe (also referred to herein as the "padlock" probe) and a second oligonucleotide probe (also referred to herein as the "primer" probe), wherein:

i) the first oligonucleotide probe comprises a portion that is complementary to the second oligonucleotide probe, a portion that is complementary to a nucleic acid of interest, a first barcode sequence, and a second barcode sequence; and

ii) the second oligonucleotide probe comprises a portion that is complementary to the nucleic acid of interest, a portion that is complementary to the first oligonucleotide probe, and a barcode sequence;

wherein the barcode sequence of the second oligonucleotide probe is complementary to the second barcode sequence of the first oligonucleotide probe.

All of the oligonucleotide probes described herein may optionally have spacers or linkers of various nucleotide lengths in between each of the recited components, or the components of the oligonucleotide probes may be joined directly to one another. All of the oligonucleotide probes described herein may comprise standard nucleotides, or some of the standard nucleotides may be substituted for any modified nucleotides known in the art.

The first oligonucleotide probe of the plurality of oligonucleotide probes described herein (also referred to herein as the "padlock" probe) includes a first barcode sequence and a second barcode sequence, each made up of a specific sequence of nucleotides. In some embodiments, the first barcode sequence of the first oligonucleotide probe is about 5 to about 15, about 6 to about 14, about 7 to about 13, about 8 to about 12, or about 9 to about 11 nucleotides in length. In some embodiments, the first barcode sequence of the first oligonucleotide probe is 5, 6, 7, 8, 9, 10, 11, 12, 13, 14, or 15 nucleotides in length. In certain embodiments, the first barcode sequence of the first oligonucleotide probe is 10 nucleotides in length. In some embodiments, the second barcode sequence of the first oligonucleotide probe is about 5 to about 15, about 6 to about 14, about 7 to about 13, about 8 to about 12, or about 9 to about 11 nucleotides in length. In some embodiments, the second barcode sequence of the first oligonucleotide probe is 5, 6, 7, 8, 9, 10, 11, 12, 13, 14, or 15 nucleotides in length. In certain embodiments, the second barcode sequence of the first oligonucleotide probe is 10 nucleotides in length. The second barcode sequence provides an additional site of complementarity between the first and the second oligonucleotide probe, providing advantages over previous oligonucleotide probe designs. The second barcode sequence of the first oligonucleotide probe may increase the specificity of the detection of a nucleic acid of interest using the plurality of oligonucleotide probes (e.g., when using the plurality of oligonucleotide probes in any of the methods disclosed herein). The second barcode sequence of the first oligonucleotide probe may also play a role in reducing non-specific amplification of a nucleic acid of interest.

The barcodes of the oligonucleotide probes described herein may comprise gene-specific sequences used to identify nucleic acids of interest. The use of the barcodes on the oligonucleotide probes described herein is further described in, for example, International Patent Application Publication No. WO 2019/199579 and Wang et al., Science 2018, 361, 380, both of which are incorporated herein by reference in their entireties.

The first oligonucleotide probe also comprises a portion that is complementary to the second oligonucleotide probe and a portion that is complementary to a nucleic acid of interest. In some embodiments, the portion of the first oligonucleotide probe that is complementary to the second oligonucleotide probe is split between the 5' end and the 3' end of the first oligonucleotide probe. In some embodiments, the portion of the first oligonucleotide probe that is complementary to a nucleic acid of interest is about 10, about 11, about 12, about 13, about 14, about 15, about 16, about 17, about 18, about 19, about 20, about 21, about 22, about 23, about 24, about 25, about 26, about 27, about 28, about 29, or about 30 nucleotides long. In some embodiments, the first oligonucleotide probe is about 20, about 21, about 22, about 23, about 24, about 25, about 26, about 27, about 28, about 29, about 30, about 31, about 32, about 33, about 34, about 35, about 36, about 37, about 38, about 39, or about 40 nucleotides long. In some embodiments, the first oligonucleotide probe comprises the structure:

5'-[portion complementary to second probe]-[portion complementary to nucleic acid of interest]-[first barcode sequence]-[second barcode sequence]-3'

wherein ]-[ comprises an optional linker (e.g., an optional nucleotide linker). In some embodiments, ]-[ represents a direct linkage between two portions of the first oligonucleotide probe (*i.e.,* a phosphodiester bond).

The second oligonucleotide probe of the plurality of oligonucleotide probes disclosed herein (also referred to herein as the "primer" probe) includes a barcode sequence made up of a specific sequence of nucleotides. In some embodiments, the barcode sequence of the second oligonucleotide probe is about 5 to about 15, about 6 to about 14, about 7 to about 13, about 8 to about 12, or about 9 to about 11 nucleotides in length. In some embodiments, the barcode sequence of the second oligonucleotide probe is 5, 6, 7, 8, 9, 10, 11, 12, 13, 14, or 15 nucleotides in length. In certain embodiments, the barcode sequence of the second oligonucleotide probe is 10 nucleotides in length.

The second oligonucleotide probe also comprises a portion that is complementary to a nucleic acid of interest and a portion that is complementary to a portion of the first oligonucleotide probe. In some embodiments, the first and the second oligonucleotide probes are complementary to and bind different portions of the nucleic acid of interest. In some embodiments, the portion of the second oligonucleotide probe that is complementary to a nucleic acid of interest is about 10, about 11, about 12, about 13, about 14, about 15, about 16, about 17, about 18, about 19, about 20, about 21, about 22, about 23, about 24, about 25, about 26, about 27, about 28, about 29, or about 30 nucleotides long. In some embodiments, the second oligonucleotide probe is about 20, about 21, about 22, about 23, about 24, about 25, about 26, about 27, about 28, about 29, about 30, about 31, about 32, about 33, about 34, about 35, about 36, about 37, about 38, about 39, or about 40 nucleotides long. In some embodiments, the second oligonucleotide probe comprises the structure:
5'-[portion complementary to nucleic acid of interest]-[portion complementary to first probe]-[barcode sequence]-3' wherein ]-[ comprises an optional linker (*e.g.,* an optional nucleotide linker). In some embodiments, ]-[ represents a direct linkage between two portions of the second oligonucleotide probe (*i.e.,* a phosphodiester bond).

In some embodiments, the plurality of oligonucleotide probes comprises a third oligonucleotide probe. In some embodiments, the third oligonucleotide probe comprises a detectable label. In certain embodiments, the detectable label is a fluorophore. As described herein, the third oligonucleotide probe is complementary to the second barcode sequence of the first oligonucleotide probe. In some embodiments, the second barcode sequence of the first oligonucleotide probe is a gene-specific sequence used to identify a nucleic acid of interest. In some embodiments, the third oligonucleotide probe is used to identify a nucleic acid of interest (*e.g.,* through SEDAL sequencing).

*Kits*

Also provided by the disclosure are kits. In one aspect, the kits provided may comprise one or more oligonucleotide probes as described herein. In some embodiments, the kits may further comprise a container (*e.g.,* a vial, ampule, bottle, and/or dispenser package, or other suitable container). In some embodiments, the kit comprises a plurality of oligonucleotide probes as described herein. In some embodiments, the kit further comprises one or more detecting agents (*e.g.,* peptides, proteins such as antibodies, nucleic acids such as aptamers, or small molecules such as fluorescent dyes), wherein each detecting agent binds to a protein of interest. In certain embodiments, the one or more detecting agents comprise an anti-p-Tau antibody. In some embodiments, the kit further comprises a small molecule detecting agent (*e.g.,* X-34, as described herein). In some embodiments, the kit further comprises a third oligonucleotide probe as described herein. The third oligonucleotide probe may comprise a sequence that is complementary to the first barcode sequence of the first oligonucleotide probe. In some embodiments, the third oligonucleotide probe comprises a detectable label (*e.g.,* a fluorophore). In some embodiments, the kit comprises a library made up of two or more sets of oligonucleotide probes, wherein each set of oligonucleotide probes is used to identify a specific nucleic acid of interest. In some embodiments, the kit comprises a library of detecting agents for detecting multiple proteins of interest. In some embodiments, the kits may further comprise other reagents for performing the methods disclosed herein (*e.g.,* cells, a ligase, a polymerase, amine-modified nucleotides, primary antibodies, secondary antibodies, buffers, and/or reagents for making a polymeric matrix (*e.g.,* a polyacrylamide matrix)). In some embodiments, the kits are useful for profiling gene and protein expression in a cell. In some embodiments, the kits are useful for diagnosing a disease (*e.g.,* Alzheimer's disease) in a subject. In some embodiments, the kits are useful for screening for an agent capable of modulating gene and/or protein expression. In some embodiments, the kits are useful for diagnosing a disease or disorder in a subject. In some embodiments, the kits are useful for treating a disease or disorder in a subject. In certain embodiments, a kit described herein further includes instructions for using the kit.

*Methods, Apparatus, and Non-Transitory Computer-Readable Storage Medium for Identifying Spatial Variations of Cell Types in At Least One Image*

In various aspects, the present disclosure provides methods for identifying spatial variations of cell types in one or more images (*i.e.,* looking at variations in the spatial distribution of specific cell types relative to one another between multiple samples, for example, a healthy tissue compared to a diseased tissue). In some embodiments, the one or more images are obtained using any of the methods disclosed herein. **FIG. 15** is a flow diagram of one embodiment of a method related to identifying spatial variations of cell types in at least one image. The process flow 1500 may be performed by at least one computer processor. In some embodiments, there may be at least one non-transitory computer-readable storage medium encoded with a plurality of instructions that, when executed by at least one computer processor, perform process flow 1500. Process flow 1500 comprises step 1502, step 1504, step 1506, step 1508, and step 1510. In step 1502, the at least one computer processor receives, for each of a plurality of cells in the at least one image, a spatial location of the cell in the at least one image. In step 1504, the at least one computer processor receives, for each of a plurality of proteins in the at least one image, a spatial location of the protein in the image. In step 1506, the at least one computer processor, for a first protein of the plurality of proteins, determines a number of cells of a first cell type having a distance to the first protein less than a threshold distance, wherein the distances are determined based on at least some of the spatial locations of the plurality of cells and at least some of the spatial locations of the plurality of proteins. In step 1508, the at least one computer processor, based on the number of cells of the first cell type, identifies a spatial variation in cells of the first cell type in the at least one image. In step 1510, the at least one computer processor outputs an indication of the spatial variation in cells of the first cell type in the at least one image.

**FIG. 16** is a flow diagram of one embodiment of a method related to identifying a spatial variation (*i.e.,* looking at variations in the spatial distribution of specific cell types relative to one another between multiple samples, for example, a healthy tissue compared to a diseased tissue) in cells of a first cell type in at least one image. The process flow 1600 may be performed by at least one computer processor. In some embodiments, there may be at least one non-transitory computer-readable storage medium encoded with a plurality of instructions that, when executed by at least one computer processor, perform process flow 1600. Process flow 1600 comprises step 1602, step 1604, step 1606, and step 1608. In step 1602, the at least one computer processor, for cells of the plurality of cells having a distance to the first protein less than the threshold distance, determines a first percentage of the cells that are associated with the first cell type. In step 1604, the at least one computer processor determines a second percentage of cells of the plurality of cells in the at least one image that are associated with the first cell type. In step 1606, the at least one computer processor compares the first percentage to the second percentage to obtain a comparison result. In step 1608, the at least one computer processor identifies the spatial variation in cells of the first cell type in the at least one image using the comparison result.

**FIG. 17** is a flow diagram of one embodiment of a method related to capturing at least one image using a camera. The process flow 1700 may be performed by at least one computer processor. In some embodiments, there may be at least one non-transitory computer-readable storage medium encoded with a plurality of instructions that, when executed by at least one computer processor, perform process flow 1700. Process flow 1700 comprises step 1702 and step 1704. In step 1702, the at least one computer processor captures a first image of the plurality of images, the first image being used to determine the spatial locations of the plurality of cells. In step 1704, the at least one computer processor captures a second image of the plurality of images, the second image being used to determine the spatial locations of the plurality of proteins.

**FIG. 18** is a flow diagram of one embodiment of spatially aligning spatial locations from a plurality of images. The process flow 1800 may be performed by at least one computer processor. In some embodiments, there may be at least one non-transitory computer-readable storage medium encoded with a plurality of instructions that, when executed by at least one computer processor, perform process flow 1800. Process flow 1800 comprises step 1802 and step 1804. In step 1802, the at least one computer processor spatially aligns the spatial locations of the plurality of cells from the first image with the spatial locations of the plurality of proteins from the second image. In step 1804, the at least one computer processor, based on the spatially aligned spatial locations, determines the number of cells of the first cell type having the distance to the first protein less than the threshold distance.

**FIG. 19** is a flow diagram of one embodiment of a method related to determining a number of cells of a first cell type having a distance to a first protein less than a threshold distance. The process flow 1900 may be performed by at least one computer processor. In some embodiments, there may be at least one non-transitory computer-readable storage medium encoded with a plurality of instructions that, when executed by at least one computer processor, perform process flow 1900. Process flow 1900 comprises step 1902 and step 1904. In step 1902, the at least one computer processor determines a minimum distance from the cell of the first cell type to a nearest protein of the plurality of proteins. In step 1904, the at least one computer processor compares the minimum distance to the threshold distance.

**FIG. 20** is a flow diagram of one embodiment of a method related to associating cells with cell types. The process flow 2000 may be performed by at least one computer processor. In some embodiments, there may be at least one non-transitory computer-readable storage medium encoded with a plurality of instructions that, when executed by at least one computer processor, perform process flow 2000. Process flow 2000 comprises step 2002 and step 2004. In step 2002, the at least one computer processor receives genetic information of the cell (e.g., information about gene and/or protein expression). In step 2004, the at least one computer processor associates with the cell, based on the genetic information of the cell, a cell type from a plurality of cell types, wherein the plurality of cell types includes the first cell type.

An illustrative implementation of a computer system 2100 that may be used in connection with any of the embodiments of the disclosure provided herein is shown in **FIG. 21**. The computer system 2100 may include one or more computer processors 2110 and one or more articles of manufacture that comprise non-transitory computer-readable storage media, for example, memory 2120 and one or more non-volatile storage media 2130. The computer processor 2110 may control writing data to and reading data from the memory 2120 and the non-volatile storage device 2130 in any suitable manner. To perform any of the functionality or methods described herein, such as the methods associated with process flows 1500, 1600, 1700, 1800, 1900, 2000, or for example, identifying spatial variations of cell types in at least one image (*i.e.,* looking at variations in the spatial distribution of specific cell types relative to one another between multiple samples, for example, a healthy tissue compared to a diseased tissue), identifying a spatial variation in cells of a first cell type in at least one image, capturing at least one image using a camera, spatially aligning spatial locations from a plurality of images, determining a number of cells of a first cell type having a distance to a first protein less than a threshold distance, or associating cells with cell types, *etc.,* the processor 2110 may execute one or more computer processor-executable instructions stored in one or more non-transitory computer-readable storage media, for example the memory 2120, which may serve as non-transitory computer-readable storage media storing processor-executable instructions for execution by the processor 2110.

*Systems for Mapping Gene and Protein Expression in a Cell*

The present disclosure also provides systems for mapping gene and protein expression in a cell. In some embodiments, the systems comprise a) a cell; and b) one or more pairs of oligonucleotide probes comprising a first oligonucleotide probe and a second oligonucleotide probe, wherein:

i) the first oligonucleotide probe comprises a portion that is complementary to the second oligonucleotide probe, a portion that is complementary to a nucleic acid of interest, a first barcode sequence, and a second barcode sequence; and

ii) the second oligonucleotide probe comprises a portion that is complementary to the nucleic acid of interest, a portion that is complementary to the first oligonucleotide probe, and a barcode sequence, wherein the barcode sequence of the second oligonucleotide probe is complementary to the second barcode sequence of the first oligonucleotide probe.

In some embodiments, the system further comprises a microscope (*e.g.*, a confocal microscope). In some embodiments, the system further comprises a computer. In some embodiments, the system further comprises software for performing microscopy and/or image analysis (*e.g.,* using any of the image analysis methods described herein). In some embodiments, the system further comprises a ligase. In some embodiments, the system further comprises a polymerase. In some embodiments, the system further comprises amine-modified nucleotides. In some embodiments, the system further comprises reagents for making a polymeric matrix (*e.g.,* a polyacrylamide matrix). The cell in the systems of the present disclosure may be any of the cell types disclosed herein. In some embodiments, the system comprises multiple cells. In some embodiments, the cells are of different cell types. In certain embodiments, the cells are present in a tissue. In some embodiments, the tissue is a tissue sample provided by or from a subject. In certain embodiments, the subject is a human.

**EXAMPLES**

*Example 1: Development of the STARmap Pro Method*

Amyloid-β plaques and neurofibrillary tau tangles are the neuropathologic hallmarks of Alzheimer's disease (AD), but the molecular events and cellular mechanisms underlying AD pathophysiology remain poorly understood in the space and time dimensions. The STARmap Pro method was developed and applied to simultaneously detect single-cell transcriptional states and protein disease markers (amyloid-β aggregates and tau pathology) in the brain tissues of an AD mouse model. With joint analyses of pseudotime trajectory construction and differential gene expression at subcellular resolution (200 nm), a high-resolution spatial map of cell types and states in AD pathology was constructed. Disease-associated microglia (DAM) cells were found to form an inner shell directly contacting amyloid-β plaques from the early onset of disease progression, while disease-associated astrocytes (DAA) and oligodendrocyte precursor cells (OPC) were enriched in the outer shells surrounding amyloid-β plaques at later disease stages. Hyperphosphorylated Tau primarily emerged in excitatory neurons and axonal processes. Furthermore, disease-associated gene pathways were pinpointed and verified across diverse cell types, suggesting inflammatory and gliosis processes in glia cells and declines in adult hippocampal neurogenesis.

The previous STARmap method was incompatible with histological staining (immuno- or small-molecule staining) and limited to detect 1024 genes. To overcome such limitations in STARmap Pro, the experimental protocol was first streamlined to incorporate antibody (AT8 antibody, detecting phosphorylated tau) and dye staining (X-34, detecting Aβ plaque) in library preparation and *in situ* sequencing steps (FIGs. 1B, 8A, and 8B). Intracellular mRNAs of the brain tissue were detected by a pair of DNA probes (primer and padlock, FIG. 1C), and enzymatically amplified as cDNA amplicons. The proteins are labeled with a primary antibody, then both cDNA amplicons and the primary antibody are embedded in a hydrogel matrix. Each cDNA amplicon contains a gene-unique identifier (barcode) that is read-out through *in situ* SEDAL (sequencing with error-reduction by dynamic annealing and ligation) sequencing (Wang *et al.,* 2018), followed by fluorescent protein staining (secondary antibody staining and small-molecule dye X-34 staining) to visualize protein signals.

The gene-coding barcode in the DNA probes was then expanded from 5 nucleotides (nt) to 10 nt ($10^6$ coding capacity) that is sufficient to encode more than 20,000 genes. Furthermore, the additional 5-nt barcode was strategically designed near the ligation site, which increases the specificity of gene detection by reducing non-specific amplification of mismatched primer-padlock pairs (FIG. 8C). The barcode design was then validated by incorporating mismatches near the ligation site. The results showed undetectable signals, suggesting that the STARmap Pro method has high specificity (FIGs. 8D and 8E).

To investigate how AD-related pathology, including amyloid deposition and hyperphosphorylated tau, influences the transcriptional response at a cellular level, eight rounds of *in situ* sequencing were performed to map 2766 genes, and one round of post-sequencing imaging was performed (FIG. 1D) to locate Aβ plaques and phosphorylated tau (p-Tau) in thin coronal sections of the brains from TauPS2APP mice and control mice. Transgenic TauPS2APP mice express mutant forms of human Amyloid Precursor Protein (APP K670N/M671L) and Presenilin 2 (N141I) that produce high levels of A beta (Aβ) peptides leading to plaque formation, in addition to expressing mutant forms of human MAPT (P301L) that become hyperphosphorylated and aggregate. Sections were analyzed at 8 months (an age where tau and Aβ pathology have set and are expanding) and 13 months (a more advanced disease stage with severe pathology and elevated neuroinflammatory activity) (Lee *et al.,* 2021). The cortex and hippocampus, two of the most susceptible regions in AD, were primarily investigated. The spatial protein signals in TauPS2APP matched with the results of previous reports (Grueninger *et al.* 2010), Lee et al. Neuron 2021) that Aβ plaque signals were distributed in both the cortex and hippocampus regions, and p-Tau immunoreactivity was mainly distributed in the CA1 regions of the hippocampus (FIG. 1D), suggesting the fidelity of protein detection in STARmap Pro. 19,932 cells from two TauPS2APP mice and 17,135 cells from two control mice (non-transgenic littermates) were mapped at subcellular resolution (with a voxel size of 95 nm x 95 nm x 346 nm, FIG. 1A). After projecting three-dimensional RNA reads to two-dimensional planes, cell segmentation and quality-control filtration of single-cell transcriptional profiles (> 1000 pixels or 9.025 $\mu m^2$ in area and > 68 RNA reads per cell, also see STAR Methods), the remaining 33,106 cells pooled from all 4 samples were subjected to downstream analysis.

*Example 2: Hierarchical Cell Classification and Spatial Analysis*

To identify cell types from the STARmap Pro data, a hierarchical clustering strategy was adopted in which top-level clustering serves to classify cells into common cell types shared by all samples, and sub-level clustering serves to further identify disease-associated subtypes. During top-level clustering, the Leiden algorithm was applied to the low dimensional representation of all transcriptomic profiles with Uniform Manifold Approximation and Projection (UMAP) (McInnes *et al.,* 2018; Traag *et al.,* 2019). Thirteen major cell types were identified with unambiguous annotations according to previously reported gene markers and tissue morphology (FIG. 2A, FIG 9A). For example, excitatory neurons were annotated by their high expression levels of genes related to the ion channel and synaptic signaling such as *Vsnl1, Snap25,* and *Dnm1.* Inhibitory neurons were separated by their enrichment of gamma-aminobutyric acid (GABA) transporters *Slc6a1.* Other non-neuronal cell type specific markers such as *Aldoc* for astrocytes, *Bsg* for endothelial cells, *Ctss* for microglia, and *Plp1* for oligodendrocytes were used to annotate corresponding clusters as well (FIG. 9A). The UMAP plots of TauPS2APP samples showed differential distribution of astrocyte, microglia, oligodendrocyte, and dentate gyrus (DG) cells in comparison with those of control samples, suggesting disease-associated cell subtypes (FIG. 2A). Thus, the heterogeneity within each major cell type was investigated, and 24 sub-level clusters were identified based on their transcriptomic signatures (FIG. 2B).

Benefiting from the spatial information reserved at subcellular resolution, a spatial cell-type atlas was generated along with histopathology hallmarks in the cortex and hippocampus regions of the four samples (FIG. 2C and FIG. 9D). All spatial cell atlases of different samples showed similar anatomic structure of cortical and hippocampal regions, confirming the robustness and reliability of top-level clustering results. Yet a few major cell types showed varied spatial distribution near Aβ plaque in TauPS2APP samples, and p-Tau signal became evident in the hippocampal region at 13 months (FIG. 2C). To further quantify the cell-type composition in spatial relationship to Aβ plaque, the distance from the centroid of each cell to the edge of its nearest plaque was measured. Cells were then counted as a function of distance (in five 10-$\mu m$ concentric rings) from Aβ plaques and grouped based on major cell types annotation (FIGs. 2D, 2E, and 9F). Among the 13 major cell types, microglia, astrocytes, and oligodendrocyte precursor cells (OPC) were enriched around the plaques in TauPS2APP samples in comparison with the overall cell-type composition. Microglia was the most prevalent cell type in the 10 $\mu m$ ring. OPC and astrocytes showed moderate enrichment at the 20-30 $\mu m$ distance. The rest of the cell types were relatively depleted in the 10-20 $\mu m$ ring.

The top-level cell clustering and spatial analyses revealed that microglia, astrocytes, OPC, oligodendrocytes, and neuronal cells showed changes in transcriptional profiles, spatial locations, or both features. These cell types were thus selected for in-depth sub-level clustering analysis to pinpoint disease-associated cell subtypes and gene pathways. AD is an inherently progressive disease which involves continuous molecular and cellular variations. Yet, clustering analysis only identifies distinct cell types and cannot describe continuous cell-state transitions. In order to capture the gradients of cell states along disease progression and determine the relationship among different subtypes, Monocle pseudotime analysis (Cao *et al.,* 2019), a widely used computational tool for reconstructing cell differentiation trajectory, was used in complement with subcluster analysis in the following sections.

*Example 3: Disease-Associated Microglia Directly Contacts* Aβ *Plaque from Early Disease Onset*

After confirming that microglia are enriched in the immediate vicinity of plaques (FIG. 2E), the heterogeneity within the microglia population was then investigated by sub-clustering analysis of transcriptomic profiles. Three subpopulations were identified and named as Micro1 *(n* = 779), Micro2 *(n* = 415), and Micro3 *(n* = 529) respectively (FIG. 3A). Micro1 and Micro2 subtypes were present in all four samples, while the Micro3 population greatly expanded from 8 months to 13 months in the TauPS2APP samples and was almost absent in control samples (FIG. 3A). Furthermore, the Micro3 subcluster expressed high levels of *C1qa, Trem2, Cst7, Ctsb,* and *Apoe,* which are known markers of disease-associated microglia (DAM) and associated with neurodegeneration (Friedman *et al.,* 2018; Keren-Shaul *et al.,* 2017) (FIG. 3B). Given its strong association with disease model and agreement with known DAM gene markers, Micro3 was annotated as DAM.

The continuous gradients of cell-state transition were then further reconstructed in microglia by pseudotime trajectory analysis. The microglia population showed a linear pseudotime trajectory that aligned very well with the real disease progression timeline. The microglia in control samples were enriched at the starting point of the trajectory, while those in TauPS2APP samples kept shifting along the continuous path from 8 months to 13 months (FIGs. 3C-3E). While the Micro3 subcluster presented at the later pseudotime points along the trajectory, both Micro1 and Micro2 spread from early to middle pseudotime values along the trajectory (FIGs. 3D-3E). Next, the spatial distribution of the three microglia subclusters was analyzed. It was observed that: (i) all three subclusters were present in both cortical and hippocampal regions of TauPS2APP samples (FIGs. 3F-3G); (ii) Micro3 almost exclusively presented in TauPS2APP mice, but not in control samples (FIG. 3H, FIG. 10A); (iii) Micro2 showed higher density in the hippocampal region than in the cortical region in both TauPS2APP and control samples (FIG. 3H). For the cell-type composition analysis around the Aβ plaque, it was observed that: (i) at 8 months, 64.5% of all cells within the 10 μm ring around the plaque were microglia, and in particular, 73.3% of the microglia are Micro3 (FIG. 10B); (ii) at 13 months, the numbers increased to 75.8% and 81.5%, respectively (FIG. 3H, Bottom). Furthermore, although Micro2 is not strictly disease-specific, it was significantly enriched within the 10-μm ring near plaques in comparison with other (non-microglia) cell types (FIG. 3H and FIG. 10B), suggesting that Micro2 also responds to Aβ plaque. The spatial cell atlas of microglia was further plotted with the pseudotime values, and the pseudotime distribution near plaque was computed using similar concentric ring quantification as was used with cell-type analysis. The results showed expected observations where the microglia cells near the plaques (within 10 μm) had higher pseudotime values than cells far away from plaques, revealing a spatiotemporal trajectory of microglia activation as it migrates toward Aβ plaque (FIGs. 10C and 10D).

To get a more comprehensive understanding of microglia response to AD pathology at the molecular level, the gene expression of microglia in the TauPS2APP mice versus control mice was then compared (FIG. 10E). Most of the upregulated DEGs identified in the AD sample were DAM gene markers involved in biological processes such as regulation of cell activation (*Cst7, Apoe, Cd9,* and *Clec7a*), inflammatory response (*i.e., Trem2, Ccl6,* and *Cd68),* antigen processing and presentation (*i.e., Ctss* and *H2-k1*). On the other hand, the downregulated genes of microglia in diseased samples were related to regulation of anatomical structure morphogenesis *(i.e.,* Sparc, Numb, Cdc42), endocytosis (*Calm1* and *Bin1*), and positive regulation of transport (*i.e., P2ry12* and *Glud1*). A subset of the most significant DEGs and cell type markers were selected, and their expression variations were validated in the 64 gene dataset with an additional set of mice (FIG. 3I).

*Example 4: Disease-Associated Astrocytes Emerge Near the Plaque-DAM Complex at a Later Stage*

Astrocytes were another non-neuronal cell population with a significant difference between TauPS2APP and the control samples. Sub-level clustering analysis of the astrocytes identified three transcriptomically distinct subpopulations Astro1 *(n* = 1,068), Astro2 *(n* = 1,271), and Astro3 (*n* = 545) (FIG. 4A). All three subclusters were observed in all samples; however, very few Astro3 cells presented in control mice, and the Astro3 population greatly expanded from 8 months to 13 months in TauPS2APP mice (FIG. 4A). Previous studies identified the disease-associated astrocytes (DAA) with characteristic upregulation of *Gfap,* and the gene markers of Astro3 resembled those of DAA (Habib *et al.,* 2020) (*i.e., Gfap, Vim,* and *Apoe,* FIG. 4B). Thus, the Astro3 cluster was annotated as DAA because of the similarity of its transcriptomic profile with DAA and its association with AD models. In addition, Astro1 and Astro2 correspond to previously reported low- and intermediate-*Gfap* cell populations (Habib *et al.,* 2020).

Despite the apparent linear gradient of many marker genes across Astro1-3 subtypes, a bifurcation path was observed from the pseudotime trajectory analysis of the astrocyte population (FIG. 4C). By visualizing the subcluster annotation along the pseudotime trajectory, it was identified that the longest path (lower path) matched with the gradient from Astro1 (the starting point) and Astro 2 (the ending point) with no obvious association with disease progression. In contrast, the short branch from the bifurcation point (upper path) represented the transition from non-diseased states (mostly Astro2) to Astro3 (DAA) (FIG. 4D).

Spatial cell map of astrocytes further showed that Astro1 locates near to cortical and hippocampal neurons, while Astro2 is enriched in corpus callosum and stratum lacunosum-moleculare (FIGs. 4F-4H and FIG. 11A). Cell-type analysis in relation to tissue pathology revealed that while DAMs directly interact with plaque within 10 $\mu$m to form a DAM-plaque complex, Astro3 (DAA) was enriched relatively further away (20-40 $\mu$m) from the plaque in both the cortical and hippocampal regions. In the cortex, corpus callosum, and hippocampal regions, about 3.1%, 7.8%, or 10.1%, respectively, of the cells in the 20-40 $\mu$m range around plaques were DAA in the 13-month TauPS2APP sample (FIG. 4H). In addition, Astro2 was also enriched near the plaques at 8 months and contributed to 3-16% of all cells in the 20-40 $\mu$m range; however, the presence of Astro2 dropped to 2-7% at 13 months (FIGs. 4H and 11B). The observed shift of the Astro2 to Astro3 population from 8 to 13 months near plaque, in combination with the disease-associated transcriptomic pseudotime trajectory from Astro2 to Astro3 (FIG. 4D, upper path), suggests that there might be a conversion of Astro2 to Astro3 (DAA) near plaques along disease progression.

Most of DEGs in the astrocytes of TauPS2APP and control samples were related to glial cell differentiation (*i.e.,* *Gfap, Vim, Clu,* and *Stat3*) and the extracellular matrix (*i.e., Ctsb* and *Bcan*) (FIG. 11E). By showing the gene expression level of top DEGs identified on the pseudotime embedding, the expression profiles of *Gfap* and *Vim* showed the best correlation with DAA and resembled molecular gradients along the disease-associated branch (FIG. 4I, upper path). Enriched GO terms from the DEGs include negative regulation of neuron projection development and positive regulation of glial cell proliferation, as well as intermediate filament organization (FIG. 4J), suggesting overall gliosis process in the AD disease model. Again, a subset of the most significant DEGs and cell type markers were selected, and their expression variations were observed in the 64 gene validation dataset as well (FIG. 4K).

*Example 5: Oligodendrocyte Subtypes and Precursor Cells are Enriched in the Intermediate Vicinity of Plaques*

Sub-level clustering analysis was next performed on oligodendrocyte lineage cells, and four clusters were identified (FIGs. 5A and 5B): Oligo1 *(n* = 4,295), Oligo2 *(n* = 181), Oligo3 *(n* = 490), and OPC *(n* = 549). The gene marker *Plp1* of oligodendrocytes exhibited relatively even expression across all clusters, whereas *Klk6* and *Cldn11* marked Oligo2 and Oligo3 populations, respectively (FIG. 5B). The TauPS2APP disease model and control samples showed similar distributions of all four cell populations on the UMAP embedding (FIG. 5A).

The pseudotime trajectory analysis of the combined population of OPC and oligodendrocyte cells recapitulated the known differentiation path from OPC to mature oligodendrocytes (FIG. 5C, lower trajectory). Similar to astrocytes, a disease-associated trajectory diverged beside the main path from OPC to oligodendrocytes (FIG. 5C, upper branch). The oligodendrocytes from TauPS2APP samples were greatly enriched around the disease-associated trajectory branch in comparison with 8-month TauPS2APP and control samples (FIG. 5C). In addition, the disease-associated trajectory contained all three Oligo1-3 subtypes (FIG. 5D). Overall, the pseudotime distribution of cells in the diseased sample at 13 months had a significantly higher mean compared to both the control sample at 13 months and the diseased sample at the 8-month time point (FIG. 5E).

From the spatial maps and the cell density calculations (FIGs. 5F-5H, top, and FIG. 12A), it was observed that the cell density of the Oligo2 and Oligo3 population in TauPS2APP was 100-200% higher in the diseased sample at 13 months in comparison with the control sample. Oligo2 and Oligo3 were thus annotated as disease-enriched oligodendrocytes. From cell-type distribution analysis near A$\beta$ plaques, it was found that OPCs were enriched in the 20-40 $\mu$m ring around plaque in TauPS2APP mice at both 8 and 13 months (FIGs. 5H and 14B). In addition, Oligo2 and Oligo3 were also enriched at the 20-40 $\mu$m distance from plaque in TauPSAPP mice, especially in the subcortical region.

Through the DEG analysis in oligodendrocytes from the TauPS2APP mice versus control mice comparison, a group of genes was identified and verified (*i.e., Cldn11, Klk6, Serpina3n,* and *C4b*) that were strongly upregulated in 13-month-old TauPS2APP mice. In contrast, the fold change of DEGs between 8-month-old TauPS2APP and control mice was less significant, and some of the DEGs showed inconsistent changes in later experimental validations (FIGs. 5K and 14F). GO term analysis indicated key biological processes of oligodendrocytes in cytokine production, regulation of neuron death, and regulation of synaptic plasticity and myelination during disease progression. Further DEG analysis along the disease-associated pseudotime trajectory further confirmed the disease association of *Klk6, Cldn11,* and *C4b* genes in oligodendrocytes (FIG. 5J).

*Example 6: Responses of Neuronal Cells to* A$\beta$ *Plaques and Tau Tangles*

Besides cellular changes in non-neuronal cells, transcriptomic responses in neurons are critical to understand the mechanism of neurodegeneration. Sub-level clustering analysis of the neuronal cell population was conducted, and eight excitatory neuron subclusters and four inhibitory neuron subclusters were identified. As visualized in the spatial cell map of neurons (FIGs. 6A, 6B, 13A, and 13B), the four subtypes of cortical excitatory neurons correspond to different cortical layers (CTX-Ex1-4). The excitatory neuronal types in the hippocampal region are the same as the major types (DG, CA1, CA2, and CA3), and no additional subcluster was identified within each major type. Among the four subtypes of inhibitory neurons, *Pvalb* and *Sst* neurons were enriched in the cortex, while *Cnr1* and *Lamp5* neurons were overrepresented in the hippocampus.

Neuron-type compositions and their transcriptomic profiles were investigated next in relation to Aβ plaques. In general, because of the migration and expansion of non-neuronal cells (microglia, astrocytes, OPCs, and oligodendrocytes) near plaques, the percentage of all neuronal cells was low near plaque and positively correlated with their distance to plaque. However, in the hippocampal region, the neuronal population around plaques comprised mostly DG cells, which is consistent with the observation that the majority of the plaques emerged near DGs. A recent report showed that adult hippocampal neurogenesis (AHN) activity in DG sharply declines in human patients of AD. It was thus tested whether Aβ plaques in the TauPS2APP mice model also impact the AHN of DG by pseudotime trajectory analysis. At 8 months, since there were very few plaques near DG, the pseudotime distribution of DG cells in TauPSAPP and control mice were indistinguishable. However, at 13 months, accompanied with the increased number of plaques near DG, the pseudotime trajectory diverged into two branches corresponding to the DG populations in TauPS2APP and control samples, respectively. Additionally, the molecular response of the neurons in the DG region was further investigated since it is related to neurogenesis. According to the pseudotime analysis, samples at 13 months followed two different paths. In the diseased sample at 13 months, genes such as *Dapk1* were upregulated, which was also involved in neuronal death regulation (FIGs. 6I and 6J).

In order to investigate the alteration induced by tau tangles, the tau protein intensity inside each cell was quantified by calculating the ratio of the number of tau positive pixels to the total pixel area of each cell. As tau tangles in axons could be wrongly attributed to cells, a threshold was then used to select the tau positive neurons in each sample. In this case, the majority of tau positive excitatory neurons in the sample at 8 months were in the CTX-Ex2 population, whereas at 13 months, most of them were found in the CA1 region. For inhibitory neurons, most tau positive cells were from the Pvalb population, whereas at 13 months, most of them came from the Sst population (FIG. 6E). The tau signal around the plaques was also quantified, and it was discovered that in the cortex region, the tau signal was enriched near the plaque, but no pattern was observed in the subcortical region (FIG. 6F).

Finally, the combined effects of Aβ plaques and tau tangles on neurons were examined by pooling all subtypes into two large categories of excitatory and inhibitory neurons and analyzing DEGs of neurons between the TauPS2APP and control samples. Overall, the DEGs identified from excitatory and inhibitory neuronal cell populations were highly consistent. GO term analysis showed that the identified DEGs were enriched in biological processes of cell cycle regulation (*Ccnb2*), neuronal differentiation (*Gpm6a*), and regulation of neuronal death (*Ddit3*) (FIGs. 6G and 6H).

*Example 7: Integrative Analysis of Disease-Associated Cells in AD Pathology*

The analysis described above has been focused on dissecting disease-associated subtypes and DEGs within individual major cell types. In order to synthesize a comprehensive picture of AD gene pathways from multiple cell types, Gene Set Enrichment Analysis (GSEA) was performed using DEGs from four major cell types (microglia, astrocytes, oligodendrocytes, and neurons). As shown in the GO term enrichment heatmap (FIGs. 7A and 14A), at 13 months, most of the significantly enriched terms of non-neuronal cells in the biological process were glia cell differentiation and glia cell development, which recapitulated the appearance of disease-associated populations, while those of neurons involve synaptic signaling, cognition, and regulation of synaptic plasticity. Then, all the annotations enriched in targeted cell types were gathered and grouped based on their similarity. A GO enrichment map was generated and showed both cell-type-specific and shared annotations of major participants in AD (FIG. 14B). As the population directly interacted with the pathological site, cell-type specific terms related to immune response, inflammatory response, and lysosome were enriched in microglia. Terms related to cell motility, morphogenesis, and differentiation were shared across multiple cell types.

Besides the DEGs identified in the comparison of AD and the control samples, the spatial DEGs were also calculated using cells close to the plaques (within 25 $\mu$m) compared with cells far away from plaques (distance larger than 25 $\mu$m). The genes specifically upregulated in the near plaque regions were regarded as plaque induced genes (PIGs). Sixteen PIGs were identified at 8 months and 29 PIGs were identified in the AD sample at 13 months (FIGs. 7B and 14G). All 16 of the PIGs in the AD 8-month sample were included in the PIGs in the AD 13-month sample (FIG. 7C). It was found that the PIGs enriched within 10$\mu$m from plaque were mainly DAM marker genes, such as *Trem2, Cst7, Ctsb, Apoe,* and *Cd9* (FIGs. 7B and 14G). The DAA marker *Vim* was upregulated in the 20 to 30 $\mu$m regions from plaque in the AD 13-month sample (FIG. 7B). This is consistent with the previous finding that DAA was enriched in the 20 to 30 $\mu$m regions from plaque in the AD 13-month sample (FIG. 4H). The PIGs uncovered were also compared with previously reported PIGs in 18-month AppNL-G-F mice. The results showed that 58.9% (17 in 29) of the PIGs in the AD 13-month sample and 87.5% (14 in 16) of the PIGs in the AD 8-month sample overlapped with reported PIGs (FIG. 7C). The high overlap ratio implied the accuracy of STARmap Pro to detect spatially reserved RNA signals.

To get a more comprehensive and quantitative understanding of the spatial relationship between each cell type and Aβ plaque, the average Euclidean distance was computed from plaque, DAM, DAA, and neurons to their nearest neighbors (FIGs. 14C and 14E). Compared with the shuffled reference (FIGs. 14D and 14F), it was found that DAM was the closest neighbor of amyloid plaque. Instead of plaque, the DAA had a shorter distance to DAM, suggesting DAA contacts DAM-plaque complex. To further validate this finding, the cells were then quantified at different distance intervals from each plaque and grouped based on their cell type identity (FIG. 7D). In summary, the DAM was closest to the plaque and enriched in the 0-20 μm regions. DAA and OPC were relatively similar in terms of the distance to the plaque and were distributed in the 20-40 μm regions. Oligodendrocytes and neurons had higher density in the outer regions (FIG. 7D). Based on this spatial information, a diagram of cell distribution patterns around plaque in the AD 8-month sample and AD 13-month sample was generated (FIG. 7E). In both stages, DAMs directly contact plaques to form an inner core. Along with disease progression, more DAAs appeared and were enriched in the second nearest plaque regions. The enrichment of OPC in the near plaque regions was also shown in the diagram of the TauPS2APP 13-month sample. The marker genes or DEGs in these targeted cell types were also labeled in the diagram.

*Discussion*

STARmap Pro was developed for *in situ* detection of RNA and protein signals in the same tissue section at single cell resolution. Based on STARmap, its detection capability and specificity have been further improved, and new functionality to profile RNA and protein simultaneously at single-cell resolution, while preserving spatial information, has been provided. STARmap Pro offers an opportunity to study biological systems in a more comprehensive way and enable multi-modal spatial gene expression analysis. The methods described herein have the potential to be widely used in pathology research, as proteins are the most common disease hallmarks. Using STARmap Pro to explore the gene expression changes near disease hallmarks will advance understanding of disease pathogenesis. The original STARmap detects only RNA signals and cannot represent the abundance of proteins accurately or detect protein modifications. In contrast, the immunostaining strategy of STARmap Pro can not only distinguish different protein kinds, but also protein modifications, providing a useful tool for cancer research since protein modifications, such as phosphorylation, play important roles during cancer development and progression.

STARmap Pro was applied to detect RNA and AD pathology protein signals in the AD mouse model. With the spatially reserved single-cell RNA signal, a cell type spatial map was generated to reveal the cell distribution pattern in relation to Aβ plaques, and it was found that microglia were significantly enriched in the near plaque regions. Sub-clustering analysis of the five targeted cell types (microglia, astrocytes, oligodendrocytes, excitatory neurons, and inhibitory neurons) was then performed. The results provide a comprehensive spatial map that identified the appearance of DAM and DAA, as well as their enrichment behavior in the near Aβ plaque regions. This was consistent with previous studies and further validated the detection accuracy of STARmap Pro. When analyzing cell distribution around plaques, it was found that DAM distributed in the nearest regions around Aβ plaques, and then DAA in the outer regions near DAM. This implies that the distribution of DAMs may be directly affected by Aβ plaques, and then DAAs may be affected by the DAM. Indeed, a previous study revealed that reactive astrocytes with high expression of DAA marker genes *Gfap* and *Vim* were induced by activated microglia. As for the cell type distribution pattern, it was also found that OPCs were enriched in the near plaque regions. These OPCs near plaque may be differentiated to oligodendrocytes involved in the pathology of AD.

Pathology-responsive transcriptional signatures in five major cell types were also provided. It was found that most gene markers were cell-type-specific and could be involved in specific perturbations, while some DEGs were shared across cell types. For example, Gfap was upregulated in all the major cell types. This was consistent with the previous single-nuclei study that also showed high Gfap expression in Alzheimer's disease-specific subclusters of various cell types. Microglia and astrocytes also shared DEGs such as *CTSB*. Pathway analysis also showed that the DEGs from different cell types were involved in similar biological processes.

*Methods*

Mice

All animal procedures followed animal care guidelines approved by the Genentech Institutional Animal Care and Use Committee (IACUC), and animal experiments were conducted in compliance with IACUC policies and NIH guidelines. The mice used for STARmap Pro include pR5-183 line expressing the P301L mutant of human tau and $PS2_{N141I}$ and $APP_{swe}$ ($PS2APP^{homo}$; $P301L^{hemi}$) and non-transgenic control.

Tissue collection and sample preparation for STARmap Pro

Animals were anesthetized with isoflurane and rapidly decapitated. Brain tissue was removed, placed in O.C.T, and then frozen in liquid nitrogen and kept at -80 °C. For the mouse brain tissue section, brains were transferred to the cryostat (Leica CM1950) and cut as 20 $\mu$m slices in coronal sections. The brain slices were fixed with 4% PFA in 1X PBS buffer at room temperature for 15 min, permeabilized with cold methanol, and placed at -80 °C for an hour.

STARmap Pro to detect spatial RNA and protein signals.

Samples were taken from -80 °C to room temperature for 5 min and then washed with PBSTR buffer (0.1% Tween-20, 0.1 U/$\mu$L SUPERase·In RNase Inhibitor in PBS). After washing, the samples were incubated with 300 $\mu$l 1X hybridization buffer (2X SSC, 10% formamide, 1% Tween-20, 0.1mg/ml yeast tRNA, 20 mM RVC, 0.1 U/$\mu$L SUPERase·In RNase Inhibitor, and pooled SNAIL probes at 1 nM per oligo) in a 40 °C humidified oven with shaking and parafilm wrapping for 36h. The samples were washed by PBSTR twice and high-salt washing buffer (4X SSC dissolved in PBSTR) once at 37 °C. Finally, the samples were rinsed with PBSTR once at room temperature. The samples were then incubated with ligation mixture (1:10 dilution of T4 DNA ligase in 1X T4 DNA ligase buffer supplemented with 0.5 mg/ml BSA and 0.2 U/$\mu$L of SUPERase-In RNase inhibitor) at room temperature for two hours with gentle shaking. After ligation, the samples were washed twice with PBASR buffer and then incubated with rolling circle amplification (RCA) mixture (1:10 dilution of Phi29 DNA polymerase in 1X Phi29 buffer supplemented with 250 $\mu$M dNTP, 20 $\mu$M 5-(3-aminoallyl)-dUTP, 0.5 mg/ml BSA, and 0.2 U/$\mu$L of SUPERase-In RNase inhibitor) at 30 °C for two hours with gentle shaking. The samples were washed twice with PBST (0.1% Tween-20 in PBS) and blocked with blocking solution (5 mg/ml BSA in PBST) at room temperature for 30 min. The samples were incubated with p-Tau primary antibody (1:100 dilution in blocking solution) for 2 hours at room temperature. The samples were washed with PBST three times for 5 min each. The samples were then treated with 20 mM Acrylic acid NHS ester in PBST for 1 hour and then rinsed once with PBST. The samples were incubated in monomer buffer (4% acrylamide, 0.2% bis-acrylamide in 2X SSC) for 15 min at room temperature. The buffer was then aspirated, and 35 polymerization mixture (0.2% ammonium persulfate, 0.2% tetramethylethylenediamine dissolved in monomer buffer) was added to the center of the sample and immediately covered by a Gel Slick coated coverslip. The polymerization reaction was performed for 1 hour at room temperature, then washed by PBST twice for 5 min each. The samples were treated with dephosphorylation mixture (1:100 dilution of Shrimp Alkaline Phosphatase in 1X CutSmart buffer supplemented with 0.5 mg/ml BSA) at 37 °C for 1 hour and then washed by PBST three times for 5 min each.

For *in situ* RNA sequencing, each cycle began with treating the sample with stripping buffer (60% formamide and 0.1% Triton-X-100 in H$_2$O) at room temperature for 10 min twice, followed by PBST washing three times, for 5 min each. The sample was incubated with sequencing mixture (1:25 dilution of T4 DNA ligase in 1XT4 DNA ligase buffer supplemented with 0.5 mg/ml BSA, 10 $\mu$M reading probe, and 5 $\mu$M fluorescent oligos) at room temperature for at least 3 hours. The samples were washed by washing and imaging buffer (10% formamide in 2X SSC) three times for 10 min each, then immersed in washing and imaging buffer for imaging. Images were acquired using Leica TCS SP8 confocal microscopy. Eight cycles of imaging were performed to detect the 2766 genes.

After *in situ* sequencing of the RNA signal, the samples were incubated in X-34 solution (10 $\mu$M X-34, 40% ethanol and 0.02 M NaOH in 1X PBS) at room temperature for 10 min. The samples were then washed with 1X PBS 3 times, incubated in 80% EtOH for 1 min, and then washed with PBS 3 times for 1 min each. Then the samples were incubated with secondary antibody (1:80 dilution in blocking solution) at room temperature for 12h. The samples were then washed three times with PBST for 5 min each. Propidium Iodide (PI) staining was performed following the manufacturer's instructions for the purpose of cell segmentation. Another round of imaging was performed to detect spatial protein signals.

Thin-section STARmap Pro Data Processing.

All image processing steps were implemented using MATLAB R2019b and related open-source packages in Python 3.6 and applied according to Wang *et al.,* 2018.

*Image Preprocessing:* Multi-dimensional histogram matching was performed on each tile with MATLAB function "imhistmatchn". The image of the first color channel in the first sequencing round was used as a reference to make the illuminance and contrast level uniform.

*Image Registration:* Image registration was applied according to Wang *et al.,* 2018. Global image registration was accomplished using a three-dimensional fast Fourier transform (FFT) to compute the cross-correlation between two image volumes at all translational offsets. The position of the maximal correlation coefficient was identified and used to translate image volumes to compensate for the offset.

*Spot Calling:* After registration, individual dots were identified separately in each color channel on the first round of sequencing. Dots of approximately 6 pixels in diameter were identified by finding local maxima in 3D. After identifying each dot, the dominant color for that dot across all four channels was determined on each round in a 5x5x3 voxel volume surrounding the dot location.

*Barcode Filtering:* Dots were first filtered based on quality score. The quality score quantified the extent to which each dot on each sequencing round came from one color rather than a mixture of colors. The barcode codebook was converted into color space, based on the expected color sequence following 2-base encoding of the barcode DNA sequence. Dot color sequences that passed the quality threshold and matched sequences in the codebook were kept and identified with the specific gene that the barcode represented; all other dots were rejected. The high-quality dots and associated gene identities in the codebook were then saved out for downstream analysis.

*2D Cell Segmentation:* Nuclei were automatically identified by the StarDist 2D machine learning model (Schmidt *et al.,* 2018) from a maximum intensity projection of the stitched DAPI channel following the final round of sequencing. Cell locations were then extracted from the segmented DAPI image. Cell bodies were represented by the overlay of stitched Nissl staining and merged amplicon images. Finally, a marker-based watershed transform was then applied to segment the thresholded cell bodies based on the combined thresholded cell body map and identified locations of nuclei. Points overlapping each segmented cell region in 2D were then assigned to that cell, to compute a per-cell gene expression matrix.

*Cell type classification:* A two-level clustering strategy was applied to identify both major and sub-level cell types in the dataset. Processing steps in this section were implemented using Scanpy v1.4.6 (Wolf *et al.,* 2018) and other customized scripts in Python 3.6 and applied according to Wang *et al.,* 2018. After filtration, normalization, and scaling, principal-components analysis (PCA) was applied to reduce the dimensionality of the cellular expression matrix. Based on the explained variance ratio, the top PCs were used to compute the neighborhood graph of observations. Then the Leiden algorithm was used to identify well-connected cells as clusters in a low dimensional representation of the transcriptomics profile. The cells were displayed using Uniform Manifold Approximation and Projection (UMAP) and color-coded according to their cell types. The cells for each top-level cluster were then subclustered using PCA decomposition followed by Leiden clustering to determine sub-level cell types.

Spatial Analysis

*Plaque Segmentation:* Spatial analysis begins with plaque segmentation. By using the 'bwlabel' function in EBImage package, plaques can be segmented from the binary image of plaque channel. Then, the size and center of each plaque is calculated by using 'computeFeatures.moment' and 'computeFeatures.shape' functions. Finally, plaques with areas less than 400 pixels (approximately equal to 36.7 $\mu m^2$) will be filtered out.

*Cell Distribution around plaque:* As cell position *PC* is obtained from data preprocessing step, consider a sample with \$n\$ cells and \$m\$ plaques, for each cell $i \in \{1, 2, ..., n\}$, its nearest plaque distance is:

$$ND_i = min\{\|PC_i - PP_j\|_2\}, j \in \{1, 2, ..., m\}$$

Next, we count the number of cells for every cell type that fall into different ranges. The ranges are set from 0-10 $\mu$m (Ring 1) to 40-50 $\mu$m (Ring 5). To remove the difference of total number of cells, the statistics are normalized by calculating the percentage of each cell type in a ring. The graphical explanation of this analysis is shown in FIG. 2D. For the overall statistics, the percentage of each cell type was calculated in the whole sample.

*Type-to-type Distance Calculation:* In a sample that has *n* cells and *m* plaques, they are treated as objects with coordinates *P* and type label (Plaque, DAA, DAM, etc.). Suppose there are *t* types of label and the set of objects for each type are $S_1$, $S_2$, ..., $S_t$, then the type-to-type distance from type *i* to type *j* is:

$$D_{ij} = \frac{\sum_{k \in S_i} min\{\|P_k - P_l\|_2\}}{\#S_i}, l \in S_j$$

Shuffled control analysis is performed using the same algorithm but randomly assigned a label (the number of cells/plaques remained the same).

Differential Expression and Pathway Analysis

*Differential Expression Analysis:* Before performing DE analysis, the dataset is normalized by: 1) Dividing the gene counts in a sample by the median of *total counts per cell* for that sample and multiplying by the scale factor, which is defined as the mean value of median of *total counts per cell* for all samples; and 2) Performing log2 transformation by adding a pseudo-count of one.

$$Scale\ Factor = \frac{\sum median(\text{total counts per cell})}{Number\ of\ samples}$$

DE genes are identified by performing Wilcoxon Rank Sum test between two groups of cells using the 'FindMarkers' function in Seurat. For a comparison like 'Disease vs. Control', the two groups of cells naturally extract a certain type of cells from TauPS2APP and control samples. As for the comparison of 'Near Plaque vs. Far away from Plaque', the 'near plaque' cells are those cells with a nearest plaque distance < 25μm and all other cells are defined as 'away from plaque'. In the comparison of 'CA1 Tau+ vs Tau-', Tau+ CA1 cells are filtered according to the fraction of Tau signal area to the cell body's area. The threshold of that fraction is set to 0.3.

In order to filter out some lowly expressed genes, the minimum threshold for the fraction of cells that genes are detected expressing in either cell group is set to 0.1. The following threshold values were also applied on the generated gene list to filter out non-significant genes: absolute value of log fold change > 0.1, p-value < 0.05.

To visualize the DE result, we used the 'EnhancedVolcanoplot' package to generate the volcano plot. DE genes with logFC > 0 are colored in red while others are colored in blue. Significant genes (p-value < 0.05) that failed to pass the LogFC threshold are green tinted. All other non-significant genes are colored in gray. Note, some genes with extremely high -log(P-value) or logFC are capped.

Gene Ontology (GO) Enrichment Analysis:

Website g:Profiler was used to perform GO enrichment analysis for DE genes of each comparison: The list of DE genes between cells from disease and control samples or Tau positive and negative or near plaque and away from plaques (25μm is used as the threshold) is the input of GO analysis. The statistical domain scope is limited to annotated genes. The significance thresholds were determined using g:SCS and the user threshold was set to 0.05. To limit the size of functional categories subjected to enrichment analysis, GO terms with < 20 or > 1000 genes were filtered out. Results were downloaded in generic enrichment map (GEM) format to be used as input for further functional enrichment analysis.

Cytoscape(v3.8.2) with EnrichmentMap(v3.3.1) and AutoAnnotate(v1.3.3) apps were used to integrate and visualize GO enrichment results from five main cell types' DEGs (Ex/In/Astro/Micro/Oligo). The lists of statistically significant GO & KEGG terms obtained with g:Profiler were imported into Cytoscape with the following parameters: nodes(GO/KEGG terms) cut-off was set to adjusted p-values < 0.05 and FDR q-values < 0.1; edges (representing similarity between the gene lists of each node) used a similarity threshold of 0.375. Each node is color coded by cell types to account for common and different contributions of DEGs from each cell cluster. The enrichment map was annotated automatically using AutoAnnotate and the three-words label of each cluster is created using the WordCould app.

The SynGO enrichment tool was used to further characterize the synapse functions enriched in DEGs from excitatory neurons, inhibitory neurons, and CA1 cells with Tau pathology. Brain expressed genes are used as the background gene list.

**REFERENCES**

1. Braak, H., and Braak, E. (1991). Neuropathological stageing of Alzheimer-related changes. Acta Neuropathologica. 82, 239-259.

2. Busche, M.A., and Hyman, B.T. (2020). Synergy between amyloid-β and tau in Alzheimer's disease. Nature Neuroscience. 23, 1183-1193.

3. Cao, J., Spielmann, M., Qiu, X., Huang, X., Ibrahim, D.M., Hill, A.J., Zhang, F., Mundlos, S., Christiansen, L., Steemers, F.J., et al. (2019). The single-cell transcriptional landscape of mammalian organogenesis. Nature. 566, 496-502.

4. Chen, W.-T., Lu, A., Craessaerts, K., Pavie, B., Sala Frigerio, C., Corthout, N., Qian, X., Laláková, J., Kühnemund, M., Voytyuk, I., et al. (2020). Spatial Transcriptomics and In Situ Sequencing to Study Alzheimer's Disease. Cell. 182, 976-991.e19.

5. Grubman, A., Chew, G., Ouyang, J.F., Sun, G., Choo, X.Y., McLean, C., Simmons, R.K., Buckberry, S., Vargas-Landin, D.B., Poppe, D., et al. (2019). A single-cell atlas of entorhinal cortex from individuals with Alzheimer's disease reveals cell-type-specific gene expression regulation. Nat. Neurosci. 22, 2087-2097.

6. Grueninger, F., Bohrmann, B., Czech, C., Ballard, T.M., Frey, J.R., Weidensteiner, C., von Kienlin, M., and Ozmen, L. (2010). Phosphorylation of Tau at S422 is enhanced by Aβ in TauPS2APP triple transgenic mice. Neurobiol. Dis. 37, 294-306.

7. Habib, N., McCabe, C., Medina, S., Varshavsky, M., Kitsberg, D., Dvir-Szternfeld, R., Green, G., Dionne, D.,

Nguyen, L., Marshall, J.L., et al. (2020). Disease-associated astrocytes in Alzheimer's disease and aging. Nat. Neurosci. 23, 701-706.

8. Hardy, J., and Selkoe, D.J. (2002). The amyloid hypothesis of Alzheimer's disease: progress and problems on the road to therapeutics. Science. 297, 353-356.

9. Keren-Shaul, H., Spinrad, A., Weiner, A., Matcovitch-Natan, O., Dvir-Szternfeld, R., Ulland, T.K., David, E., Baruch, K., Lara-Astaiso, D., Toth, B., et al. (2017). A Unique Microglia Type Associated with Restricting Development of Alzheimer's Disease. Cell. 169, 1276-1290.e17.

10. Lau, S.-F., Cao, H., Fu, A.K.Y., and Ip, N.Y. (2020). Single-nucleus transcriptome analysis reveals dysregulation of angiogenic endothelial cells and neuroprotective glia in Alzheimer's disease. Proceedings of the National Academy of Sciences. 117, 25800-25809.

11. Lee, S.-H., Meilandt, W.J., Xie, L., Gandham, V.D., Ngu, H., Barck, K.H., Rezzonico, M.G., Imperio, J., Lalehzadeh, G., Huntley, M.A., et al. (2021). Trem2 restrains the enhancement of tau accumulation and neurodegeneration by β-amyloid pathology. Neuron. 109, 1283-1301.e6.

12. Masters, C.L., Bateman, R., Blennow, K., Rowe, C.C., Sperling, R.A., and Cummings, J.L. (2015). Alzheimer's disease. Nat Rev Dis Primers. 1, 15056.

13. Mathys, H., Davila-Velderrain, J., Peng, Z., Gao, F., Mohammadi, S., Young, J.Z., Menon, M., He, L., Abdurrob, F., Jiang, X., et al. (2019). Single-cell transcriptomic analysis of Alzheimer's disease. Nature. 570, 332-337.

14. McInnes, L., Healy, J., Saul, N., and Großberger, L. (2018). UMAP: Uniform Manifold Approximation and Projection. Journal of Open Source Software. 3, 861.

15. Ståhl, P.L., Salmén, F., Vickovic, S., Lundmark, A., Navarro, J.F., Magnusson, J., Giacomello, S., Asp, M., Westholm, J.O., Huss, M., et al. (2016). Visualization and analysis of gene expression in tissue sections by spatial transcriptomics. Science. 353, 78-82.

16. Stuart, T., and Satija, R. (2019). Integrative single-cell analysis. Nat. Rev. Genet. 20, 257-272.

17. Traag, V.A., Waltman, L., and van Eck, N.J. (2019). From Louvain to Leiden: guaranteeing well-connected communities. Sci. Rep. 9, 5233.

18. Wang, X., Allen, W.E., Wright, M.A., Sylwestrak, E.L., Samusik, N., Vesuna, S., Evans, K., Liu, C., Ramakrishnan, C., Liu, J., et al. (2018). Three-dimensional intact-tissue sequencing of single-cell transcriptional states. Science. 361.

19. Zhou, Y., Song, W.M., Andhey, P.S., Swain, A., Levy, T., Miller, K.R., Poliani, P.L., Cominelli, M., Grover, S., Gilfillan, S., et al. (2020). Human and mouse single-nucleus transcriptomics reveal TREM2-dependent and TREM2-independent cellular responses in Alzheimer's disease. Nat. Med. 26, 131-142.

## INCORPORATION BY REFERENCE

The present application refers to various issued patent, published patent applications, scientific journal articles, and other publications, all of which are incorporated herein by reference. The details of one or more embodiments of the invention are set forth herein. Other features, objects, and advantages of the invention will be apparent from the Detailed Description, the Figures, the Examples, and the Claims.

## EQUIVALENTS AND SCOPE

In the articles such as "a," "an," and "the" may mean one or more than one unless indicated to the contrary or otherwise evident from the context. Embodiments or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention includes embodiments in which more than one, or all of the group members are present in, employed in, or otherwise relevant to a given product or process.

Furthermore, the disclosure encompasses all variations, combinations, and permutations in which one or more limitations, elements, clauses, and descriptive terms from one or more of the listed claims is introduced into another claim. For example, any claim that is dependent on another claim can be modified to include one or more limitations found in any other claims that is dependent on the same base claim. Where elements are presented as lists, *e.g.,* in Markush group format, each subgroup of the elements is also disclosed, and any element(s) can be removed from the group. It should it be understood that, in general, where the invention, or aspects of the invention, is/are referred to as comprising particular elements and/or features, certain embodiments of the disclosure or aspects of the disclosure consist, or consist essentially of, such elements and/or features. For purposes of simplicity, those embodiments have not been specifically set forth *in haec verba* herein. It is also noted that the terms "comprising" and "containing" are intended to be open and permits the inclusion of additional elements or steps. Where ranges are given, endpoints are included. Furthermore, unless otherwise indicated or otherwise evident from the context and understanding of one of ordinary skill in the art, values that are expressed as ranges can assume any specific value or subrange within the stated ranges in different embodiments of the invention, to the tenth of the unit of the lower limit of the range, unless the context clearly dictates otherwise.

This application refers to various issued patents, published patent applications, journal articles, and other publications, all of which are incorporated herein by reference. If there is a conflict between any of the incorporated references and the instant specification, the specification shall control. In addition, any particular embodiment of the present invention that falls within the prior art may be explicitly excluded from any one or more of the embodiments. Because such embodiments are deemed to be known to one of ordinary skill in the art, they may be excluded even if the exclusion is not set forth explicitly herein. Any particular embodiment of the invention can be excluded from any embodiment, for any reason, whether or not related to the existence of prior art.

Those skilled in the art will recognize or be able to ascertain using no more than routine experimentation many equivalents to the specific embodiments described herein. The scope of the present embodiments described herein is not intended to be limited to the above Description, but rather is as set forth in the appended embodiments. Those of ordinary skill in the art will appreciate that various changes and modifications to this description may be made without departing from the spirit or scope of the present invention, as defined in the following claims.

**CLAUSES**

1. A method for mapping gene and protein expression in a cell, the method comprising:

> a) contacting the cell with one or more pairs of oligonucleotide probes, wherein each pair of oligonucleotide probes comprises a first oligonucleotide probe and a second oligonucleotide probe, wherein

>> i) the first oligonucleotide probe comprises a portion that is complementary to the second oligonucleotide probe, a portion that is complementary to a nucleic acid of interest, a first barcode sequence, and a second barcode sequence; and
>> ii) the second oligonucleotide probe comprises a portion that is complementary to the nucleic acid of interest, a portion that is complementary to the first oligonucleotide probe, and a barcode sequence, wherein the barcode sequence of the second oligonucleotide probe is complementary to the second barcode sequence of the first oligonucleotide probe;

> b) ligating the 5' end and the 3' end of the first oligonucleotide probe together to produce a circular oligonucleotide;
> c) performing rolling circle amplification to amplify the circular oligonucleotide using the second oligonucleotide probe as a primer to produce one or more concatenated amplicons;
> d) contacting the cell with one or more detecting agents, wherein each detecting agent binds to a protein of interest;
> e) embedding the one or more concatenated amplicons and the one or more detecting agents in a polymeric matrix;
> f) contacting the one or more concatenated amplicons embedded in the polymeric matrix with a third oligonucleotide probe comprising a sequence that is complementary to the first barcode sequence of the first oligonucleotide probe; and
> g) imaging the one or more concatenated amplicons embedded in the polymeric matrix and the one or more detecting agents embedded in the polymeric matrix to determine the location of the nucleic acids of interest and the proteins of interest within the cell and, optionally, map gene and protein expression.

2. The method of clause 1, wherein gene and protein expression are profiled in multiple cells.

3. The method of clause 2, wherein the cells comprise a plurality of cell types.

4. The method of clause 3, wherein the cell types are selected from the group consisting of stem cells, progenitor cells, neuronal cells, astrocytes, dendritic cells, endothelial cells, microglia, oligodendrocytes, muscle cells, myocardial cells, mesenchymal cells, epithelial cells, immune cells, and hepatic cells.

5. The method of any one of clauses 1-4, wherein the cell is a permeabilized cell.

6. The method of any one of clauses 1-4, wherein cell is present within an intact tissue.

7. The method of clause 6, wherein the intact tissue is a fixed tissue sample.

8. The method of any one of clauses 1-7, wherein the nucleic acid of interest is DNA.

9. The method of any one of clauses 1-8, wherein the nucleic acid of interest is RNA.

10. The method of clause 9, wherein the RNA is mRNA.

11. The method of any one of clauses 1-10, wherein gene expression for more than 100, more than 200, more than 500, more than 1000, more than 2000, or more than 3000 nucleic acids of interest is mapped.

12. The method of any one of clauses 1-11, wherein gene expression for up to one million nucleic acids of interest is mapped.

13. The method of any one of clauses 1-12, wherein the barcode sequences on the first and second oligonucleotide probes are 5-15, 6-14, 7-13, 8-12, or 9-11 nucleotides in length.

14. The method of any one of clauses 1-13, wherein barcode sequences on the first and second oligonucleotide probes are 5, 6, 7, 8, 9, 10, 11, 12, 13, 14, or 15 nucleotides in length.

15. The method of any one of clauses 1-14, wherein the barcode sequences on the first and second oligonucleotide probes are 10 nucleotides in length.

16. The method of any one of clauses 1-15, wherein the first and second oligonucleotide probes bind different portions of the nucleic acid of interest.

17. The method of any one of clauses 1-16, wherein the first oligonucleotide probe comprises the structure: 5'-[portion complementary to second probe]-[portion complementary to nucleic acid of interest]-[first barcode sequence]-[second barcode sequence]-3'.

18. The method of any one of clauses 1-17, wherein the second oligonucleotide probe comprises the structure: 5'-[portion complementary to nucleic acid of interest]-[portion complementary to first probe]-[barcode sequence]-3'.

19. The method of any one of clauses 1-18, wherein the second barcode sequence of the first oligonucleotide probe increases the specificity of the detection of the nucleic acid of interest.

20. The method of any one of clauses 1-19, wherein the second barcode sequence of the first oligonucleotide probe reduces non-specific amplification.

21. The method of any one of clauses 1-20, wherein the third oligonucleotide probe comprises a detectable label.

22. The method of clause 21, wherein the detectable label is a fluorophore.

23. The method of any one of clauses 1-22, wherein the one or more detecting agents are antibodies that each comprise a detectable label.

24. The method of any one of clauses 1-22 further comprising contacting the one or more detecting agents with a secondary detecting agent.

25. The method of any one of clauses 1-24, wherein the one or more detecting agents comprise a small molecule.

26. The method of clause 25, wherein the small molecule is X-34.

27. The method of clause 24, wherein the secondary detecting agent is a secondary antibody.

28. The method of clause 27, wherein the secondary antibody comprises a detectable label.

29. The method of clause 28, wherein the detectable label is a fluorophore.

**EP 4 644 569 A2**

30. The method of any one of clauses 1-22, wherein the one or more detecting agents are antibodies that are each conjugated to an oligonucleotide sequence, and that each bind to a protein of interest.

31. The method of clause 30 further comprising contacting each of the one or more antibodies that bind to a protein of interest with an oligonucleotide conjugated to a detectable label, wherein the oligonucleotide conjugated to a detectable label is complementary to the oligonucleotide sequence conjugated to the one or more antibodies.

32. The method of clause 31, wherein the detectable label is a fluorophore.

33. The method of any one of clauses 1-32, wherein the step of imaging comprises fluorescence imaging.

34. The method of any one of clauses 1-33, wherein the step of imaging comprises confocal microscopy or epifluorescence microscopy.

35. The method of any one of clauses 24-34, wherein the step of contacting each of the one or more detecting agents embedded in the polymeric matrix with a secondary detecting agent is performed after the step of performing rolling circle amplification to amplify the circular oligonucleotide.

36. The method of any one of clauses 1-35, wherein the step of contacting the cell with one or more detecting agents is performed before the step of embedding.

37. The method of any one of clauses 1-36, wherein the locations of the nucleic acids of interest and the proteins of interest are determined in the same round of imaging.

38. The method of any one of clauses 1-36, wherein the locations of the nucleic acids of interest and the proteins of interest are determined in separate rounds of imaging.

39. The method of any one of clauses 1-38, wherein the polymeric matrix is a hydrogel.

40. The method of any one of clauses 1-39, wherein the hydrogel is a polyvinyl alcohol hydrogel, a polyethylene glycol hydrogel, a sodium polyacrylate hydrogel, an acrylate polymer hydrogel, or a polyacrylamide hydrogel.

41. The method of any one of clauses 1-40, wherein the step of performing rolling circle amplification further comprises providing amine-modified nucleotides, wherein the amine-modified-nucleotides are incorporated into the one or more concatenated amplicons.

42. The method of any one of clauses 1-41, wherein the step of embedding the one or more concatenated amplicons in the polymer matrix comprises reacting the amine-modified nucleotides of the one or more amplicons with acrylic acid N-hydroxysuccinimide ester and co-polymerizing the one or more concatenated amplicons and the polymer matrix.

43. The method of any one of clauses 1-42, wherein the first barcode sequence of the first oligonucleotide probe is a gene-specific sequence used to identify the nucleic acid of interest.

44. The method of any one of clauses 1-43, wherein the method is performed at subcellular resolution.

45. The method of any one of clauses 1-44, wherein the method is performed at a subcellular resolution of 200 nm, 150 nm, 100 nm, 50 nm, 40 nm, 30 nm, 20 nm, or 10 nm.

46. The method of any one of clauses 1-45, wherein the method is performed at a subcellular resolution of 200 nm.

47. A method for mapping gene expression in a cell, the method comprising:

a) contacting the cell with one or more pairs of oligonucleotide probes, wherein each pair of oligonucleotide probes comprises a first oligonucleotide probe and a second oligonucleotide probe, wherein

i) the first oligonucleotide probe comprises a portion that is complementary to the second oligonucleotide probe, a portion that is complementary to a nucleic acid of interest, a first barcode sequence, and a second barcode sequence; and

ii) the second oligonucleotide probe comprises a portion that is complementary to the nucleic acid of interest, a portion that is complementary to the first oligonucleotide probe, and a barcode sequence, wherein the barcode sequence of the second oligonucleotide probe is complementary to the second barcode sequence of the first oligonucleotide probe;

b) ligating the 5' end and the 3' end of the first oligonucleotide probe together to produce a circular oligonucleotide;
c) performing rolling circle amplification to amplify the circular oligonucleotide using the second oligonucleotide probe as a primer to produce one or more concatenated amplicons;
d) embedding the one or more concatenated amplicons in a polymeric matrix;
e) contacting the one or more concatenated amplicons embedded in the polymeric matrix with a third oligonucleotide probe comprising a sequence that is complementary to the first barcode sequence of the first oligonucleotide probe; and
f) imaging the one or more concatenated amplicons embedded in the polymeric matrix to determine the location of the nucleic acids of interest within the cell and, optionally, map gene and protein expression.

48. A method for mapping gene and protein expression in a cell, the method comprising:

a) contacting the cell with one or more pairs of oligonucleotide probes, wherein each pair of oligonucleotide probes comprises a first oligonucleotide probe and a second oligonucleotide probe, wherein

i) the first oligonucleotide probe comprises a portion that is complementary to the second oligonucleotide probe, a portion that is complementary to a nucleic acid of interest, and a barcode sequence; and
ii) the second oligonucleotide probe comprises a portion that is complementary to the nucleic acid of interest and a portion that is complementary to the first oligonucleotide probe;

b) ligating the 5' end and the 3' end of the first oligonucleotide probe together to produce a circular oligonucleotide;
c) performing rolling circle amplification to amplify the circular oligonucleotide using the second oligonucleotide probe as a primer to produce one or more concatenated amplicons;
d) contacting the cell with one or more detecting agents, wherein each detecting agent binds to a protein of interest;
e) embedding the one or more concatenated amplicons and the one or more detecting agents in a polymeric matrix;
f) contacting the one or more concatenated amplicons embedded in the polymeric matrix with a third oligonucleotide probe comprising a sequence that is complementary to the first barcode sequence of the first oligonucleotide probe; and
g) imaging the one or more concatenated amplicons embedded in the polymeric matrix and the one or more detecting agents embedded in the polymeric matrix to determine the location of the nucleic acids of interest and the proteins of interest within the cell and, optionally, map gene and protein expression.

49. A method for diagnosing a disease or disorder in a subject, the method comprising:

a) contacting a cell taken from the subject with one or more pairs of oligonucleotide probes, wherein each pair of oligonucleotide probes comprises a first oligonucleotide probe and a second oligonucleotide probe, wherein

i) the first oligonucleotide probe comprises a portion that is complementary to the second oligonucleotide probe, a portion that is complementary to a nucleic acid of interest, a first barcode sequence, and a second barcode sequence; and
ii) the second oligonucleotide probe comprises a portion that is complementary to the nucleic acid of interest, a portion that is complementary to the first oligonucleotide probe, and a barcode sequence, wherein the barcode sequence of the second oligonucleotide probe is complementary to the second barcode sequence of the first oligonucleotide probe;

b) ligating the 5' end and the 3' end of the first oligonucleotide probe together to produce a circular oligonucleotide;
c) performing rolling circle amplification to amplify the circular oligonucleotide using the second oligonucleotide probe as a primer to produce one or more concatenated amplicons;
d) contacting the cell with one or more detecting agents, wherein each detecting agent binds to a protein of interest;
e) embedding the one or more concatenated amplicons and the one or more detecting agents in a polymeric matrix;

f) contacting the one or more concatenated amplicons embedded in the polymeric matrix with a third oligonucleotide probe comprising a sequence that is complementary to the first barcode sequence of the first oligonucleotide probe; and

g) imaging the one or more concatenated amplicons embedded in the polymeric matrix and the one or more detecting agents embedded in the polymeric matrix to determine the location of the nucleic acids of interest and the proteins of interest within the cell and, optionally, map gene and protein expression;

wherein an alteration in the expression of the nucleic acids of interest and/or the proteins of interest relative to expression in one or more non-diseased cells indicates that the subject has the disease or disorder.

50. The method of clause 49, wherein the gene and protein expression in one or more non-diseased cells is profiled simultaneously as a control experiment.

51. The method of clause 50, wherein the gene and protein expression in one or more non-diseased cells comprises reference data.

52. The method of any one of clauses 49-51, wherein the disease or disorder is a genetic disease, a proliferative disease, an inflammatory disease, an autoimmune disease, a liver disease, a spleen disease, a lung disease, a hematological disease, a neurological disease, a gastrointestinal (GI) tract disease, a genitourinary disease, an infectious disease, a musculoskeletal disease, an endocrine disease, a metabolic disorder, an immune disorder, a central nervous system (CNS) disorder, or a cardiovascular disease.

53. The method of any one of clauses 49-52, wherein the disease or disorder is Alzheimer's disease.

54. The method of any one of clauses 49-53, wherein the cell is present in a tissue.

55. The method of clause 54, wherein the tissue is a tissue sample from a subject.

56. The method of clause 55, wherein the subject is a non-human experimental animal.

57. The method of clause 55, wherein the subject is a human.

58. The method of any one of clauses 54-57, wherein the tissue is a fixed tissue sample.

59. The method of any one of clauses 54-58, wherein the tissue is brain tissue.

60. The method of any one of clauses 54-59, wherein gene and protein expression is profiled in multiple cells.

61. The method of clause 60, wherein the cells comprise a plurality of cell types.

62. The method of clause 61, wherein the cell types are selected from the group consisting of stem cells, progenitor cells, neuronal cells, astrocytes, dendritic cells, endothelial cells, microglia, oligodendrocytes, muscle cells, myocardial cells, mesenchymal cells, epithelial cells, immune cells, and hepatic cells.

63. The method of any one of clauses 49-62, wherein the proteins of interest comprise amyloid beta (Aβ) peptides.

64. The method of clause 63, wherein the Aβ peptides are present in the cell in the form of Aβ plaques.

65. The method of any one of clauses 49-64, wherein the proteins of interest comprise tau protein.

66. The method of clause 65, wherein the tau protein is present in the cell in the form of inclusion bodies (p-Tau).

67. The method of any one of clauses 49-66, wherein the nucleic acids of interest are selected from the group consisting of *Vsnl1, Snap25, Dnm1, Slc6a1, Aldoc, Bsg, Ctss, Plp1, Cst7, Ctsb, Apoe, Trem2, C1qa, P2ry12, Gfap, Vim, Aqp4, Clu, Plp1, Mbp, C4b, Ccnb2, Gpm6a, Ddit3, Dapk1, Myo5a, Tspan7,* and *Rhoc.*

68. The method of any one of clauses 63-67, wherein the Aβ peptides are detected using a small molecule.

69. The method of clause 68, wherein the small molecule is X-34.

70. The method of any one of clauses 65-69, wherein the tau protein is detected using a p-Tau primary antibody.

71. The method of clause 70 further comprising detecting the p-Tau primary antibody with a secondary antibody.

72. The method of clause 71, wherein the secondary antibody is conjugated to a detectable label.

73. The method of clause 72, wherein the detectable label is fluorophore.

74. The method of any one of clauses 46-73, wherein an alteration in the expression of the nucleic acids of interest is used to identify cell types in close proximity to plaques, and wherein the subject has or is suspected of having Alzheimer's disease if certain cell types are identified in close proximity to plaques.

75. The method of clause 74, wherein the plaques are Aβ plaques.

76. The method of clause 74 or 75, wherein the identification of disease-associated microglia cell types in close proximity to plaques indicates that the subject has or is at risk of having Alzheimer's disease.

77. The method of any one of clauses 74-76, wherein the identification of disease-associated astrocyte cell types in close proximity to plaques indicates that the subject has or is at risk of having Alzheimer's disease.

78. The method of any one of clauses 74-77, wherein the identification of oligodendrocyte precursor cell types in close proximity to plaques indicates that the subject has or is at risk of having Alzheimer's disease.

79. The method of clause 49, wherein the disease or disorder is cancer.

80. The method of any one of clauses 49-79, wherein the nucleic acid of interest is DNA.

81. The method of any one of clauses 49-79, wherein the nucleic acid of interest is RNA.

82. The method of clause 81, wherein the RNA is mRNA.

83. The method of any one of clauses 49-82, wherein gene expression for more than 100, more than 200, more than 500, more than 1000, more than 2000, or more than 3000 nucleic acids of interest is mapped.

84. The method of any one of clauses 49-83, wherein gene expression for up to one million nucleic acids of interest is mapped.

85. The method of any one of clauses 49-84, wherein the barcode sequences on the oligonucleotide probes are 5-15, 6-14, 7-13, 8-12, or 9-11 nucleotides in length.

86. The method of any one of clauses 49-85, wherein barcode sequences on the oligonucleotide probes are 5, 6, 7, 8, 9, 10, 11, 12, 13, 14, or 15 nucleotides in length.

87. The method of any one of clauses 49-86, wherein the barcode sequences on the oligonucleotide probes are 10 nucleotides in length.

88. The method of any one of clauses 49-87, wherein first and second oligonucleotide probes bind different sections of the nucleic acid of interest.

89. The method of any one of clauses 49-88, wherein the first oligonucleotide probe comprises the structure: 5'-[portion complementary to second probe]-[portion complementary to nucleic acid of interest]-[first barcode sequence]-[second barcode sequence]-3'.

90. The method of any one of clauses 49-89, wherein the second oligonucleotide probe comprises the structure: 5'-[portion complementary to nucleic acid of interest]-[portion complementary to first probe]-[barcode sequence]-3'.

91. The method of any one of clauses 49-90, wherein the second barcode sequence increases the specificity of the detection of the nucleic acid of interest.

92. The method of any one of clauses 49-91, wherein the second barcode sequence reduces non-specific amplification.

93. The method of any one of clauses 49-92, wherein the third oligonucleotide probe comprises a detectable label.

94. The method of clause 93, wherein the detectable label is a fluorophore.

95. The method of any one of clauses 49-94, wherein the step of imaging comprises fluorescence imaging.

96. The method of any one of clauses 49-95, wherein the step of imaging comprises confocal microscopy or epifluorescence microscopy.

97. A method for screening for an agent capable of modulating gene and/or protein expression, the method comprising:

a) contacting a cell that is being treated or has been treated with the candidate agent with one or more pairs of oligonucleotide probes, wherein each pair of oligonucleotide probes comprises a first oligonucleotide probe and a second oligonucleotide probe, wherein

i) the first oligonucleotide probe comprises a portion that is complementary to the second oligonucleotide probe, a portion that is complementary to a nucleic acid of interest, a first barcode sequence, and a second barcode sequence; and
ii) the second oligonucleotide probe comprises a portion that is complementary to the nucleic acid of interest, a portion that is complementary to the first oligonucleotide probe, and a barcode sequence, wherein the barcode sequence of the second oligonucleotide probe is complementary to the second barcode sequence of the first oligonucleotide probe;

b) ligating the 5' end and the 3' end of the first oligonucleotide probe together to produce a circular oligonucleotide;
c) performing rolling circle amplification to amplify the circular oligonucleotide using the second oligonucleotide probe as a primer to produce one or more concatenated amplicons;
d) contacting the cell with one or more detecting agents, wherein each detecting agent binds to a protein of interest;
e) embedding the one or more concatenated amplicons and the one or more detecting agents in a polymeric matrix;
f) contacting the one or more concatenated amplicons embedded in the polymeric matrix with a third oligonucleotide probe comprising a sequence that is complementary to the first barcode sequence of the first oligonucleotide probe; and
g) imaging the one or more concatenated amplicons embedded in the polymeric matrix and the one or more detecting agents embedded in the polymeric matrix to determine the location of the nucleic acids of interest and the proteins of interest in the cell and, optionally, map gene and protein expression;

wherein an alteration in the expression of the nucleic acids of interest and/or the proteins of interest in the presence of the candidate agent relative to expression in the absence of the candidate agent indicates that the candidate agent modulates gene and/or protein expression.

98. The method of clause 97, wherein the candidate agent is a small molecule, a protein, a peptide, a nucleic acid, a lipid, or a carbohydrate.

99. The method of clause 98, wherein the small molecule is an anti-cancer therapeutic agent.

100. The method of clause 98, wherein the protein is an antibody.

101. The method of clause 98, wherein the nucleic acid is an mRNA, an antisense RNA, an miRNA, an siRNA, an RNA aptamer, a double stranded RNA (dsRNA), a short hairpin RNA (shRNA), or an antisense oligonucleotide (ASO).

102. The method of any one of clauses 97-101, wherein modulation of gene and/or protein expression by the candidate agent is associated with reducing, relieving, or eliminating the symptoms of a disease or disorder.

103. The method of clause 102, wherein the disease or disorder is a genetic disease, a proliferative disease, an inflammatory disease, an autoimmune disease, a liver disease, a spleen disease, a lung disease, a hematological disease, a neurological disease, a gastrointestinal (GI) tract disease, a genitourinary disease, an infectious disease, a musculoskeletal disease, an endocrine disease, a metabolic disorder, an immune disorder, a central nervous system (CNS) disorder, or a cardiovascular disease.

104. The method of clause 102 or 103, wherein the disease or disorder is Alzheimer's disease.

105. The method of clause 102 or 103, wherein the disease or disorder is cancer.

106. A method for treating a disease or disorder in a subject, the method comprising:

a) contacting a cell taken from the subject with one or more pairs of oligonucleotide probes, wherein each pair of oligonucleotide probes comprises a first oligonucleotide probe and a second oligonucleotide probe, wherein

i) the first oligonucleotide probe comprises a portion that is complementary to the second oligonucleotide probe, a portion that is complementary to a nucleic acid of interest, a first barcode sequence, and a second barcode sequence; and
ii) the second oligonucleotide probe comprises a portion that is complementary to the nucleic acid of interest, a portion that is complementary to the first oligonucleotide probe, and a barcode sequence, wherein the barcode sequence of the second oligonucleotide probe is complementary to the second barcode sequence of the first oligonucleotide probe;

b) ligating the 5' end and the 3' end of the first oligonucleotide probe together to produce a circular oligonucleotide;
c) performing rolling circle amplification to amplify the circular oligonucleotide using the second oligonucleotide probe as a primer to produce one or more concatenated amplicons;
d) contacting the cell with one or more detecting agents, wherein each detecting agent binds to a protein of interest;
e) embedding the one or more concatenated amplicons and the one or more detecting agents in a polymeric matrix;
f) contacting the one or more concatenated amplicons embedded in the polymeric matrix with a third oligonucleotide probe comprising a sequence that is complementary to the first barcode sequence of the first oligonucleotide probe;
g) imaging the one or more concatenated amplicons embedded in the polymeric matrix and the one or more detecting agents embedded in the polymeric matrix to determine the location of the nucleic acids of interest and the proteins of interest in the cell; and
h) administering a treatment for the disease or disorder to the subject if an alteration in the expression of the nucleic acids of interest and/or the proteins of interest relative to expression in one or more non-diseased cells is observed.

107. The method of clause 106, wherein gene and protein expression in one or more non-diseased cells is profiled simultaneously as a control experiment.

108. The method of clause 106, wherein gene and protein expression in one or more non-diseased cells comprises reference data.

109. The method of any one of clauses 106-108, wherein the treatment comprises administering a therapeutic agent, surgery, or radiation therapy.

110. The method of clause 109, wherein the therapeutic agent is a small molecule, a protein, a peptide, a nucleic acid, a lipid, or a carbohydrate.

111. The method of clause 110, wherein the small molecule is an anti-cancer therapeutic agent.

112. The method of clause 110, wherein the protein is an antibody.

113. The method of clause 110, wherein the nucleic acid is an mRNA, an antisense RNA, an miRNA, an siRNA, an RNA aptamer, a double stranded RNA (dsRNA), a short hairpin RNA (shRNA), or an antisense oligonucleotide (ASO).

114. The method of any one of clauses 106-113, wherein the disease or disorder is a genetic disease, a proliferative disease, an inflammatory disease, an autoimmune disease, a liver disease, a spleen disease, a lung disease, a hematological disease, a neurological disease, a gastrointestinal (GI) tract disease, a genitourinary disease, an infectious disease, a musculoskeletal disease, an endocrine disease, a metabolic disorder, an immune disorder, a central nervous system (CNS) disorder, or a cardiovascular disease.

115. The method of any one of clauses 106-114, wherein the disease or disorder is Alzheimer's disease.

116. The method of any one of clauses 106-114, wherein the disease or disorder is cancer.

117. A plurality of oligonucleotide probes comprising a first oligonucleotide probe and a second oligonucleotide probe, wherein

i) the first oligonucleotide probe comprises a portion that is complementary to the second oligonucleotide probe, a portion that is complementary to a nucleic acid of interest, a first barcode sequence, and a second barcode sequence; and
ii) the second oligonucleotide probe comprises a portion that is complementary to the nucleic acid of interest, a portion that is complementary to the first oligonucleotide probe, and a barcode sequence,

wherein the barcode sequence of the second oligonucleotide probe is complementary to the second barcode sequence of the first oligonucleotide probe.

118. The plurality of oligonucleotide probes of clause 117, wherein the barcode sequences on the first and second oligonucleotide probes are 5-15, 6-14, 7-13, 8-12, or 9-11 nucleotides in length.

119. The plurality of oligonucleotide probes of clause 117 or 118, wherein the barcode sequences on the first and second oligonucleotide probes are 5, 6, 7, 8, 9, 10, 11, 12, 13, 14, or 15 nucleotides in length.

120. The plurality of oligonucleotide probes of any one of clauses 117-119, wherein the barcode sequences on the first and second oligonucleotide probes are 10 nucleotides in length.

121. The plurality of oligonucleotide probes of any one of clauses 117-120, wherein the first oligonucleotide probe comprises the structure:
5'-[portion complementary to second probe]-[portion complementary to nucleic acid of interest]-[first barcode sequence]-[second barcode sequence]-3'.

122. The plurality of oligonucleotide probes of any one of clauses 117-121, wherein the second oligonucleotide probe comprises the structure:
5'-[portion complementary to nucleic acid of interest]-[portion complementary to first probe]-[barcode sequence]-3'.

123. The plurality of oligonucleotide probes of any one of clauses 117-122, wherein the second barcode sequence increases the specificity of the detection of the nucleic acid of interest.

124. The plurality of oligonucleotide probes of any one of clauses 117-123, wherein the second barcode sequence reduces non-specific amplification.

125. A kit comprising the plurality of oligonucleotide probes of any one of clauses 117-124.

126. The kit of clause 125, wherein the kit further comprises one or more antibodies, wherein each antibody binds to a protein of interest.

127. The kit of clause 125 or 126, wherein the antibodies comprise an anti-p-Tau antibody.

128. The kit of any one of clauses 125-127 further comprising a small molecule detecting agent.

129. The kit of clause 128, wherein the small molecule detecting agent is X-34.

130. The kit of any one of clauses 125-129, wherein the kit further comprises a third oligonucleotide probe.

131. The kit of clause 130, wherein the third oligonucleotide probe comprises a sequence that is complementary to the first barcode sequence of the first oligonucleotide probe.

132. The kit of clause 130 or 131, wherein the third oligonucleotide probe comprises a detectable label.

133. The kit of clause 132, wherein the detectable label is a fluorophore.

134. A method of identifying spatial variations of cell types in at least one image, the method comprising:

receiving, for each of a plurality of cells in the at least one image, a spatial location of the cell in the at least one image;
receiving, for each of a plurality of proteins in the at least one image, a spatial location of the protein in the image;
for a first protein of the plurality of proteins, determining a number of cells of a first cell type having a distance to the first protein less than a threshold distance, wherein the distances are determined based on at least some of the spatial locations of the plurality of cells and at least some of the spatial locations of the plurality of proteins;
based on the number of cells of the first cell type, identifying a spatial variation in cells of the first cell type in the at least one image; and
outputting an indication of the spatial variation in cells of the first cell type in the at least one image.

135. The method of clause 134, wherein identifying the spatial variation in cells of the first cell type in the at least one image comprises:

for cells of the plurality of cells having a distance to the first protein less than the threshold distance, determining a first percentage of the cells that are associated with the first cell type;
determining a second percentage of cells of the plurality of cells in the at least one image that are associated with the first cell type;
comparing the first percentage to the second percentage to obtain a comparison result; and
identifying the spatial variation in cells of the first cell type in the at least one image using the comparison result.

136. The method of clause 135, wherein:

the comparison result indicates that the first percentage is greater than the second percentage; and
identifying the spatial variation in cells of the first cell type in the at least one image using the comparison result comprises identifying that there is an enrichment of cells of the first cell type within a region having a distance to the first protein less than the threshold distance.

137. The method of clause 134, further comprising capturing the at least one image using a camera.

138. The method of clause 137, wherein the at least one image comprises a plurality of images and wherein capturing the at least one image using a camera comprises:

capturing a first image of the plurality of images, the first image being used to determine the spatial locations of the plurality of cells; and
capturing a second image of the plurality of images, the second image being used to determine the spatial locations of the plurality of proteins.

139. The method of clause 138, further comprising:

spatially aligning the spatial locations of the plurality of cells from the first image with the spatial locations of the plurality of proteins from the second image; and
based on the spatially aligned spatial locations, determining the number of cells of the first cell type having the distance to the first protein less than the threshold distance.

140. The method of clause 134, wherein the at least one image comprises a plurality of images comprising:

a first image used to determine the spatial locations of the plurality of cells; and
a second image used to determine the spatial locations of the plurality of proteins.

141. The method of clause 138, wherein:

the first image and the second image are spatially aligned; and
the number of cells of the first cell type having the distance to the first protein less than the threshold distance is determined based on the spatially aligned images.

142. The method of clause 134, wherein determining the number of cells of the first cell type having the distance to the first protein less than the threshold distance comprises determining the number of cells of the first cell type having the distance from a centroid of the cell to an edge of the first protein less than the threshold distance.

143. The method of clause 134, wherein determining the number of cells of the first cell type having the distance to the first protein less than the threshold distance comprises, for each cell associated with the first cell type:

determining a minimum distance from the cell of the first cell type to a nearest protein of the plurality of proteins; and
comparing the minimum distance to the threshold distance.

144. The method of clause 143, further comprising, based on the minimum distance from the cell of the first cell type to the nearest protein for each cell associated with the first cell type, determining an average minimum distance from cells of the first cell type to nearest proteins.

145. The method of clause 134, further comprising, for each of the plurality of cells:

receiving genetic information of the cell; and
associating with the cell, based on the genetic information of the cell, a cell type from a plurality of cell types, wherein the plurality of cell types includes the first cell type.

146. An apparatus comprising:

at least one computer processor; and
at least one non-transitory computer-readable storage medium encoded with a plurality of instructions that, when executed by at least one computer processor, perform a method of identifying spatial variations of cell types in at least one image, the method comprising:

receiving, for each of a plurality of cells in the at least one image, a spatial location of the cell in the at least one image;
receiving, for each of a plurality of proteins in the at least one image, a spatial location of the protein in the image;
for a first protein of the plurality of proteins, determining a number of cells of a first cell type having a distance to the first protein less than a threshold distance, wherein the distances are determined based on at least some of the spatial locations of the plurality of cells and at least some of the spatial locations of the plurality of proteins;
based on the number of cells of the first cell type, identifying a spatial variation in cells of the first cell type in the at least one image; and
outputting an indication of the spatial variation in cells of the first cell type in the at least one image.

147. The apparatus of clause 146, wherein identifying the spatial variation in cells of the first cell type in the at least one image comprises:

for cells of the plurality of cells having a distance to the first protein less than the threshold distance, determining a first percentage of the cells that are associated with the first cell type;
determining a second percentage of cells of the plurality of cells in the at least one image that are associated with the first cell type;
comparing the first percentage to the second percentage to obtain a comparison result; and
identifying the spatial variation in cells of the first cell type in the at least one image using the comparison result.

148. The apparatus of clause 147, wherein:

the comparison result indicates that the first percentage is greater than the second percentage; and identifying the spatial variation in cells of the first cell type in the at least one image using the comparison result comprises identifying that there is an enrichment of cells of the first cell type within a region having a distance to the first protein less than the threshold distance.

149. The apparatus of clause 146, wherein the method further comprises capturing the at least one image using a camera.

150. The apparatus of clause 149, wherein the at least one image comprises a plurality of images, and wherein capturing the at least one image using a camera comprises:

capturing a first image of the plurality of images, the first image being used to determine the spatial locations of the plurality of cells; and capturing a second image of the plurality of images, the second image being used to determine the spatial locations of the plurality of proteins.

151. The apparatus of clause 150, wherein the method further comprises:

spatially aligning the spatial locations of the plurality of cells from the first image with the spatial locations of the plurality of proteins from the second image; and based on the spatially aligned spatial locations, determining the number of cells of the first cell type having the distance to the first protein less than the threshold distance.

152. The apparatus of clause 146, wherein the at least one image comprises a plurality of images comprising:

a first image used to determine the spatial locations of the plurality of cells; and a second image used to determine the spatial locations of the plurality of proteins.

153. The apparatus of clause 150, wherein:

the first image and the second image are spatially aligned; and the number of cells of the first cell type having the distance to the first protein less than the threshold distance is determined based on the spatially aligned images.

154. The apparatus of clause 146, wherein determining the number of cells of the first cell type having the distance to the first protein less than the threshold distance comprises determining the number of cells of the first cell type having the distance from a centroid of the cell to an edge of the first protein less than the threshold distance.

155. The apparatus of clause 146, wherein determining the number of cells of the first cell type having the distance to the first protein less than the threshold distance comprises, for each cell associated with the first cell type:

determining a minimum distance from the cell of the first cell type to a nearest protein of the plurality of proteins; and comparing the minimum distance to the threshold distance.

156. The apparatus of clause 155, wherein the method further comprises, based on the minimum distance from the cell of the first cell type to the nearest protein for each cell associated with the first cell type, determining an average minimum distance from cells of the first cell type to nearest proteins.

157. The apparatus of clause 146, wherein the method further comprises, for each of the plurality of cells:

receiving genetic information of the cell; and associating with the cell, based on the genetic information of the cell, a cell type from a plurality of cell types, wherein the plurality of cell types includes the first cell type.

158. At least one non-transitory computer-readable storage medium encoded with a plurality of instructions that, when executed at least one computer processor, perform a method of identifying spatial variations of cell types in at least one image, the method comprising:

receiving, for each of a plurality of cells in the at least one image, a spatial location of the cell in the at least one image;

receiving, for each of a plurality of proteins in the at least one image, a spatial location of the protein in the image;

for a first protein of the plurality of proteins, determining a number of cells of a first cell type having a distance to the first protein less than a threshold distance, wherein the distances are determined based on at least some of the spatial locations of the plurality of cells and at least some of the spatial locations of the plurality of proteins;

based on the number of cells of the first cell type, identifying a spatial variation in cells of the first cell type in the at least one image; and

outputting an indication of the spatial variation in cells of the first cell type in the at least one image.

159. The at least one non-transitory computer-readable storage medium of clause 158, wherein identifying the spatial variation in cells of the first cell type in the at least one image comprises:

for cells of the plurality of cells having a distance to the first protein less than the threshold distance, determining a first percentage of the cells that are associated with the first cell type;

determining a second percentage of cells of the plurality of cells in the at least one image that are associated with the first cell type;

comparing the first percentage to the second percentage to obtain a comparison result; and

identifying the spatial variation in cells of the first cell type in the at least one image using the comparison result.

160. The at least one non-transitory computer-readable storage medium of clause 159, wherein:

the comparison result indicates that the first percentage is greater than the second percentage; and

identifying the spatial variation in cells of the first cell type in the at least one image using the comparison result comprises identifying that there is an enrichment of cells of the first cell type within a region having a distance to the first protein less than the threshold distance.

161. The at least one non-transitory computer-readable storage medium of clause 158, wherein the method further comprises capturing the at least one image using a camera.

162. The at least one non-transitory computer-readable storage medium of clause 161, wherein the at least one image comprises a plurality of images, and wherein capturing the at least one image using a camera comprises:

capturing a first image of the plurality of images, the first image being used to determine the spatial locations of the plurality of cells; and

capturing a second image of the plurality of images, the second image being used to determine the spatial locations of the plurality of proteins.

163. The at least one non-transitory computer-readable storage medium of clause 162, wherein the method further comprises:

spatially aligning the spatial locations of the plurality of cells from the first image with the spatial locations of the plurality of proteins from the second image; and

based on the spatially aligned spatial locations, determining the number of cells of the first cell type having the distance to the first protein less than the threshold distance.

164. The at least one non-transitory computer-readable storage medium of clause 158, wherein the at least one image comprises a plurality of images comprising:

a first image used to determine the spatial locations of the plurality of cells; and

a second image used to determine the spatial locations of the plurality of proteins.

165. The at least one non-transitory computer-readable storage medium of clause 162, wherein:

the first image and the second image are spatially aligned; and

the number of cells of the first cell type having the distance to the first protein less than the threshold distance is determined based on the spatially aligned images.

166. The at least one non-transitory computer-readable storage medium of clause 158, wherein determining the number of cells of the first cell type having the distance to the first protein less than the threshold distance comprises determining the number of cells of the first cell type having the distance from a centroid of the cell to an edge of the first protein less than the threshold distance.

167. The at least one non-transitory computer-readable storage medium of clause 158, wherein determining the number of cells of the first cell type having the distance to the first protein less than the threshold distance comprises, for each cell associated with the first cell type:

determining a minimum distance from the cell of the first cell type to a nearest protein of the plurality of proteins; and
comparing the minimum distance to the threshold distance.

168. The at least one non-transitory computer-readable storage medium of clause 167, wherein the method further comprises, based on the minimum distance from the cell associated with the first cell type to the nearest protein for each cell of the first cell type, determining an average minimum distance from cells of the first cell type to nearest proteins.

169. The at least one non-transitory computer-readable storage medium of clause 158, wherein the method further comprises, for each of the plurality of cells:

receiving genetic information of the cell; and
associating with the cell, based on the genetic information of the cell, a cell type from a plurality of cell types, wherein the plurality of cell types includes the first cell type.

170. A system comprising:

a) a cell; and
b) one or more pairs of oligonucleotide probes comprising a first oligonucleotide probe and a second oligonucleotide probe, wherein:

i) the first oligonucleotide probe comprises a portion that is complementary to the second oligonucleotide probe, a portion that is complementary to a nucleic acid of interest, a first barcode sequence, and a second barcode sequence; and
ii) the second oligonucleotide probe comprises a portion that is complementary to the nucleic acid of interest, a portion that is complementary to the first oligonucleotide probe, and a barcode sequence, wherein the barcode sequence of the second oligonucleotide probe is complementary to the second barcode sequence of the first oligonucleotide probe.

171. The system of clause 170 further comprising a microscope.

172. The system of clause 171, wherein the microscope is a confocal microscope.

173. The system of any one of clauses 170-172 further comprising a computer.

174. The system of any one of clauses 170-173 further comprising one or more additional cells.

175. The system of clause 174, wherein the cells are part of a tissue sample.

**Claims**

1. A system comprising:

a cell;

one or more pairs of oligonucleotide probes, wherein each pair of oligonucleotide probes comprises a first oligonucleotide probe and a second oligonucleotide probe, wherein:

(i) the first oligonucleotide probe comprises a portion that is complementary to the second oligonucleotide probe, a portion that is complementary to a nucleic acid of interest, a first barcode sequence, and a second barcode sequence, wherein the portion of the first oligonucleotide probe that is complementary to the second oligonucleotide probe is split between a 5' end and a 3' end of the first oligonucleotide probe; and
(ii) the second oligonucleotide probe comprises a portion that is complementary to the nucleic acid of interest, a portion that is complementary to the first oligonucleotide probe, and a barcode sequence, wherein the barcode sequence of the second oligonucleotide probe is complementary to the second barcode sequence of the first oligonucleotide probe; and

a computer;
wherein the system is configured to perform the following method steps:

(a) contacting the cell with the one or more pairs of oligonucleotide probes;
(b) ligating the 5' end and the 3' end of the first oligonucleotide probe together to produce a circular oligonucleotide;
(c) performing rolling circle amplification to amplify the circular oligonucleotide using the second oligonucleotide probe as a primer to produce one or more amplicons;
(d) contacting the cell with a detecting agent, wherein the detecting agent binds to a protein of interest;
(e) embedding the one or more amplicons and the detecting agent in a polymeric matrix to generate one or more embedded amplicons;
(f) contacting the one or more embedded amplicons with a third oligonucleotide probe comprising a sequence that is complementary to the first barcode sequence of the first oligonucleotide probe; and
(g) identifying a nucleic acid of interest or a protein of interest, wherein the nucleic acid of interest is RNA.

2. The system of claim 1, further comprising a microscope.

3. The system of claim 1 or 2, wherein step (g) comprises imaging the third oligonucleotide probe.

4. The system of any one of claims 1-3, wherein step (c) comprises providing amine-modified nucleotides, wherein the amine-modified nucleotides are incorporated into the one or more amplicons.

5. The system of claim 4, wherein step (e) comprises reacting the amine-modified nucleotides.

6. The system of any one of claims 1-5, wherein the polymeric matrix is a hydrogel.

7. The system of any one of claims 1-6, wherein step (b) comprises a ligase; optionally, wherein, the ligase is a T4 ligase.

8. The system of any one of claims 1-7, wherein step (c) comprises a polymerase; optionally, wherein, the polymerase is a DNA polymerase.

9. The system of any one of claims 2-8, wherein step (g) comprises use of the microscope to detect a fluorescence signal associated with the RNA and/or the protein.

10. The system of any one of claims 1-9, wherein the detecting agent comprises an antibody.

11. The system of any one of claims 1-10, wherein the RNA is a messenger RNA.

12. The system of any one of claims 1-11, wherein the third oligonucleotide comprises a detectable label; optionally, wherein the detectable label comprises a fluorophore.

13. The system of any one of claims 1-12, wherein the detecting agent comprises a detectable label; optionally, wherein the detectable label comprises a fluorophore.

14. One or more non-transitory computer-readable storage media comprising instructions which, when executed by the computer of claim 1, cause the system of claim 1 or 2 to carry out the method of any one of claims 1-13.

15. A computer system (2100) comprising:
    a processor (2110) configured to execute one or more computer processor-executable instructions stored in the one or more non-transitory computer-readable storage media of claim 14.

**FIG. 1A**

**FIG. 1B**

Coding capacity: $4^{16} \approx 10^8$

**FIG. 1C**

**FIG. 1D**

**FIG. 1E**

**FIG. 1F**

FIG. 2A

**FIG. 2B**

**FIG. 2C**

**FIG. 2D**

**FIG. 2E**

FIG. 2F

FIG. 2G

FIG. 2H

FIG. 2I

**FIG. 2J**

**FIG. 2K**

FIG. 2L

**FIG. 3A**

FIG. 3B

FIG. 3C

**FIG. 3D**

FIG. 3E

Control 13M

FIG. 3F

TauPS2APP 13M

**FIG. 3G**

**FIG. 3H**

**FIG. 3I**

**FIG. 3J**

**FIG. 3K**

**FIG. 3L**

DC 2

DC 1

Diseased-associated
trajectory

Pseudotime

1.0

0.0

Control 8mo

TauPS2APP 8mo

Control 13mo

TauPS2APP 13mo

**FIG. 3M**

All

Micro1

Micro2

Micro3

**FIG. 3N**

**FIG. 3O**

**FIG. 3P**

**FIG. 3Q**

Ctsb

0.0   2.2

P2ry12

0.0   2.4

Cst7

0.0   2.6

Trem2

0.0   2.7

Log10(Expression)

**FIG. 3R**

FIG. 3S

**FIG. 4A**

**FIG. 4B**

FIG. 4C

**All**

**Astro1**

**Astro2**

**Astro3**

**FIG. 4D**

**FIG. 4E**

Control 13M

**FIG. 4F**

TauPS2APP 13M

**FIG. 4G**

FIG. 4H

**FIG. 4I**

## GO:BP Enrichment

FIG. 4J

FIG. 4K

FIG. 4L

FIG. 4M

FIG. 4N

All

Astro1

Astro2

Astro3

FIG. 4O

**FIG. 4P**

**FIG. 4Q**

**FIG. 4R**

Gfap

0.0    2.5

Vim

0.0    2.5

Cst3

0.0    3.5

Aqp4

0.0    2.0

Log10(Expression)

FIG. 4S

FIG. 4T

FIG. 5A

**FIG. 5B**

**FIG. 5C**

**FIG. 5D**

FIG. 5E

FIG. 5F

TauPS2APP 13M

FIG. 5G

**FIG. 5H**

**FIG. 5I**

**FIG. 5J**

**FIG. 5K**

**FIG. 5L**

**FIG. 5M**

**FIG. 5N**

**FIG. 5O**

**FIG. 5P**

**FIG. 5Q**

**FIG. 5R**

**FIG. 5S**

**FIG. 5T**

FIG. 5U

FIG. 5V

Excitatory neuron

FIG. 6A

Inhibitory neuron

Cnr1
Lamp5
Pvalb
Sst
Aß-plaque
p-Tau

100 µm

10 µm

**FIG. 6B**

**FIG. 6C**

**FIG. 6D**

**FIG. 6E**

**FIG. 6F**

FIG. 6G

**FIG. 6H**

FIG. 6I

FIG. 6J

FIG. 6K

FIG. 6L

**FIG. 6M**

**FIG. 6N**

Excitatory neuron

100 μm

CTX-Ex1    CA1-Ex
CTX-Ex2    CA2-Ex
CTX-Ex3    CA3-Ex
CTX-Ex4    DG
Aβ-plaque
p-Tau

**FIG. 6O**

FIG. 6P

**FIG. 6Q**

**FIG. 6R**

**FIG. 6S**

**FIG. 6T**

**FIG. 6U**

**FIG. 7A**

**FIG. 7B**

**FIG. 7C**

**FIG. 7D**

FIG. 7E

FIG. 7F

FIG. 7G

FIG. 7H

**FIG. 7I**

**FIG. 7J**

**FIG. 7K**

**FIG. 7L**

FIG. 7M

FIG. 8A

FIG. 8B

**FIG. 8C**

**FIG. 8D**

**FIG. 8E**

FIG. 9A

**FIG. 9B**

**FIG. 9C**

Control 8M (2766 genes)     Control 13M (2766 genes)

TauPS2APP 8M (2766 genes)     TauPS2APP 13M (2766 genes)

**FIG. 9D**

Control 8M (validation)     Control 13M (validation)

TauPS2APP 8M (validation)     TauPS2APP 13M (validation)

**FIG. 9E**

**FIG. 9F**

**FIG. 9G**

Spatial map of microglia subtypes

FIG. 10A

**FIG. 10B**

Spatial map of microglia pseudotime analysis

FIG. 10C

FIG. 10D

**FIG. 10E**

FIG. 11A

FIG. 11B

Spatial map of astrocyte pseudotime analysis

FIG. 11C

**TauPS2APP 8M**

**TauPS2APP 13M**

**FIG. 11D**

**FIG. 11E**

Spatial map of oligodendrocyte subtypes and OPC

**FIG. 12A**

FIG. 12B

Spatial map of oligodendrocyte subtypes and OPC pseudotime analysis

FIG. 12C

**FIG. 12D**

**FIG. 12E**

FIG. 12F

FIG. 13A

Control 8M (Inhibitory)

Control 13M (Inhibitory)

100 µm

100 µm

TauPS2APP 8M (Inhibitory)

TauPS2APP 13M (Inhibitory)

100 µm

100 µm

FIG. 13B

**FIG. 13C**

**FIG. 13D**

FIG. 13E

Inhibitory neuron

-log10 Q-value
too few genes
not significant
2
4
6
8
10
12
≥14

**FIG. 13F**

Tau + vs. Tau -

-log10 Q-value
too few genes
not significant
2
4
6
8
10
≥12

**FIG. 13G**

FIG. 14A

FIG. 14B

EP 4 644 569 A2

FIG. 14C

FIG. 14D

**FIG. 14E**

1500

Start

1502

Receive, for each of a plurality of cells in the at least one image, a spatial location of the cell in the at least one image

1504

Receive, for each of a plurality of proteins in the at least one image, a spatial location of the protein in the image

1506

For a first protein of the plurality of proteins, determine a number of cells of a first cell type having a distance to the first protein less than a threshold distance, wherein the distances are determined based on at least some of the spatial locations of the plurality of cells and at least some of the spatial locations of the plurality of proteins

1508

Based on the number of cells of the first cell type, identify a spatial variation in cells of the first cell type in the at least one image

1510

Output an indication of the spatial variation in cells of the first cell type in the at least one image

**FIG. 15**

1600

```
                              ┌─────────┐
                              │  Start  │
                              └─────────┘
                                   │
                                   ▼
1602  ┌──────────────────────────────────────────────┐
      │ For cells of the plurality of cells having a  │
      │ distance to the first protein less than the   │
      │ threshold distance, determine a first         │
      │ percentage of the cells that are associated   │
      │ with the first cell type                      │
      └──────────────────────────────────────────────┘
                                   │
                                   ▼
1604  ┌──────────────────────────────────────────────┐
      │ Determine a second percentage of cells of the │
      │ plurality of cells in the at least one image  │
      │ that are associated with the first cell type  │
      └──────────────────────────────────────────────┘
                                   │
                                   ▼
1606  ┌──────────────────────────────────────────────┐
      │ Compare the first percentage to the second    │
      │ percentage to obtain a comparison result      │
      └──────────────────────────────────────────────┘
                                   │
                                   ▼
1608  ┌──────────────────────────────────────────────┐
      │ Identify the spatial variation in cells of    │
      │ the first cell type in the at least one image │
      │ using the comparison result                   │
      └──────────────────────────────────────────────┘
```

**FIG. 16**

1700

Start

1702

Capture a first image of the plurality of images, the first image being used to determine the spatial locations of the plurality of cells

1704

Capture a second image of the plurality of images image, the second image being used to determine the spatial locations of the plurality of proteins

**FIG. 17**

1800

Start

1802

Spatially align the spatial locations of the plurality of cells from the first image with the spatial locations of the plurality of proteins from the second image

1804

Based on the spatially aligned spatial locations, determine the number of cells of the first cell type having the distance to the first protein less than the threshold distance

**FIG. 18**

1900

Start

1902

Determine a minimum distance from the cell of the
first cell type to a nearest protein of the plurality of
proteins

1904

Compare the minimum distance to the threshold
distance

**FIG. 19**

2000

Start

2002

Receive genetic information of the cell

2004

Associate with the cell, based on the genetic information of the cell, a cell type from a plurality of cell types, wherein the plurality of cell types includes the first cell type

**FIG. 20**

2100

Processor
2110

Memory
2120

Non-Volatile Storage
2130

**FIG. 21**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63194536
- WO 2019199579 A

### Non-patent literature cited in the description

- **SINGLETON et al.** Dictionary of Microbiology and Molecular Biology. 1994
- The Cambridge Dictionary of Science and Technology. 1988
- The Glossary of Genetics. Springer Verlag, 1991
- **HALE** ; **MARHAM**. *The Harper Collins Dictionary of Biology*, 1991
- Stedman's Medical Dictionary. Williams & Wilkins, 1990
- **VAN DEN BERGE, K. et al.** Trajectory-based differential expression analysis for single-cell sequencing data.. *Nature Communications*, 2020, vol. 11, 1-13
- **WANG et al.** *Science*, 2018, vol. 361, 380
- **WANG, X. et al.** Three-dimensional intact-tissue sequencing of single-cell transcriptional states.. *Science*, 2018, vol. 36, eaat5691
- **STYREN, S. D. et al.** X-34, a fluorescent derivative of Congo red: a novel histochemical stain for Alzheimer's disease pathology.. *J. Histochem. Cytochem.*, 2000, vol. 48 (9), 1223-1232
- **LEE et al.** *Neuron*, 2021
- **BRAAK, H** ; **BRAAK, E**. Neuropathological stageing of Alzheimer-related changes.. *Acta Neuropathologica.*, 1991, vol. 82, 239-259
- **BUSCHE, M.A.** ; **HYMAN, B.T**. Synergy between amyloid-β and tau in Alzheimer's disease.. *Nature Neuroscience.*, 2020, vol. 23, 1183-1193
- **CAO, J.** ; **SPIELMANN, M** ; **QIU, X** ; **HUANG, X.** ; **IBRAHIM, D.M** ; **HILL, A.J** ; **ZHANG, F** ; **MUNDLOS, S** ; **CHRISTIANSEN, L** ; **STEEMERS, F.J. et al.** The single-cell transcriptional landscape of mammalian organogenesis.. *Nature*, 2019, vol. 566, 496-502
- **CHEN, W.-T.** ; **LU, A** ; **CRAESSAERTS, K** ; **PAVIE, B** ; **SALA FRIGERIO, C** ; **CORTHOUT, N.** ; **QIAN, X.** ; **LALÁKOVÁ, J.** ; **KÜHNEMUND, M** ; **VOYTYUK, I. et al.** Spatial Transcriptomics and In Situ Sequencing to Study Alzheimer's Disease.. *Cell*, 2020, vol. 182, 976-991
- **GRUBMAN, A.** ; **CHEW, G** ; **OUYANG, J.F** ; **SUN, G.** ; **CHOO, X.Y** ; **MCLEAN, C** ; **SIMMONS, R.K** ; **BUCKBERRY, S** ; **VARGAS-LANDIN, D.B** ; **POPPE, D et al.** A single-cell atlas of entorhinal cortex from individuals with Alzheimer's disease reveals cell-type-specific gene expression regulation.. *Nat. Neurosci.*, 2019, vol. 22, 2087-2097
- **GRUENINGER, F** ; **BOHRMANN, B.** ; **CZECH, C** ; **BALLARD, T.M** ; **FREY, J.R** ; **WEIDENSTEINER, C.** ; **VON KIENLIN, M** ; **OZMEN, L.** Phosphorylation of Tau at S422 is enhanced by Aβ in TauPS2APP triple transgenic mice.. *Neurobiol. Dis.*, 2010, vol. 37, 294-306
- **HABIB, N.** ; **MCCABE, C** ; **MEDINA, S** ; **VARSHAVSKY, M** ; **KITSBERG, D** ; **DVIR-SZTERNFELD, R** ; **GREEN, G** ; **DIONNE, D** ; **NGUYEN, L** ; **MARSHALL, J.L et al.** Disease-associated astrocytes in Alzheimer's disease and aging.. *Nat. Neurosci.*, 2020, vol. 23, 701-706
- **HARDY, J.** ; **SELKOE, D.J**. The amyloid hypothesis of Alzheimer's disease: progress and problems on the road to therapeutics.. *Science*, 2002, vol. 297, 353-356
- **KEREN-SHAUL, H** ; **SPINRAD, A.** ; **WEINER, A.** ; **MATCOVITCH-NATAN, O** ; **DVIR-SZTERNFELD, R** ; **ULLAND, T.K** ; **DAVID, E** ; **BARUCH, K** ; **LARA-ASTAISO, D** ; **TOTH, B. et al.** A Unique Microglia Type Associated with Restricting Development of Alzheimer's Disease.. *Cell*, 2017, vol. 169, 1276-1290
- **LAU, S.-F** ; **CAO, H.** ; **FU, A.K.Y** ; **IP, N.Y**. Single-nucleus transcriptome analysis reveals dysregulation of angiogenic endothelial cells and neuroprotective glia in Alzheimer's disease.. *Proceedings of the National Academy of Sciences*, 2020, vol. 117, 25800-25809
- **LEE, S.-H.** ; **MEILANDT, W.J** ; **XIE, L** ; **GANDHAM, V.D** ; **NGU, H** ; **BARCK, K.H** ; **REZZONICO, M.G** ; **IMPERIO, J.** ; **LALEHZADEH, G** ; **HUNTLEY, M.A. et al.** Trem2 restrains the enhancement of tau accumulation and neurodegeneration by β-amyloid pathology.. *Neuron.*, 2021, vol. 109, 1283-1301

- **MASTERS, C.L.** ; **BATEMAN, R** ; **BLENNOW, K** ; **ROWE, C.C.** ; **SPERLING, R.A** ; **CUMMINGS, J.L**. Alzheimer's disease. *Nat Rev Dis Primers.*, 2015, vol. 1, 15056
- **MATHYS, H** ; **DAVILA-VELDERRAIN, J.** ; **PENG, Z** ; **GAO, F** ; **MOHAMMADI, S** ; **YOUNG, J.Z.** ; **MENON, M** ; **HE, L** ; **ABDURROB, F** ; **JIANG, X. et al.** Single-cell transcriptomic analysis of Alzheimer's disease.. *Nature*, 2019, vol. 570, 332-337
- **MCINNES, L.** ; **HEALY, J.** ; **SAUL, N** ; **GROßBERGER, L**. UMAP: Uniform Manifold Approximation and Projection.. *Journal of Open Source Software.*, 2018, vol. 3, 861
- **STÅHL, P.L.** ; **SALMÉN, F** ; **VICKOVIC, S** ; **LUND-MARK, A** ; **NAVARRO, J.F** ; **MAGNUSSON, J.** ; **GIACOMELLO, S.** ; **ASP, M.** ; **WESTHOLM, J.O** ; **HUSS, M et al.** Visualization and analysis of gene expression in tissue sections by spatial transcriptomics.. *Science*, 2016, vol. 353, 78-82
- **STUART, T.** ; **SATIJA, R.** Integrative single-cell analysis.. *Nat. Rev. Genet.*, 2019, vol. 20, 257-272
- **TRAAG, V.A** ; **WALTMAN, L** ; **VAN ECK, N.J.** From Louvain to Leiden: guaranteeing well-connected communities.. *Sci. Rep.*, 2019, vol. 9, 5233
- **WANG, X** ; **ALLEN, W.E** ; **WRIGHT, M.A** ; **SYLWES-TRAK, E.L** ; **SAMUSIK, N** ; **VESUNA, S** ; **EVANS, K.** ; **LIU, C.** ; **RAMAKRISHNAN, C.** ; **LIU, J. et al.** Three-dimensional intact-tissue sequencing of single-cell transcriptional states.. *Science*, 2018, vol. 361
- **ZHOU, Y.** ; **SONG, W.M** ; **ANDHEY, P.S** ; **SWAIN, A** ; **LEVY, T** ; **MILLER, K.R** ; **POLIANI, P.L** ; **COMINELLI, M** ; **GROVER, S.** ; **GILFILLAN, S. et al.** Human and mouse single-nucleus transcriptomics reveal TREM2-dependent and TREM2-independent cellular responses in Alzheimer's disease.. *Nat. Med.*, 2020, vol. 26, 131-142